# EUROPÄISCHE PATENTSCHRIFT

(11) **EP 1 318 977 B1**
(45) Veröffentlichungstag und Bekanntmachung des Hinweises auf die Patenterteilung: **06.12.2006**
(21) Anmeldenummer: 01980296.6
(22) Anmeldetag: 29.08.2001
(51) Int. Cl.: C07C 275/42, C07C 275/26, C07C 271/24, C07C 233/63, C07C 235/12, C07D 295/205, C07D 295/215, C07D 213/75, C07D 211/48, A61K 31/17, A61K 31/44, A61K 31/495, A61K 31/5375, A61P 9/10

(54) **SUBSTITUIERTE PHENYLCYCLOHEXANCARBONSÄUREAMIDE UND IHRE VERWENDUNG**
SUBSTITUTED PHENYLCYCLOHEXANE CARBOXYLIC ACID AMIDES AND THE USE THEREOF
AMIDES D'ACIDE CARBOXYLIQUE DE PHENYLCYCLOHEXANE SUBSTITUEES ET LEUR UTILISATION

(30) Priorität: 11.09.2000 DE 10044792
(43) Veröffentlichungstag der Anmeldung: 18.06.2003
(73) Patentinhaber: Bayer HealthCare AG, 51368 Leverkusen (DE)
(72) Erfinder: BISCHOFF, Erwin, 42115 Wuppertal (DE); KRAHN, Thomas, 58135 Hagen (DE); MÜLLER, Stephan-Nicholas, 42105 Wuppertal (DE); PAULSEN, Holger, 42115 Wuppertal (DE); SCHUHMACHER, Joachim, 42109 Wuppertal (DE); STEINHAGEN, Henning, D 65843 Sulzbach (DE); THIELEMANN, Wolfgang, D 42107 Wuppertal (DE)
(86) Internationale Anmeldenummer: PCT/EP2001/009938
(87) Internationale Veröffentlichungsnummer: WO 2002/020472

(56) Entgegenhaltungen:
- EP-A- 0 582 164
- EP-A- 0 611 767

## Beschreibung

Die vorliegende Erfindung betrifft substituierte Phenylcyclohexancarbonsäureamide, ein Verfahren zu ihrer Herstellung und ihre Verwendung in Arzneimitteln, insbesondere zur Prävention und/oder Behandlung von kardiovaskulären Erkrankungen, z.B. zur akuten und chronischen Behandlung von ischämischen Erkrankungen.

Adenosin ist ein endogener Effektor mit zellprotektiver Wirksamkeit, insbesondere unter zellschädigenden Bedingungen mit begrenzter Sauerstoffversorgung wie z.B bei Ischämie. Adenosin ist ein stark wirksamer Vasodilatator. Es verstärkt das ischämische "preconditioning" (R. Strasser, A. Vogt, W. Scharper, Z. Kardiologie 85, 1996, 79-89) und es kann das Wachstum von Kollateralgefäßen fördern. Es wird unter hypoxischen Bedingungen z.B. bei kardialen oder peripheren Verschlusskrankheiten freigesetzt (W. Makarewicz "Purine and Pyrimidine Metabolism in Man", Plenum Press New York, 11, 1998, 351-357). Daher schützt Adenosin vor den Folgen Ischaemie-bedingter Erkrankungen, z.B. indem es die koronare oder periphere Durchblutung durch Vasodilatation steigert, die Thombozytenaggregation inhibiert und die Angiogenese stimuliert. Der Vorteil der Adenosinaufnahme-Hemmer gegenüber systemisch verabreichtem Adenosin liegt in der Ischämieselektivität. Außerdem hat systemisch verabreichtes Adenosin eine sehr kurze Halbwertszeit. Systemisch verabreichtes Adenosin führt zu einer starken systemischen Blutdrucksenkung, welche unerwünscht ist, da der Blutfluß in die ischämischen Gebiete noch weiter reduziert werden kann ("steal phenomenon", L.C. Becker, Circulation 57, 1978, 1103-1110). Der Adenosinaufnahme-Hemmer verstärkt die Wirkung des lokal durch die Ischämie entstandenen Adenosins und dilatiert daher nur die Gefäße in den ischämischen Bereichen. Somit können Adenosinaufnahme-Hemmer durch orale oder intravenöse Applikation zur Prävention und/oder Behandlung von ischämischen Erkrankungen eingesetzt werden.

Verschiedene Hinweise deuten darüber hinaus auf ein neuroprotektives, antikonvulsives, analgetisches und Schlaf-induzierendes Potential von Adenosinaufnahme-Hemmern, da sie die Eigeneffekte von Adenosin durch eine Hemmung seiner zellulären Rückaufnahme verstärken (K.A. Rudolphi et al., Cerebrovascular and Brain Metabolism Reviews 4, 1992, 364-369; T.F. Murray et al., Drug Dev. Res. 28, 1993, 410-415; T. Porkka-Heiskanen et al., Science 276, 1997, 1265-1268; 'Adenosine in the Nervous System', Ed.: Trevor Stone, Academic Press Ltd. 1991, 217-227; M.P. DeNinno, Annual Reports in Medicinal Chemistry 33, 1998, 111-120).

EP-A- 0 582 164 offenbart 1-Piperazinyl-N-phenylacetamid Derivate mit Adenosin aufnahme hemmender Wirkung.

Aufgabe der vorliegenden Erfindung ist nunmehr die Bereitstellung neuer Substanzen zur Prävention und/oder Behandlung von kardiovaskulären Erkrankungen, wobei die Substanzen über verbesserte Applikationseigenschaften verfügen.

Die vorliegende Erfindung betrifft Verbindungen der Formel 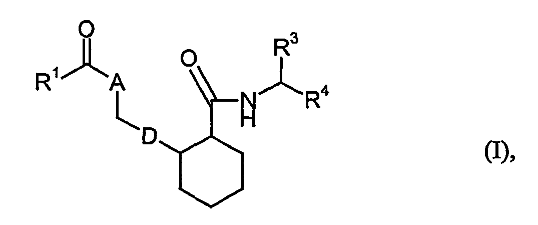 worin
- D: einen Rest 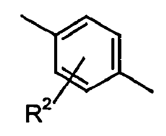 oder 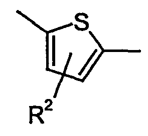 bedeutet,
worin
R² Wasserstoff, Halogen, Hydroxy, Carboxyl, Cyano, Nitro, Trifluormethyl, Trifluormethoxy, (C₁-C₆)-Alkyl, (C₁-C₆)-Alkoxy oder (C₁-C₆)-Alkoxycarbonyl bedeutet,
- A: ein Sauerstoffatom oder eine Gruppe der Formel N-R⁵ oder CH-R⁶ bedeutet,
worin
R⁵ Wasserstoff, (C₁-C₆)-Alkyl, (C₃-C₇)-Cycloalkyl, wobei Alkyl und Cycloalkyl ihrerseits bis zu dreifach unabhängig voneinander durch Hydroxy oder Mono- oder Di-(C₁-C₆)-alkylamino substituiert sein können, (C₆-C₁₀)-Aryl, 5- bis 10-gliedriges Heteroaryl mit bis zu drei Heteroatomen aus der Reihe N, O und/oder S oder 5- bis 6-gliedriges Heterocyclyl mit bis zu drei Heteroatomen aus der Reihe N, O und/oder S bedeutet, wobei Aryl, Heteroaryl und Heterocyclyl ihrerseits bis zu dreifach unabhängig voneinander durch Halogen, Hydroxy, Cyano, Nitro, Trifluormethyl, Trifluormethoxy, (C₁-C₆)-Alkyl, (C₁-C₆)-Akoxy, (C₁-C₆)-Alkoxycarbonyl oder Mono- oder Di-(C₁-C₆)-alkylamino substituiert sein können,
R⁶ Wasserstoff, (C₁-C₆)-Alkoxycarbonyl oder Carboxyl bedeutet,
- R¹: Wasserstoff, (C₁-C₆)-Alkyl, das seinerseits durch Hydroxy oder (C₁-C₄)-Alkoxy substituiert sein kann, (C₃-C₇)-Cycloalkyl, (C₆-C₁₀)-Aryl, 5- bis 10-gliedriges Heteroaryl mit bis zu zwei Heteroatomen aus der Reihe N, O und/oder S, worin Aryl und Heteroaryl ihrerseits unabhängig voneinander durch Halogen substituiert sein können, oder einen Rest der Formel -NR⁷R⁸ oder -OR⁹ bedeutet,
worin
R⁷ und R⁸ unabhängig voneinander Wasserstoff, (C₆-C₁₀)-Aryl, Adamantyl, (C₁-C₈)-Alkyl, dessen Kette durch ein oder zwei Sauerstoffatome unterbrochen sein kann und das bis zu dreifach unabhängig voneinander durch Hydroxy, Phenyl, Trifluormethyl, (C₃-C₈)-Cycloalkyl, (C₁-C₆)-Alkoxy, Mono- oder Di-(C₁-C₆)-alkylamino, 5- oder 6-gliedriges Heterocyclyl mit bis zu drei Heteroatomen aus der Reihe N, O und/oder S oder durch 5- bis 10-gliedriges Heteroaryl mit bis zu drei Heteroatomen aus der Reihe N, O und/oder S substituiert sein kann, (C₃-C₈)-Cycloalkyl, das bis zu dreifach unabhängig voneinander durch (C₁-C₄)-Alkyl, Hydroxy oder Oxo substituiert sein kann, oder 5-oder 6-gliedriges Heterocyclyl mit bis zu zwei Heteroatomen aus der Reihe N, O und/oder S, wobei N durch Wasserstoff oder (C₁-C₄)-Alkyl substituiert ist, bedeuten,
oder
R⁷ und R⁸ gemeinsam mit dem Stickstoffatom, an das sie gebunden sind, einen 4- bis 7-gliedrigen gesättigten Heterocyclus bilden, der bis zu zwei weitere Heteroatome aus der Reihe N, O und/oder S enthalten kann und gegebenenfalls substituiert ist durch Hydroxy, Oxo oder (C₁-C₆)-Alkyl, welches seinerseits durch Hydroxy substituiert sein kann,
und
R⁹ (C₆-C₁₀)-Aryl, Adamantyl, (C₁-C₈)-Alkyl, dessen Kette durch ein oder zwei Sauerstoffatome unterbrochen sein kann und das bis zu dreifach unabhängig voneinander durch Hydroxy, Phenyl, Trifluormethyl, (C₃-C₈)-Cycloalkyl, (C₁-C₆)-Alkoxy, Mono- oder Di-(C₁-C₆)-alkylamino, 5- odet 6-gliedriges Heterocyclyl mit bis zu drei Heteroatomen aus der Reihe N, O und/oder S oder durch 5- bis 10-gliedriges Heteroaryl mit bis zu drei Heteroatomen aus der Reihe N, O und/oder S substituiert sein kann, (C₃-C₈)-Cycloalkyl, das bis zu dreifach unabhängig voneinander durch (C₁-C₄)-Alkyl, Hydroxy oder Oxo substituiert sein kann, oder 5- oder 6-gliedriges Heterocyclyl mit bis zu zwei Heteroatomen aus der Reihe N, O und/oder S, wobei N durch Wasserstoff oder (C₁-C₄)-Alkyl substituiert ist, bedeuten,
- R³: (C₁-C₈)-Alkyl, dessen Kette durch ein Schwefel- oder Sauerstoffatom oder eine S(O)- oder SO₂-Gruppe unterbrochen sein kann, Phenyl, Benzyl oder 5-oder 6-gliedriges Heteroaryl mit bis zu zwei Heteroatomen aus der Reihe N, O und/oder S bedeutet, worin Phenyl, Benzyl und Heteroaryl bis zu dreifach unabhängig voneinander durch Halogen, Trifluormethyl, Cyano, Nitro, Hydroxy, (C₁-C₆)-Alkyl oder (C₁-C₆)-Alkoxy substituiert sein können,
und
- R⁴: einen Rest der Formel -C(O)-NR¹⁰R¹¹ bedeutet,
worin
R¹⁰ und R¹¹ unabhängig voneinander Wasserstoff oder (C₁-C₆)-Alkyl bedeuten,
und ihre Salze, Hydrate, Hydrate der Salze und Solvate.

Salze der erfindungsgemäßen Verbindungen sind physiologisch unbedenkliche Salze der erfindungsgemäßen Stoffe mit Mineralsäuren, Carbonsäuren oder Sulfonsäuren. Besonders bevorzugt sind z.B. Salze von Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Salze können ebenso physiologisch unbedenkliche Metall- oder Ammoniumsalze der erfindungsgemäßen Verbindungen sein. Besonders bevorzugt sind Alkalimetallsalze (z.B. Natrium- oder Kaliumsalze), Erdalkalisalze (z.B. Magnesium- oder Calciumsalze), sowie Ammoniumsalze, die abgeleitet sind von Ammoniak oder organischen Aminen, wie beispielsweise Ethylamin, Di- bzw. Triethylamin, Di- bzw. Triethanolamin, Dicyclohexylamin, Dimethylaminoethanol, Arginin, Lysin, Ethylendiamin oder 2-Phenylethylamin.

Die erfindungsgemäßen Verbindungen können in Abhängigkeit von dem Substitutionsmuster in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweilige Mischungen. Die Racemformen lassen sich ebenso wie die Diastereomeren in bekannter Weise in die stereoisomer einheitlichen Bestandteile trennen.

Ausserdem umfasst die Erfindung auch Prodrugs der erfindungsgemäßen Verbindungen. Als Prodrugs werden erfindungsgemäß solche Formen der Verbindungen der obigen Formel (I) bezeichnet, welche selbst biologisch aktiv oder inaktiv sein können, jedoch unter physiologischen Bedingungen in die entsprechende biologisch aktive Form überführt werden können (beispielsweise metabolisch oder solvolytisch).

Als "Hydrate" bzw. "Solvate" werden erfindungsgemäß solche Formen der Verbindungen der obigen Formel (I) bezeichnet, welche in festem oder flüssigem Zustand durch Hydratation mit Wasser oder Koordination mit Lösungsmittelmolekülen eine Molekül-Verbindung bzw. einen Komplex bilden. Beispiele für Hydrate sind Sesquihydrate, Monohydrate, Dihydrate oder Trihydrate. Gleichermaßen kommen auch die Hydrate bzw. Solvate von Salzen der erfindungsgemäßen Verbindungen in Betracht.

Halogen steht für Fluor, Chlor, Brom und Iod. Bevorzugt sind Chlor oder Fluor.

(C₁-C₈)-Alkyl steht für einen geradkettigen oder verzweigten Alkykest mit 1 bis 8 Kohlenstoffatomen. Beispielsweise seien genannt: Methyl, Ethyl, n-Propyl, Isopropyl, n-Butyl, Isobutyl, tert.-Butyl, n-Pentyl, n-Hexyl und n-Octyl. Aus dieser Definition leiten sich analog die entsprechenden Alkylgruppen mit weniger Kohlenstoffatomen wie z.B. (C₁-C₆)-Alkyl, (C₁-C₄)-Alkyl und (C₁-C₃)-Alkyl ab. Im Allgemeinen gilt, dass (C₁-C₃)-Alkyl bevorzugt ist.

Aus dieser Definition leitet sich auch die Bedeutung des entsprechenden Bestandteils anderer komplexerer Substituenten ab wie z.B. bei Di-Alkylamino, Mono- oder Di-Alkylamino.

Mono- oder Di-(C₁-C₄)-alkylamino steht für eine Amino-Gruppe mit einem oder mit zwei gleichen oder verschiedenen geradkettigen oder verzweigten Alkylsubstituenten, die jeweils 1 bis 4 Kohlenstoffatome aufweisen. Beispielsweise seien genannt: Methylamino, Ethylamino, n-Propylamino, Isopropylamino, t-Butylamino, *N,N-*Dimethylamino, *N,N*-Diethylamino, *N*-Ethyl-*N*-methylamino, *N*-Methyl-*N*-n-propylamino, *N*-Isopropyl-*N*-n-propylamino und *N*-t-Butyl-*N*-methylamino.

(C₃-C₈)-Cycloalkyl steht für einen cyclischen Alkylrest mit 3 bis 8 Kohlenstoffatomen. Beispielsweise seien genannt: Cyclopropyl, Cyclobutyl, Cyclopentyl, Cyclohexyl, Cycloheptyl oder Cyclooctyl. Aus dieser Definition leiten sich analog die entsprechenden Cycloalkylgruppen mit weniger Kohlenstoffatomen wie z.B. (C₃-C₇)-Cycloalkyl oder (C₃-C₆)-Cycloalkyl ab. Bevorzugt sind Cyclopropyl, Cyclopentyl und Cyclohexyl.

(C₁-C₆)-Alkoxy steht für einen geradkettigen oder verzweigten Alkoxyrest mit 1 bis 6 Kohlenstoffatomen. Beispielsweise seien genannt: Methoxy, Ethoxy, n-Propoxy, Isopropoxy, n-Butoxy, Isobutoxy, tert.-Butoxy, n-Pentoxy und n-Hexoxy. Aus dieser Definition leiten sich analog die entsprechenden Alkoxygruppen mit weniger Kohlenstoffatomen wie z.B. (C₁-C₄)-Alkoxy oder (C₁-C₃)-Alkoxy ab. Im Allgemeinen gilt, dass (C₁-C₃)-Alkoxy bevorzugt ist.

Aus dieser Definition leitet sich auch die Bedeutung des entsprechenden Bestandteils anderer komplexerer Substituenten ab wie z.B. Alkoxycarbonyl, das für einen Alkoxyrest steht, der über eine Carbonylgruppe verknüpft ist.

(C₆-C₁₀)-Aryl steht für einen aromatischen Rest mit 6 bis 10 Kohlenstoffatomen. Beispielsweise seien genannt: Phenyl und Naphthyl.

5- bis 10-gliedriges Heteroaryl mit bis zu 3 Heteroatomen aus der Reihe N, O und/oder S steht für einen mono- oder bicyclischen Heteroaromaten, der über ein Ringkohlenstoffatom des Heteroaromaten, gegebenenfalls auch über ein Ringstickstoffatom des Heteroaromaten, verknüpft ist. Beispielsweise seien genannt: Pyridyl, Pyrimidyl, Pyridazinyl, Pyrazinyl, Thienyl, Furyl, Pyrrolyl, Pyrazolyl, Imidazolyl, Triazolyl, Thiazolyl, Oxazolyl, Oxdiazolyl, Isoxazolyl, Benzofuranyl, Benzothienyl oder Benzimidazolyl. Aus dieser Definition leiten sich analog die entsprechenden Heterocyclen mit weniger Heteroatomen wie z.B. mit bis zu 2 Heteroatomen aus der Reihe N, O und/oder S ab. Im Allgemeinen gilt, dass 5- oder 6-gliedrige aromatische Heterocyclen mit bis zu 2 Heteroatomen aus der Reihe N, O und/oder S wie z. B. Pyridyl, Pyrimidyl, Pyridazinyl, Furyl, Imidazolyl und Thienyl bevorzugt sind.

5- oder 6-gliedriges Heterocyclyl mit bis zu 3 Heteroatomen aus der Reihe N, O und/oder S steht für einen gesättigten oder teilweise ungesättigten Heterocyclus, der über ein Ringkohlenstoffatom oder ein Ringstickstoffatom verknüpft ist. Beispielsweise seien genannt: Tetrahydrofuryl, Pyrrolidinyl, Pyrrolinyl, Dihydropyridinyl, Piperidinyl, Piperazinyl, Morpholinyl, Thiomorpholinyl. Bevorzugt sind gesättigte Heterocyclen, insbesondere Piperidinyl, Piperazinyl, Morpholinyl und Pyrrolidinyl.

Die erfindungsgemäßen Verbindungen der Formel (I) können in mindestens acht verschiedenen Konfigurationen vorliegen, wobei die folgenden vier unterschiedlichen Konfigurationen (Ia) bis (Id) bevorzugt sind:

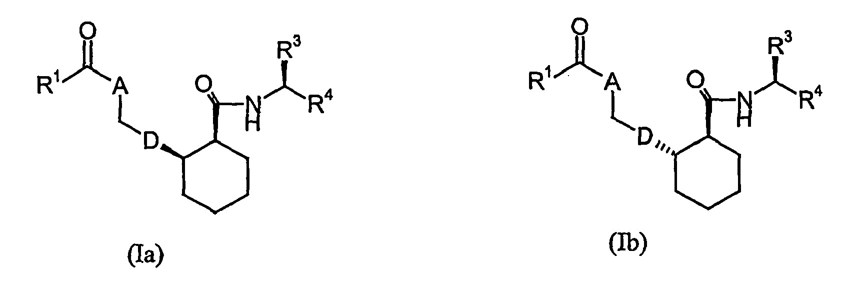

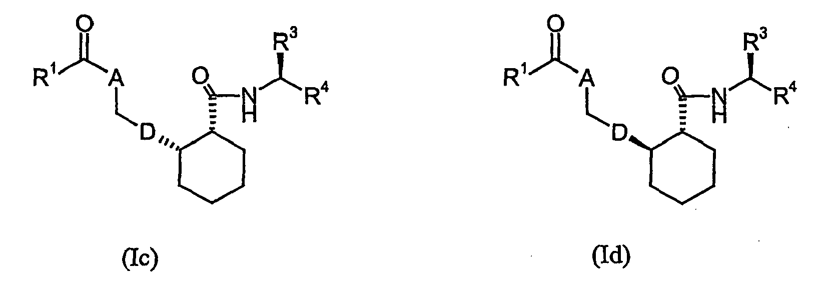

Besonders bevorzugt ist die Konfiguration (Id).

Bevorzugt sind ferner erfindungsgemäße Verbindungen der Formel (I),
worin
- D: einen Rest 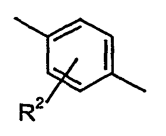 bedeutet,
worin
R² Wasserstoff, Halogen, Hydroxy, Carboxyl, Cyano, Nitro, Trifluormethyl, Trifluormethoxy, (C₁-C₆)-Alkyl, (C₁-C₆)-Alkoxy oder (C₁-C₆)-Alkoxycarbonyl bedeutet,
- A: ein Sauerstoffatom oder eine Gruppe der Formel N-R⁵ oder CH-R⁶ bedeutet,
worin
R⁵ Wasserstoff, (C₁-C₆)-Alkyl, (C₃-C₇)-Cycloalkyl, wobei Alkyl und Cycloalkyl ihrerseits bis zu dreifach unabhängig voneinander durch Hydroxy oder Mono- oder Di-(C₁-C₆)-alkylamino substituiert sein können, (C₆-C₁₀)-Aryl, 5- bis 10-gliedriges Heteroaryl mit bis zu drei Heteroatomen aus der Reihe N, O und/oder S oder 5- bis 6-gliedriges Heterocyclyl mit bis zu drei Heteroatomen aus der Reihe N, O und/oder S bedeutet, wobei Aryl, Heteroaryl und Heterocyclyl ihrerseits bis zu dreifach unabhängig voneinander durch Halogen, Hydroxy, Cyano, Nitro, Trifluormethyl, Trifluormethoxy, (C₁-C₆)-Alkyl, (C₁-C₆)-Alkoxy, (C₁-C₆)-Alkoxycarbonyl oder Mono- oder Di-(C₁-C₆)-alkylamino substituiert sein können,
R⁶ Wasserstoff, (C₁-C₆)-Alkoxycarbonyl oder Carboxyl bedeutet,
- R¹: Wasserstoff, (C₁-C₆)-Alkyl, das seinerseits durch Hydroxy oder (C₁-C₄)-Alkoxy substituiert sein kann, (C₃-C₇)-Cycloalkyl, (C₆-C₁₀)-Aryl, 5- bis 10-gliedriges Heteroaryl mit bis zu zwei Heteroatomen aus der Reihe N, O und/oder S, worin Aryl und Heteroaryl ihrerseits unabhängig voneinander durch Halogen substituiert sein können, oder einen Rest der Formel -NR⁷R⁸ oder -OR⁹ bedeutet,
worin
R⁷ und R⁸ unabhängig voneinander Wasserstoff, (C₆-C₁₀)-Aryl, Adamantyl, (C₁-C₈)-Alkyl, dessen Kette durch ein oder zwei Sauerstoffatome unterbrochen sein kann und das bis zu dreifach unabhängig voneinander durch Hydroxy, Phenyl, Trifluormethyl, (C₃-C₈)-Cycloalkyl, (C₁-C₆)-Alkoxy, Mono- oder Di-(C₁-C₆)-alkylamino, 5-oder 6-gliedriges Heterocyclyl mit bis zu drei Heteroatomen aus der Reihe N, O und/oder S oder durch 5- bis 10-gliedriges Heteroaryl mit bis zu drei Heteroatomen aus der Reihe N, O und/oder S substituiert sein kann, (C₃-C₈)-Cycloalkyl, das bis zu dreifach unabhängig voneinander durch (C₁-C₄)-Alkyl, Hydroxy oder Oxo substituiert sein kann, oder 5- oder 6-gliedriges Heterocyclyl mit bis zu zwei Heteroatomen aus der Reihe N, O und/oder S, wobei N durch Wasserstoff oder (C₁-C₄)-Alkyl substituiert ist, bedeuten,
oder
R⁷ und R⁸ gemeinsam mit dem Stickstoffatom, an das sie gebunden sind, einen 4- bis 7-gliedrigen gesättigten Heterocyclus bilden, der bis zu zwei weitere Heteroatome aus der Reihe N, O und/oder S enthalten kann und gegebenenfalls substituiert ist durch Hydroxy, Oxo oder (C₁-C₆)-Alkyl, welches seinerseits durch Hydroxy substituiert sein kann,
und
R⁹ (C₆-C₁₀)-Aryl, Adamantyl, (C₁-C₈)-Alkyl, dessen Kette durch ein oder zwei Sauerstoffatome unterbrochen sein kann und das bis zu dreifach unabhängig voneinander durch Hydroxy, Phenyl, Trifluormethyl, (C₃-C₈)-Cycloalkyl, (C₁-C₆)-Alkoxy, Mono- oder Di-(C₁-C₆)-alkyl-amino, 5- odet 6-gliedriges Heterocyclyl mit bis zu drei Heteroatomen aus der Reihe N, O und/oder S oder durch 5- bis 10-gliedriges Heteroaryl mit bis zu drei Heteroatomen aus der Reihe N, O und/oder S substituiert sein kann, (C₃-C₈)-Cycloalkyl, das bis zu dreifach unabhängig voneinander durch (C₁-C₄)-Alkyl, Hydroxy oder Oxo substituiert sein kann, oder 5- oder 6-gliedriges Heterocyclyl mit bis zu zwei Heteroatomen aus der Reihe N, O und/oder S, wobei N durch Wasserstoff oder (C₁-C₄)-Alkyl substituiert ist, bedeuten,
- R³: (C₁-C₈)-Alkyl, dessen Kette durch ein Schwefelatom oder eine S(O)- oder SO₂-Gruppe unterbrochen sein kann, Phenyl, Benzyl oder 5- oder 6-gliedriges Heteroaryl mit bis zu zwei Heteroatomen aus der Reihe N, O und/oder S bedeutet, worin Phenyl, Benzyl und Heteroaryl bis zu dreifach unabhängig voneinander durch Halogen, Trifluormethyl, Cyano, Nitro, Hydroxy, (C₁-C₆)-Alkyl oder (C₁-C₆)-Alkoxy substituiert sein können,
und
- R⁴: einen Rest der Formel -C(O)-NR¹⁰R¹¹ bedeutet,
worin
R¹⁰ und R¹¹ unabhängig voneinander Wasserstoff oder (C₁-C₆)-Alkyl bedeuten,
und ihre Salze, Hydrate, Hydrate der Salze und Solvate.

Besonders bevorzugt sind erfindungsgemäße Verbindungen der Formel (I),
worin
- D: einen Rest 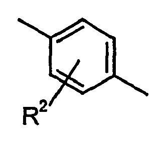 bedeutet,
worin
R² Wasserstoff, Chlor oder Fluor bedeutet,
- A: ein Sauerstoffatom oder eine Gruppe der Formel N-R⁵ bedeutet,
worin
R⁵ Wasserstoff, (C₁-C₆)-Alkyl, das seinerseits bis zu zweifach durch Hydroxy substituiert sein kann, (C₃-C₇)-Cycloalkyl, Phenyl oder 5-oder 6-gliedriges Heteroaryl mit bis zu drei Heteroatomen aus der Reihe N, O und/oder S bedeutet, wobei Phenyl und Heteroaryl ihrerseits bis zu zweifach unabhängig voneinander durch Halogen, Cyano, Trifluormethyl, Trifluormethoxy, (C₁-C₄)-Alkyl, (C₁-C₄)-Alkoxy oder Di-(C₁-C₄)-alkylamino substituiert sein können,
- R¹: Wasserstoff, (C₁-C₆)-Alkyl, das seinerseits durch Hydroxy oder (C₁-C₄)-Alkoxy substituiert sein kann, (C₃-C₇)-Cycloalkyl, Phenyl, 5- bis 6-gliedriges Heteroaryl mit bis zu zwei Heteroatomen aus der Reihe N, O und/oder S, worin Phenyl und Heteroaryl ihrerseits unabhängig voneinander durch Halogen substituiert sein können, oder einen Rest der Formel -NR⁷R⁸ oder -OR⁹ bedeutet,
worin
R⁷ und R⁸ unabhängig voneinander Wasserstoff, Phenyl, Adamantyl, (C₁-C₆)-Alkyl, dessen Kette durch ein oder zwei Sauerstoffatome unterbrochen sein kann und das bis zu zweifach unabhängig voneinander durch Hydroxy, Phenyl, Trifluormethyl, (C₃-C₆)-Cycloalkyl, (C₁-C₄)-Alkoxy, Mono- oder Di-(C₁-C₄)-alkylamino, 5- bis 6-gliedriges Heterocyclyl mit bis zu zwei Heteroatomen aus der Reihe N und/oder O oder durch 5- bis 6-gliedriges Heteroaryl mit bis zu drei Heteroatomen aus der Reihe N, O und/oder S substituiert sein kann, (C₃-C₈)-Cycloalkyl, das bis zu zweifach durch Hydroxy substituiert sein kann, oder 5- bis 6-gliedriges Heterocyclyl mit bis zu zwei Heteroatomen aus der Reihe N, O und/oder S, wobei N durch Wasserstoff oder (C₁-C₄)-Alkyl substituiert ist, bedeuten,
oder
R⁷ und R⁸ gemeinsam mit dem Stickstoffatom, an das sie gebunden sind, einen 4- bis 7-gliedrigen gesättigten Heterocyclus bilden, der bis zu zwei weitere Heteroatome aus der Reihe N, O und/oder S enthalten kann und gegebenenfalls substituiert ist durch Hydroxy, Oxo oder (C₁-C₆)-Alkyl, welches seinerseits durch Hydroxy substituiert sein kann,
und
R⁹ Phenyl, Adamantyl, (C₁-C₆)-Alkyl, dessen Kette durch ein oder zwei Sauerstoffatome unterbrochen sein kann und das bis zu zweifach unabhängig voneinander durch Hydroxy, Phenyl, Trifluormethyl, (C₃-C₆)-Cycloalkyl, (C₁-C₃)-Alkoxy, Mono- oder Di-(C₁-C₄)-alkyl-amino, 5- bis 6-gliedriges Heterocyclyl mit bis zu zwei Heteroatomen aus der Reihe N und/oder O oder durch 5- bis 6-gliedriges Heteroaryl mit bis zu drei Heteroatomen aus der Reihe N, O und/oder S substituiert sein kann, (C₃-C₈)-Cycloalkyl, das bis zu zweifach durch Hydroxy substituiert sein kann, oder 5- bis 6-gliedriges Heterocyclyl mit bis zu zwei Heteroatomen aus der Reihe N, O und/oder S, wobei N durch Wasserstoff oder (C₁-C₄)-Alkyl substituiert ist, bedeuten,
- R³: (C₁-C₈)-Alkyl, dessen Kette durch ein Schwefelatom oder eine S(O)- oder SO₂-Gruppe unterbrochen sein kann, Phenyl, Benzyl oder 5- bis 6-gliedriges Heteroaryl mit bis zu zwei Heteroatomen aus der Reihe N, O und/oder S bedeutet, worin Phenyl, Benzyl und Heteroaryl bis zu zweifach unabhängig voneinander durch Halogen, Trifluormethyl, Cyano, (C₁-C₃)-Alkyl, (C₁-C₃)-Alkoxy oder Hydroxy substituiert sein können,
und
- R⁴: einen Rest der Formel -C(O)-NR¹⁰R¹¹ bedeutet,
worin
R¹⁰ und R¹¹ unabhängig voneinander Wasserstoff oder (C₁-C₆)-Alkyl bedeuten,
und ihre Salze, Hydrate, Hydrate der Salze und Solvate.

Ganz besonders bevorzugt sind erfindungsgemäße Verbindungen der Formel (I),
worin
- D: einen Rest 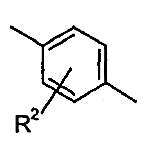 bedeutet,
worin
R² Wasserstoff bedeutet,
- A: ein Sauerstoffatom oder eine Gruppe der Formel N-R⁵ bedeutet,
worin
R⁵ Wasserstoff, (C₁-C₆)-Alkyl, das seinerseits bis zu zweifach durch Hydroxy substituiert sein kann, (C₃-C₇)-Cycloalkyl, Phenyl oder 5- bis 6-gliedriges Heteroaryl mit bis zu drei Heteroatomen aus der Reihe N, O und/oder S bedeutet, wobei Phenyl und Heteroaryl ihrerseits bis zu zweifach unabhängig voneinander durch Fluor, Chlor, Cyano, Trifluormethyl, Trifluormethoxy, (C₁-C₃)-Alkyl, (C₁-C₃)-Alkoxy oder Di-(C₁-C₃)-alkylamino substituiert sein können,
- R¹: (C₁-C₄)-Alkyl oder einen Rest der Formel -NR⁷R⁸ bedeutet,
worin
R⁷ und R⁸ unabhängig voneinander Wasserstoff, Phenyl, Adamantyl, (C₁-C₄)-Alkyl, dessen Kette durch ein oder zwei Sauerstoffatome unterbrochen sein kann und das bis zu zweifach unabhängig voneinander durch Hydroxy, Phenyl, Trifluormethyl, (C₃-C₆)-Cycloalkyl, (C₁-C₃)-Alkoxy, Mono- oder Di-(C₁-C₃)-alkylamino, 5- bis 6-gliedriges Heterocyclyl mit bis zu zwei Heteroatomen aus der Reihe N und/oder O oder durch 5- bis 6-gliedriges Heteroaryl mit bis zu drei Heteroatomen aus der Reihe N, O und/oder S substituiert sein kann, (C₃-C₈)-Cycloalkyl, das bis zu zweifach durch Hydroxy substituiert sein kann, oder 5- bis 6-gliedriges Heterocyclyl mit bis zu zwei Heteroatomen aus der Reihe N, O und/oder S, wobei N durch Wasserstoff oder (C₁-C₄)-Alkyl substituiert ist, bedeuten,
oder
R⁷ und R⁸ gemeinsam mit dem Stickstoffatom, an das sie gebunden sind, einen 4- bis 7-gliedrigen gesättigten Heterocyclus bilden, der bis zu zwei weitere Heteroatome aus der Reihe N, O und/oder S enthalten kann und gegebenenfalls substituiert ist durch Hydroxy, Oxo oder (C₁-C₆)-Alkyl, welches seinerseits durch Hydroxy substituiert sein kann,
- R³: (C₁-C₈)-Alkyl, dessen Kette durch ein Schwefelatom oder eine S(O)- oder SO₂-Gruppe unterbrochen sein kann, Phenyl, Benzyl oder 5- bis 6-gliedriges Heteroaryl mit bis zu zwei Heteroatomen aus der Reihe N, O und/oder S bedeutet, worin Phenyl, Benzyl und Heteroaryl bis zu zweifach unabhängig voneinander durch Halogen, Trifluormethyl, Cyano, (C₁-C₃)-Alkyl, (C₁-C₃)-Alkoxy oder Hydroxy substituiert sein können,
und
- R⁴: einen Rest der Formel -C(O)-NR¹⁰R¹¹ bedeutet,
worin
R¹⁰ und R¹¹ unabhängig voneinander Wasserstoff, Methyl oder Ethyl bedeuten,
und ihre Salze, Hydrate, Hydrate der Salze und Solvate.

Insbesondere ganz besonders bevorzugt sind Verbindungen der Formel (I),
worin
- D: einen Rest 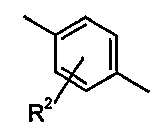 bedeutet,
worin
R² Wasserstoff bedeutet,
- A: ein Sauerstoffatom oder eine Gruppe der Formel N-R⁵ bedeutet,
worin
R⁵ (C₃-C₇)-Cycloalkyl, Phenyl, das seinerseits durch Fluor substituiert sein kann, oder Pyridyl bedeutet,
- R¹: Methyl oder einen Rest der Formel -NR⁷R⁸ bedeutet,
worin
R⁷ und R⁸ unabhängig voneinander (C₁-C₄)-Alkyl, das ein- oder zweifach durch Hydroxy substituiert sein kann, bedeuten,
oder
R⁷ und R⁸ gemeinsam mit dem Stickstoffatom, an das sie gebunden sind, einen 5- bis 6-gliedrigen gesättigten Heterocyclus bilden, der ein weiteres Heteroatom O oder N enthalten kann, wobei N durch Wasserstoff oder (C₁-C₃)-Alkyl, das seinerseits durch Hydroxy substituiert sein kann, substituiert ist,
- R³: Phenyl, das gegebenenfalls in para-Position durch Fluor substituiert sein kann, oder Pyridyl bedeutet,
und
- R⁴: einen Rest der Formel -C(O)-NR¹⁰R¹¹ bedeutet,
worin
R¹⁰ und R¹¹ Wasserstoff bedeuten,
und ihre Salze, Hydrate, Hydrate der Salze und Solvate.

Ebenfalls insbesondere ganz besonders bevorzugt sind:
(1*R*,2*R*)-*N*-[(1*S*)-2-Amino-1-(4-fluorphenyl)-2-oxoethyl]-2-(4-{[{[ethyl(2-hydroxyethyl)amino]carbonyl}(4-fluorphenyl)amino]methyl}phenyl)cyclohexancarbonsäureamid 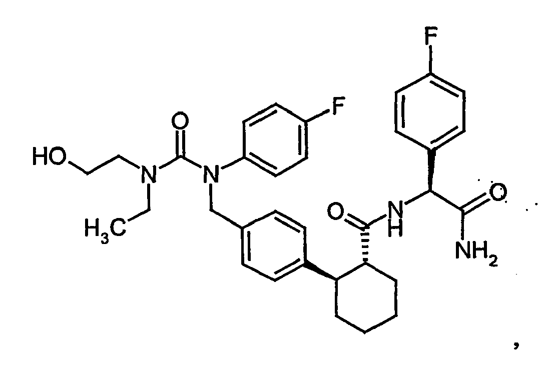
(1*R*,2*R*)-*N*-[(1*S*)-2-Amino-2-oxo-1-phenylethyl]-2-(4-{[[(dimethylamino)carbonyl]-(phenyl)amino]methyl}phenyl)cyclohexancarbonsäureamid 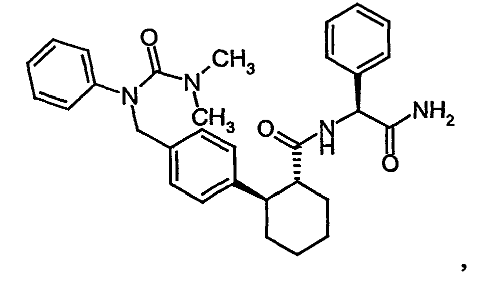
(1*R*,2*R*)-*N*-[(1*S*)-2-Amino-2-oxo-1-phenylethyl]-2-[4-({cyclopropyl[(dimethylamino)carbonyl]amino}methyl)phenyl]cyclohexancarbonsäureamid 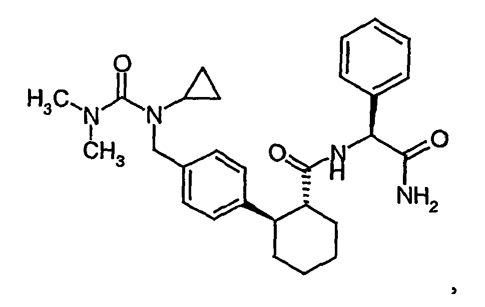
(1*R*,2*R*)-*N*-[(1*S*)-2-Amino-2-oxo-1-phenylethyl]-2-(4-{[[(diethylamino)carbonyl](2-pyridinyl)amino]methyl}phenyl)cyclohexancarbonsäureamid 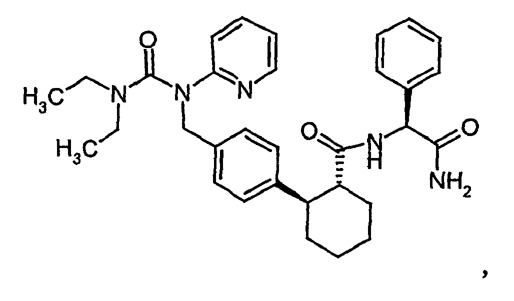
*N*-{4-[(1*R*,2*R*)-2-({[(1*S*)-2-Amino-2-oxo-1-phenylethyl]amino}carbonyl)cyclohexyl]benzyl}-*N*-phenyl-4-morpholincarbonsäureamid 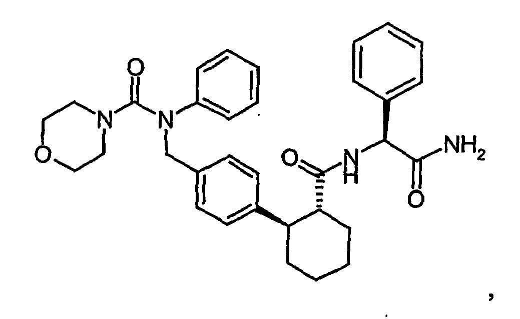
(*S*)-N-{{(1*R*,2*R*)-2-(4-{[{[2-Hydroxyethylamino]carbonyl}(phenyl)amino]methyl}-phenyl)cyclohex-1-yl}carbonyl}-phenylglycinamid 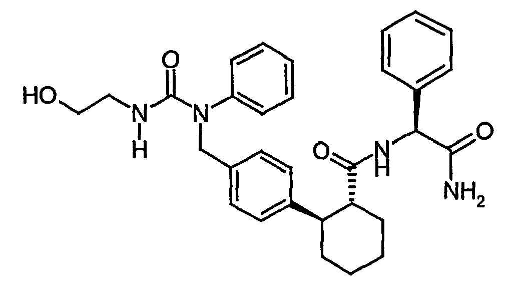
(1*R*,2*R*)-2-(4-{[Acetyl(2-pyridinyl)amino]methyl}phenyl)-N-[(1*S*)-2-amino-2-oxo-1-phenylethyl]cyclohexancarboxamid 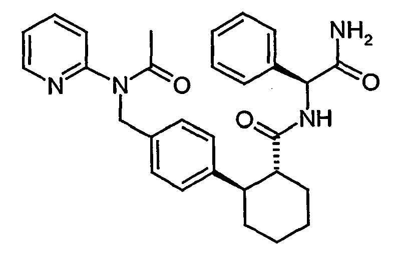
(1*R*,2*R*)-*N*-[(1*S*)-2-Amino-1-phenyl-2-oxoethyl]-2-(4-{[{[ethyl(2-hydroxyethyl)-amino]carbonyl}(phenyl)amino]methyl}phenyl)cyclohexancarbonsäureamid 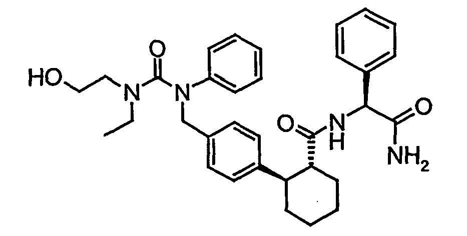
4-[(1*R*,2*R*)-2-({[(1*S*)-2-Amino-1-(4-fluorphenyl)-2-oxoethyl]amino}carbonyl)cyclohexyl]benzyl 4-(2-hydroxyethyl)-1-piperazincarbamat 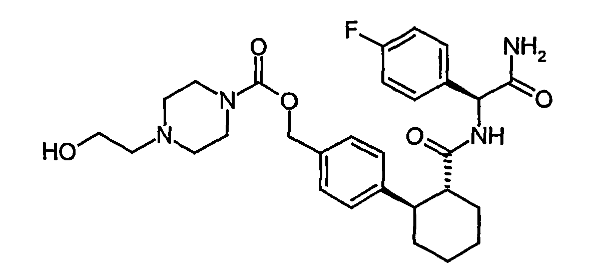
4-[(1*R*,2*R*)-2-({[(1S)-2-Amino-1-phenyl-2-oxoethyl]amino}carbonyl)cyclohexyl]-benzyl-4-(2-hydroxyethyl)-1-piperazincarbamat 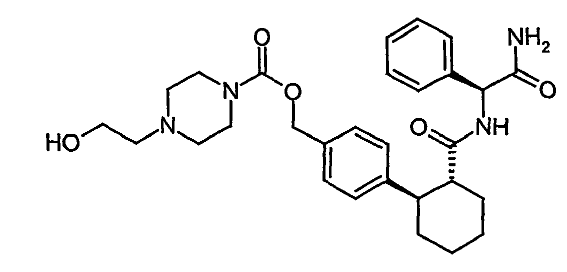
und ihre Salze, Hydrate, Hydrate der Salze und Solvate.

Außerdem wurde ein Verfahren zur Herstellung der erfindungsgemäßen Verbindungen der Formel (I) gefunden, bei dem man
[A] Verbindungen der Formel (II) 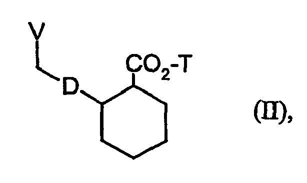 in welcher
   - D: die oben angegebene Bedeutung hat,
   - T: für (C₁-C₄)-Alkyl, vorzugsweise für Methyl oder tert.-Butyl steht,
   und
   - V: für eine geeignete Abgangsgruppe, wie beispielsweise Halogen, Mesylat oder Tosylat, vorzugsweise für Brom steht,
   zunächst durch Umsetzung mit Verbindungen der Formel (III)

   B-H (III),

   in welcher
   - B: für 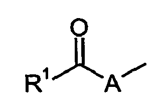 oder
   gegebenenfalls für den Fall, dass R¹ für OR⁹ steht,
   für 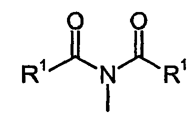 steht,
   und
   R¹ und A die oben angegebenen Bedeutungen haben, wobei gegebenenfalls vorhandene Amino- und Hydroxyfunktionen gegebenenfalls durch übliche Amino- bzw. Hydroxyschutzgruppen blockiert sind,
   in die Verbindungen der Formel (IV) 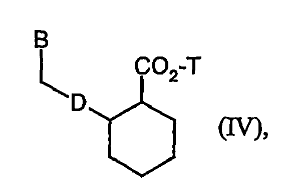 in welcher B, D und T die oben angegebene Bedeutung haben,
   überführt,
   das erhaltene Reaktionsprodukt in einem nächsten Schritt mit Säuren oder Basen in die entsprechenden Carbonsäuren der Formel (V) 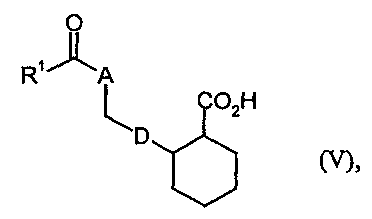 in welcher
   R¹, A und D die oben angegebene Bedeutung haben,
   überführt,
   diese gegebenenfalls aktiviert, insbesondere durch Überführung in ein entsprechendes Carbonsäurederivat wie Carbonsäurehalogenid, Carbonsäureanhydrid oder Carbonsäureester,
   und abschließend nach bekannten Methoden mit Verbindungen der Formel (VI) oder deren Salzen 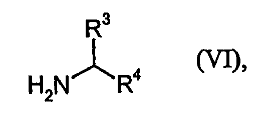 in welcher
   R³ und R⁴ die oben angegebene Bedeutung haben,
   in inerten Lösemitteln umsetzt
   oder
[B] für den Fall, dass A ein Sauerstoffatom oder NR⁵ bedeutet,
   Verbindungen der Formel (VII) 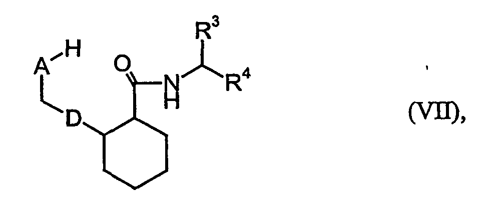 in welcher
   D, R³ und R⁴ die oben angegebenen Bedeutungen haben,
   und
   - A: ein Sauerstoffatom oder eine Gruppe der Formel N-R⁵ bedeutet,
   wobei R⁵ die oben angegebene Bedeutung hat,
gegebenenfalls in Anwesenheit einer Base
entweder mit Verbindungen der Formel (VTII) 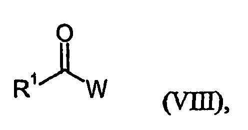 in welcher
- R¹: die oben angegebene Bedeutung hat und W für eine geeignete Abgangsgruppe, wie beispielsweise das entsprechende symmetrische Anhydrid oder ein Halogen, vorzugsweise Chlor, steht
oder
mit einem Phosgenäquivalent wie beispielsweise Disuccinimidylcarbonat und anschließend mit Verbindungen der Formel (IX)

R⁷R⁸NH (IX),

in welcher
R⁷ und R⁸ die oben angegebenen Bedeutungen haben,
oder
mit einem Isocyanat der Formel (X)

R⁷NCO (X)

in welcher
- R⁷: die oben angegebenen Bedeutungen hat,
umsetzt.

Die gemäß der Verfahrensvariante [A] oder [B] erhaltenen Verbindungen der Formel (I) können gegebenenfalls anschließend durch Umsetzung z.B. mit einer Säure in die entsprechenden Salze überführt werden.

Die Herstellung der Verbindungen der entsprechenden diastereomeren und enantiomeren Formen erfolgt entsprechend, und zwar entweder unter Verwendung enantiomeren- oder diastereomerenreiner Ausgangsstoffe oder aber durch nachträgliche Trennung der gebildeten Racemate mit üblichen Methoden (z.B. Racematspaltung, Chromatographie an chiralen Säulen etc.).

Das erfindungsgemäße Verfahren kann durch folgende Formelschemata beispielhaft erläutert werden:

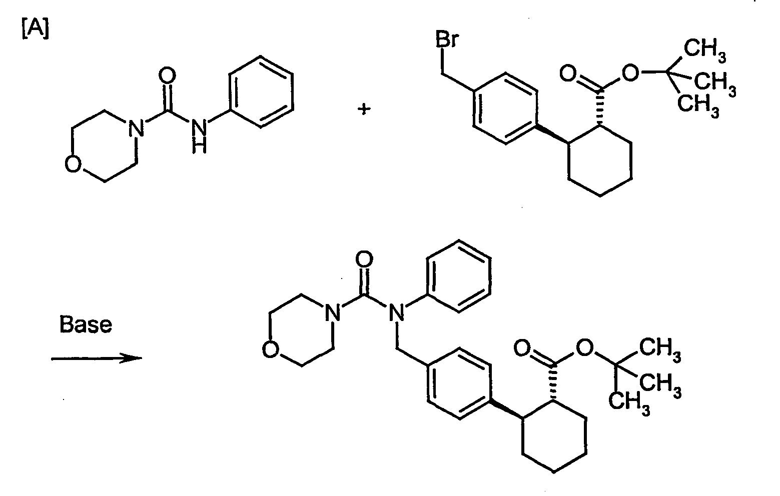

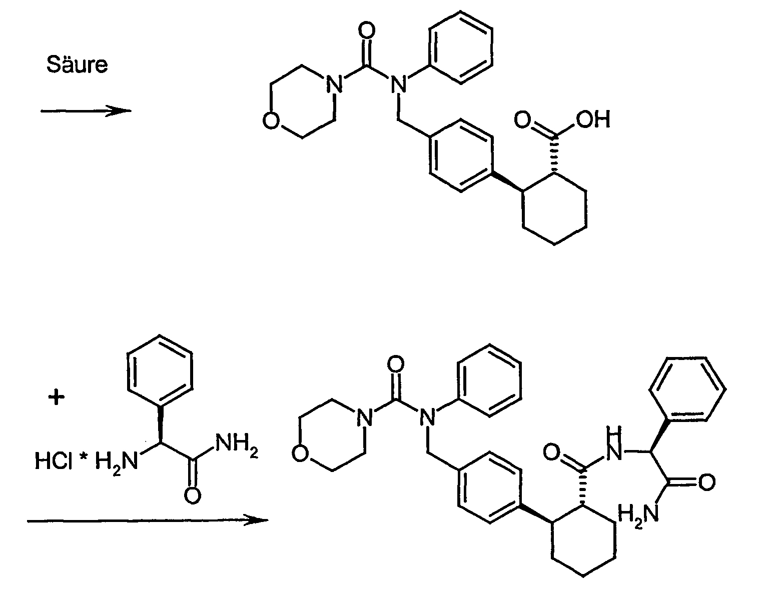

Für den Fall, dass R¹ für OR⁹ steht, ist die folgende Synthesesequenz ebenfalls möglich:

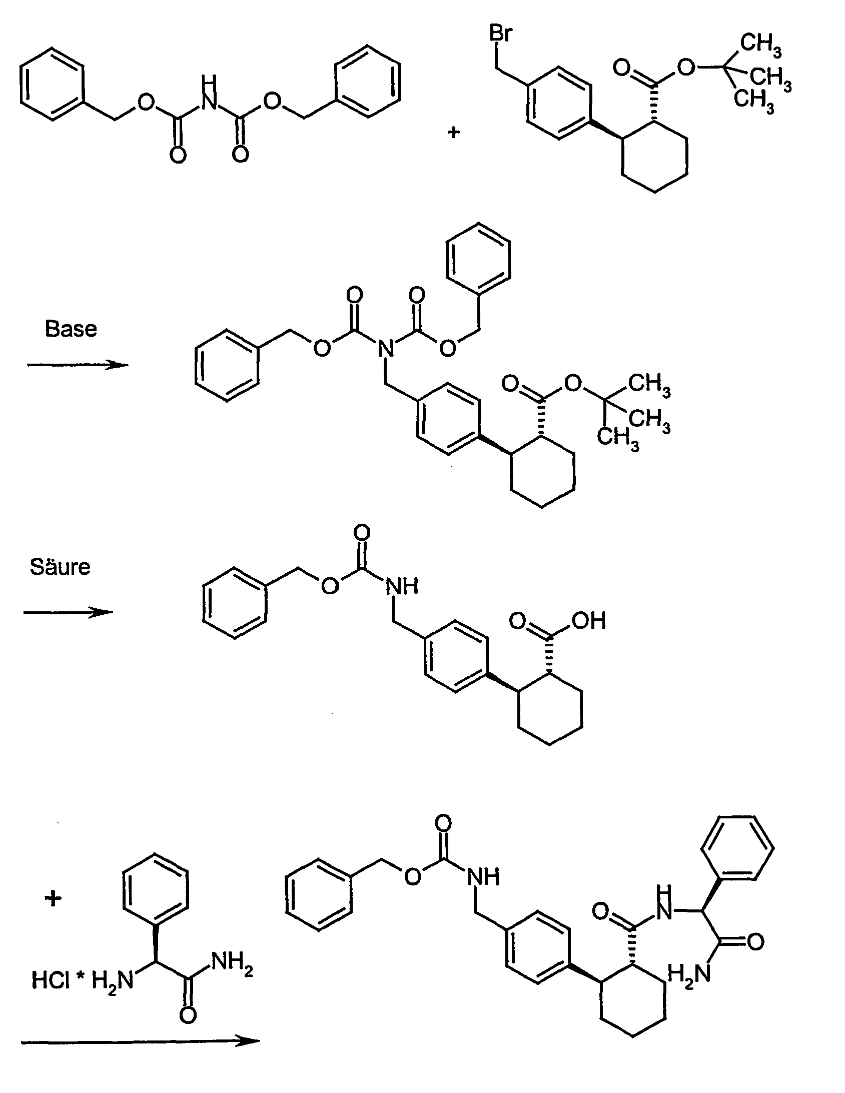

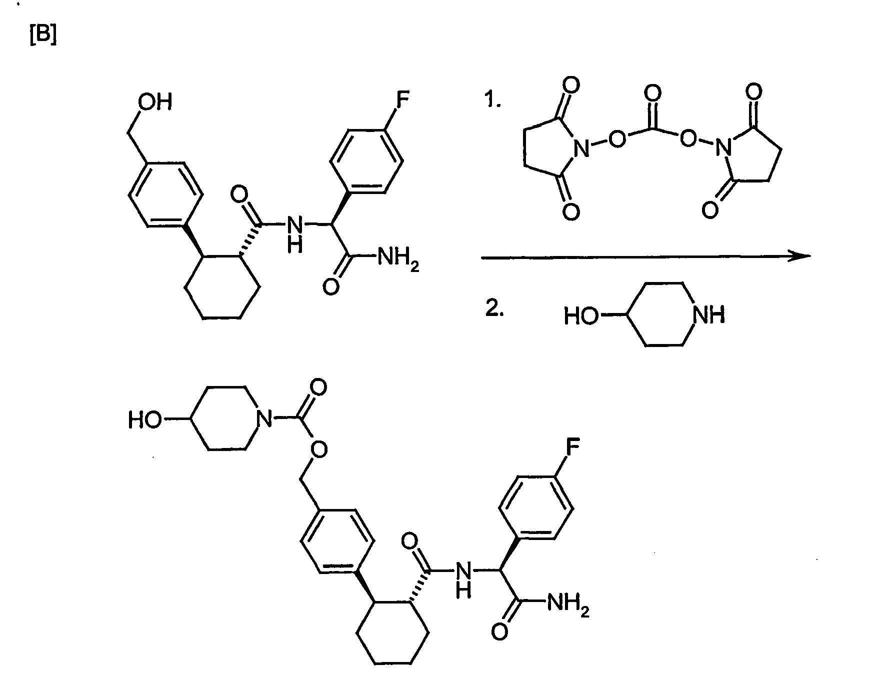

Das erfindungsgemäße Verfahren wird im Allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Übliche Aminoschutzgruppen im Rahmen der Erfindung sind die in der PeptidChemie verwendeten Aminoschutzgruppen.

Hierzu gehören bevorzugt: Benzyloxycarbonyl, 3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitro-4,5-dimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert.-Butoxycarbonyl, Allyloxycarbonyl, Vinyloxycarbonyl, 2-Nitrobenzyloxycarbonyl, 3,4,5-Trimethoxybenzyloxycarbonyl, Cyclohexoxycarbonyl, 1,1-Dimethylethoxycarbonyl, Adamantylcarbonyl, Phthaloyl, 2,2,2-Trichlorethoxycarbonyl, 2,2,2-Trichlor-tert.-butoxycarbonyl, Menthyloxycarbonyl, Phenoxycarbonyl, 4-Nitrophenoxycarbonyl, Fluorenyl-9-methoxycarbonyl, Formyl, Acetyl, Propionyl, Pivaloyl, 2-Chloracetyl, 2-Bromacetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl oder 4-Nitrophenyl. Eine bevorzugte Schutzgruppe für primäre Amine ist Phthalimid. Bevorzugte Schutzgruppen für sekundäre Amine sind Benzyloxycarbonyl und tert.-Butoxycarbonyl.

Die Abspaltung der Aminoschutzgruppen erfolgt in an sich bekannter Weise, indem man beispielsweise unter hydrogenolytischen, sauren oder basischen Bedingungen, bevorzugt mit Säuren, wie beispielsweise Chlorwasserstoffsäure oder Trifluoressigsäure in inerten Lösemitteln wie Ether, Dioxan und Methylenchlorid arbeitet.

Eine übliche Hydroxyschutzgruppe im Rahmen der oben angegebenen Definition steht im Allgemeinen für eine Schutzgruppe aus der Reihe: Trimethylsilyl, Triethylsilyl, Triisopropylsilyl, tert.-Butyl-dimethylsilyl, tert.-Butyldiphenylsilyl, Dimethylhexylsilyl, Dimethylthexylsilyl, Trimethylsilylethoxycarbonyl, Benzyl, Triphenylmethyl(Trityl), Monomethoxytrityl (MMTr), Dimethyloxytrityl (DMTr), Benzyloxycarbonyl, 2-Nitrobenzyl, 4-Nitrobenzyl, 2-Nitrobenzyloxycarbonyl, 4-Nitrobenzyloxycarbonyl, tert.-Butyloxycarbonyl, 4-Methoxybenzyl, 4-Methoxybenzyloxycarbonyl, Formyl, Acetyl, Trichloracetyl, 2,2,2-Trichlorethoxycarbonyl, 2,4-Dimethoxybenzyl, 2,4-Dimethoxybenzyloxycarbonyl, Methoxymethyl, Methylthiomethyl, Methoxyethoxymethyl, [2-(Trimethylsilyl)ethoxy]-methyl, 2-(Methylthiomethoxy)ethoxycarbonyl, Tetrahydropyranyl, Benzoyl, N-Succinimid, 4-Methylbenzoyl, 4-Nitrobenzoyl, 4-Fluorbenzoyl, 4-Chlorbenzoyl oder 4-Methoxybenzoyl. Bevorzugt ist tert.-Butyl-dimethylsilyl.

Die Abspaltung der Hydroxyschutzgruppe erfolgt in an sich bekannter Weise z.B. durch Säure, Base oder durch Zugabe von Tetrabutylammoniumfluorid.

Als Lösemittel für das Verfahren eignen sich übliche organische Lösemittel, die sich unter den Reaktionsbedingungen nicht verändern. Hierzu gehören Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Pyridin, Dimethylsulfoxid, Dimethylformamid, N,N'-Dimethylpropylenharnstoff (DMPU), N-Methylpyrrolidon (NMP), Acetonitril, Aceton oder Nitromethan. Ebenso ist es möglich, Gemische der genannten Lösemittel zu verwenden.

Als Basen für das erfindungsgemäße Verfahren können im Allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat, Kaliumcarbonat oder Cäsiumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.-butylat, oder organische Amine wie Triethylamin, oder Heterocyclen wie 1,4-Diazabicyclo[2.2.2]octan (DABCO), 1,8-Diazabicyclo[5.4.0]undec-7-en (DBU), 1,5-Diazabicyclo [4.3.0]non-5-en (DBN), Pyridin, Diaminopyridin, N-Methylpiperidin oder N-Methyl-morpholin. Es ist auch möglich, als Basen Alkalimetalle wie Natrium oder deren Hydride wie Natriumhydrid einzusetzen.

Bevorzugte Lösemittel für den Verfahrensschritt [A] (II) + (III) → (IV) sind Diethylether, Tetrahydrofuran und Dimethylformamid. Besonders bevorzugt ist Dimethylformamid.

Bevorzugte Basen für den Verfahrensschritt [A] (II) + (III) → (IV) sind Natriumhydrid und Natriumhydroxid.

Im Allgemeinen setzt man die Base in einer Menge von 0,05 mol bis 10 mol, bevorzugt von 1 Mol bis 2 Mol bezogen auf 1 Mol der Verbindung der Formel (II) ein.

Der erfindungsgemäße Verfahrensschritt [A] (II) + (III) → (IV) wird im Allgemeinen in einem Temperaturbereich von -20°C bis +100°C, insbesondere von -20°C bis +80°C, bevorzugt von 0°C bis +80°C, durchgeführt.

Die Hydrolyse der Carbonsäureester im Verfahrensschritt [A] (IV) → (V) erfolgt nach üblichen Methoden, indem man die Ester in inerten Lösemitteln mit Basen behandelt, wobei die zunächst entstehenden Salze durch Behandeln mit Säure in die freien Carbonsäuren überführt werden. Im Falle der t-Butylester erfolgt die Hydrolyse bevorzugt mit Säuren.

Als Lösemittel eignen sich für die Hydrolyse der Carbonsäureester Wasser oder die für eine Esterspaltung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, Dimethylformamid, Dichlormethan oder Dimethylsulfoxid. Ebenso ist es möglich, Gemische der genannten Lösemittel einzusetzen. Bevorzugt sind Wasser/Tetrahydrofuran und im Falle der Umsetzung mit Trifluoressigsäure Dichlormethan sowie im Falle von Chlorwasserstoff Tetrahydrofuran, Diethylether, Dioxan oder Wasser.

Als Basen eignen sich für die Hydrolyse die üblichen anorganischen Basen. Hierzu gehören bevorzugt Alkalihydroxide oder Erdalkalihydroxide wie beispielsweise Natriumhydroxid, Lithiumhydroxid, Kaliumhydroxid oder Bariumhydroxid, oder Alkalicarbonate wie Natrium- oder Kaliumcarbonat oder Natriumhydrogencarbonat. Besonders bevorzugt werden Natriumhydroxid oder Lithiumhydroxid eingesetzt.

Als Säuren eignen sich im Allgemeinen Trifluoressigsäure, Schwefelsäure, Chlorwasserstoff, Bromwasserstoff und Essigsäure oder deren Gemisch gegebenenfalls unter Zusatz von Wasser. Bevorzugt sind Chlorwasserstoff oder Trifluoressigsäure im Falle der tert.-Butylester und Salzsäure im Falle der Methylester.

Bei der Durchführung der Hydrolysen wird die Base oder die Säure im Allgemeinen in einer Menge von 1 bis 100 mol, bevorzugt von 1,5 bis 40 mol bezogen auf 1 mol des Esters eingesetzt.

Die Hydrolyse wird im Allgemeinen in einem Temperaturbereich von 0°C bis +100°C durchgeführt.

Die Amidbildungen im Verfahrensschritt [A] (V) + (VI) → (I) wird bevorzugt in Dimethylformamid oder Dichlormethan als Lösemittel durchgeführt.

Als Hilfsstoffe für die Amidbildung werden bevorzugt übliche Kondensationsmittel eingesetzt, wie Carbodiimide z.B. N,N'-Diethyl-, N,N,'-Dipropyl-, N,N'-Diisopropyl-, N,N'-Dicyclohexylcarbodiimid, N-(3-Dimethylaminopropyl)-N'-ethylcarbodiimid-Hydrochlorid (EDC), oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder O-(Benzotriazol-1-yl)-N,N,N',N'-tetra-methyluronium-hexafluorophosphat (HBTU), 2-(2-Oxo-1-(2H)-pyridyl)-1,1,3,3-tetramethyluroniumtetrafluoroborat (TPTU) oder O-(7-Azabenzotriazol-1-yl)-N,N,N',N'-tetramethyl-uroniumhexafluorophosphat (HATU), gegebenenfalls in Kombination mit weiteren Hilfsstoffen wie 1-Hydroxybenztriazol oder N-Hydroxysuccinimid, sowie als Basen Alkalicarbonate z.B. Natrium- oder Kaliumcarbonat, oder -hydrogencarbonat, oder organische Basen wie Trialkylamine z.B. Triethylamin, N-Methylmorpholin, N-Methylpiperidin oder Diisopropylethylamin eingesetzt. Besonders bevorzugt ist die Kombination von EDC, N-Methylmorpholin und 1-Hydroxybenztriazol.

Die Amidbildung wird im Allgemeinen in einem Temperaturbereich von 0°C bis +100°C durchgeführt.

Als Lösemittel für die Acylierung im Verfahrensschritt [B] (VII) → (I) eignen sich die üblichen unter den Reaktionsbedingungen inerten Lösemittel, bevorzugt sind hierbei Dimethylformamid und Dichlormethan.

Als gegebenenfalls verwendete Base für die Acylierung eignen sich die üblichen anorganischen oder organischen Basen, bevorzugt ist Triethylamin.

Die Acylierung wird im Allgemeinen in einem Temperaturbereich von 0°C bis +100°C durchgeführt.

Die Verbindungen der Formeln (II), (III), (VI), (VIII), (IX) und (X) sind bekannt oder nach üblichen Methoden herstellbar (vgl. EP-A-0 725 061, EP-A-0 725 064, EP-A-0 581 003, EP-A-0 611 767, WO-A-00/73274).

Die Verbindungen der Formel (VII) können hergestellt werden, indem man Verbindungen der Formel (II) mit Verbindungen der Formel (XI)

Y-A-H (XI),

in welcher
- A: ein Sauerstoffatom oder eine Gruppe der Formel N-R⁵ bedeutet,
wobei R⁵ die oben angegebene Bedeutung hat,
und
- Y: für eine geeignete Amino- oder Hydroxyschutzgruppe steht,
gegebenenfalls in Anwesenheit einer Base, in Verbindungen der Formel (XII) 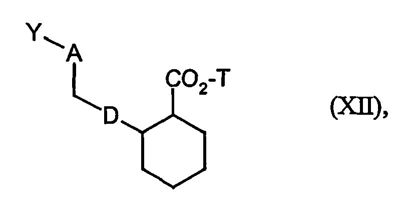 in welcher
A, D, T und Y die oben angegebenen Bedeutungen haben,
überführt,
in den nächsten Schritten, analog zu den unter [A] beschriebenen Reaktionsschritten, zunächst durch Hydrolyse zu Verbindungen der Formel (XIII), 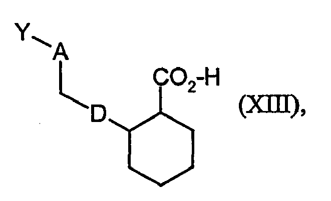 in welcher
A, D und Y die oben angegebenen Bedeutungen haben,
umsetzt,
dann mit Verbindungen der Formel (VI) zu Verbindungen der Formel (XIV) 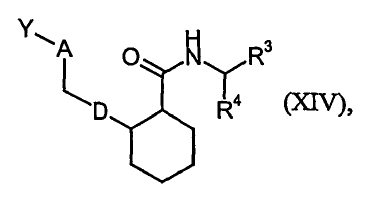 in welcher
A, D, Y, R³ und R⁴die oben angegebenen Bedeutungen haben,
umsetzt
und schließlich die Schutzgruppe Y nach üblichen Methoden abspaltet.

Die Herstellung von Verbindungen der Formel (VII) kann durch folgendes Formelschema beispielhaft erläutert werden:

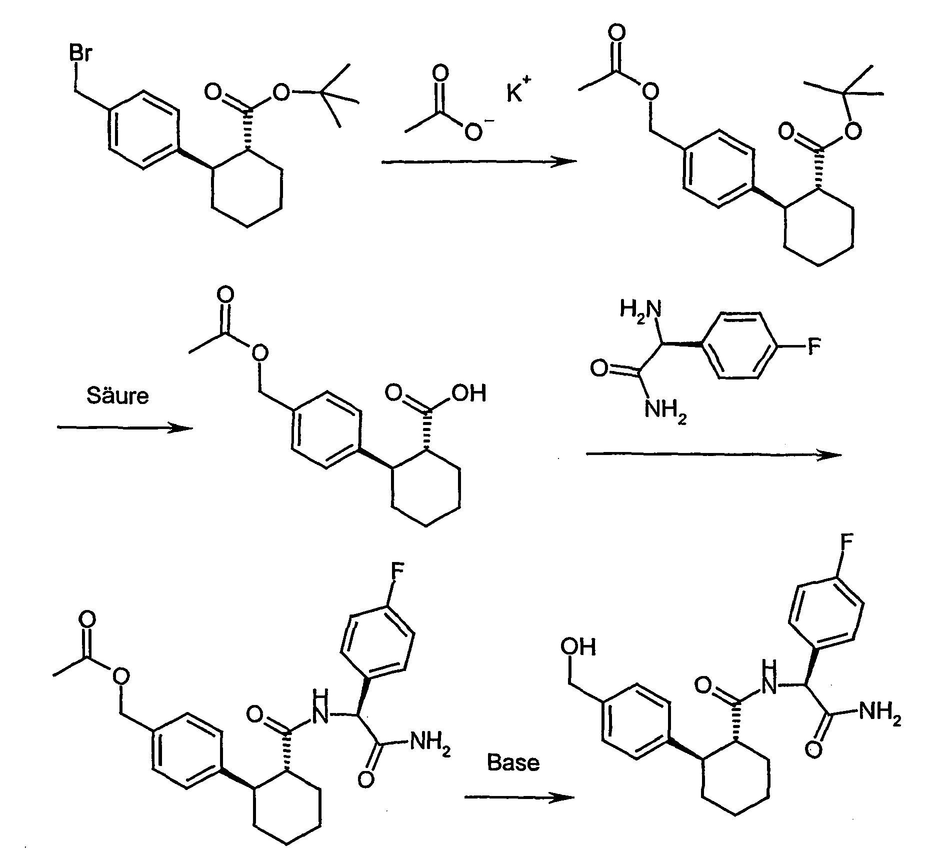

Die Umsetzung (II) + (XI) → (XII) erfolgt in den üblichen unter den Reaktionsbedingungen inerten Lösungsmitteln. Bevorzugt ist Acetonitril.

Als gegebenenfalls verwendete Base für diese Reaktion eignen sich die üblichen anorganischen oder organischen Basen.

Die Umsetzung wird im Allgemeinen in einem Temperaturbereich von 0°C bis +100°C durchgeführt.

Überraschenderweise zeigen die erfindungsgemäßen Verbindungen der Formel (I) ein nicht vorhersehbares, wertvolles pharmakologisches Wirkspektrum kombiniert mit verbesserten Applikationseigenschaften.

Die erfindungsgemäßen Verbindungen wirken als Adenosinaufnahme-Hemmer. Sie können zur Herstellung von Arzneimitteln zur Prävention und/oder Behandlung von ischämiebedingten peripheren und kardiovaskulären Erkrankungen, insbesondere zur akuten und chronischen Behandlung von ischämischen Erkrankungen des Herz-Kreislauf-Systems, wie z.B. der koronaren Herzkrankheit, der stabilen und instabilen Angina pectoris, von peripheren und arteriellen Verschlusskrankheiten, von thrombotischen Gefäßverschlüssen, des Myocardinfarkts und von Reperfusionsschäden, verwendet werden.

Außerdem sind sie durch ihr Potential, die Angiogenese zu verstärken, besonders für eine dauerhafte Therapie aller Verschlusskrankheiten geeignet.

Darüber hinaus können die erfindungsgemäßen Verbindungen allein oder in Kombination mit anderen Arzneimitteln in oraler oder intravenöser Applikation zur Prävention und/oder Behandlung von cerebraler Ischämie, Hirnschlag, Reperfusionsschäden, Hirntrauma, Ödemen, Krämpfen, Epilepsie, Atemstillstand, Herzstillstand, Reye-Syndrom, zerebraler Thrombose, Embolie, Tumoren, Blutungen, Enzephalomyelitis, Hydroenzephalitis, Rückenmarksverletzungen, post-operative Hirnschäden, Verletzungen der Retina oder des optischen Nervs nach Glaukom, Ischämie, Hypoxie, Ödem oder Trauma sowie in der Behandlung von Schizophrenie, Schlafstörungen und akuten und/oder chronischen Schmerzen sowie neurodegenerativen Erkrankungen, insbesondere zur Behandlung von Krebs-induzierten Schmerzen und chronischen neuropathischen Schmerzen, wie zum Beispiel bei diabetischer Neuropathie, posttherpeutischer Neuralgie, peripheren Nervenbeschädigungen, zentralem Schmerz (beispielsweise als Folge von cerebraler Ischämie) und trigeminaler Neuralgie und anderen chronischen Schmerzen, wie zum Beispiel Lumbago, Rückenschmerz (lower back pain) oder rheumatischen Schmerzen, eingesetzt werden.

Adenosinaufnahme-Hemmer wie die erfindungsgemäßen Verbindungen können ausserdem auch bei der Behandlung von Bluthochdruck und Herzinsuffizienz, Myocarditis, Nephritis, Pancreatitis, diabetischer Nephropathie, Ödemen und zur Potenzierung der Wirkung von Nukleobase-, Nukleosid- oder Nukleotid-Antimetaboliten in der chemotherapeutischen Behandlung von Krebs und in der antiviralen (z.B. HIV) Chemotherapie Verwendung finden.

Die erfindungsgemäßen Verbindungen zeichnen sich durch erhöhte Wasserlöslichkeit und verbesserte Bioverfügbarkeit, insbesondere bei oraler Applikation, aus. Diese vorteilhaften Eigenschaften lassen sich gegebenenfalls unter Zuhilfenahme von Formulierungshilfsmitteln und/oder durch Einstellung eines geeigneten pH-Wertes noch verbessern. Gute Wasserlöslichkeit und hohe Bioverfügbarkeit sind bei Arzneimittelwirkstoffen und -formulierungen bekanntermaßen vorteilhafte Eigenschaften; so eignen sich die erfindungsgemäßen Verbindungen beispielsweise besonders gut zur oralen und intravenösen Applikation.

### A Bewertung der physiologischen Wirksamkeit

### 1. Bestimmung der Löslichkeit

Zur Löslichkeitsbestimmung wurde eine Fällungsmethode herangezogen:

10 mg der Testsubstanz werden in 50µl DMSO vollständig gelöst (Stammlösung). Von dieser Lösung gibt man 20µl in 2000µl physiologische Kochsalzlösung. Diese Lösung wiederum wird zur Equilibrierung bei 25°C im Thermomixer Comfort (Fa. Eppendorf) bei 1400 upm 24 Stunden geschüttelt.

Die ausgefallenen Teile der Testsubstanz werden mit der Biofuge 15 Fa. Heraeus 5 min bei 14000 upm abzentrifugiert. 1300 µl des Überstandes werden erneut mit der Microfuge Fa. Beckmann bei 45000 upm =125000g zentrifugiert.

10 µl dieses Zentrifugationsüberstandes werden nun mit 1000 µl DMSO verdünnt und diese Lösung an der HPLC gemessen. (Fa. Hewlett Packard 1090, Methode: Gradient von 100 % PBS-Puffer pH=4 innerhalb von 15 min auf 10 % Puffer/90 % Acetonitril, Säule: RP18)

Die gemessene Peakfläche der HPLC-Messung wird mit einer Eichgerade auf die Substanzkonzentration umgerechnet. Für die Eichgerade werden 20 µl der Stammlösung sukzessiv mit DMSO so verdünnt, dass 5 Konzentrationen von 2,5 mg/l bis 2000 mg/l entstehen. Diese Lösungen werden ebenfalls an der HPLC gemessen (Methode s. o.) und die Peakflächen gegen die Konzentrationen aufgetragen.

### 2. Hemmung der Adenosinaufnahme in Kaninchenerythrozyten durch die erfindungsgemäßen Verbindungen.

Die Fähigkeit von Substanzen das Adenosinaufnahme-System zu beeinflussen wird durch die Bestimmung der hemmenden Wirkung der Substanzen auf die funktionelle Adenosinaufnahme untersucht.

Für den funktionellen Adenosinaufnahme-Test wird eine Erythrozyten Präparation aus Kaninchenblut verwendet. Das Blut wird intravenös entnommen, als Anticoagulans wird Citrat (3ml Monovette 9NC Firma Sarstedt) verwendet. Das Blut wird 5 min bei 3000 g zentrifugiert und die Erythrozyten in 10 mM 3-(N-Morpholino)-propansulfonsäure-Puffer (MOPS) / 0,9 %ige wässrige Natriumchloridlösung pH 7,4 suspendiert. Die Suspension wird auf das Einhundertfache des ursprünglichen Blutvolumens verdünnt. Je 990 µl der Suspension werden mit 10 µl einer geeigneten Konzentration der zu untersuchenden Substanz vesetzt und 5 min bei 30°C inkubiert. Danach werden 5 µl einer 4 mM Adenosinlösung zugegeben und weitere 15 min bei 30°C inkubiert. Danach werden die Proben 5 min bei 3000 g zentrifugiert und 700 µl der Überstände mit 28 µl 70 %iger HClO₄ versetzt 30 min im Eisbad stehen gelassen, 3 min bei 16000 g zentrifugiert und 350 µl der Probe mit 30 µl 5N NaOH neutralisiert. 50 µl der Probe werden auf eine Säule (Waters Symmetry C18 5µm 3,9x150mm) aufgetragen. Als Vorsäule wird eine Spherisorb ODS II 5µm 4,6x10mm verwendet. Als Fließmittel wird ein Gradient aus 50 mM KH₂PO₄/5 mM Tributylamin pH 7 (Fließmittel A) und einer Mischung Fließmittel A/Methanol 1/1 (Fließmittel B) verwendet. Der Gradient wird von 10 bis 40 % B bei einer Fließrate von 0,5 ml/min gefahren. Das vorhandene Adenosin wird durch seine Absorbtion bei 260 nm quantifiziert, ebenso das entstandene Inosin und Hypoxanthin. Der IC₅₀ bezeichnet die Konzentration des Wirkstoffs bei der 15 min nach Adenosinzugabe noch 50 % der ursprünglich eingesetzten Adenosinkonzentration vorhanden ist.

Nach diesem Test wurde für Beispiel 1-1 ein IC₅₀-Wert von 30 nM, für Beispiel 1-3 ein IC₅₀-Wert von 20 nM, für Beispiel 1-14 ein IC₅₀-Wert von 30 nM, für Beispiel 1-33 ein IC₅₀-Wert von 40 nM, für Beispiel 2-1 ein IC₅₀-Wert von 20 nM und für Beispiel 2-18 ein IC₅₀- Wert von 20 nM bestimmt.

### 3. In vivo Testmodell zur Prüfung von Adenosinaufnahme-Hemmern

Erwachsene FBI(Foxhound-Beagle-Irish-Setter)-Hunde (20-30 kg) werden initial mit einer Kombination von Trapanal 500mg und Alloferin 55 mg narkotisiert. Die Narkose wird durch Infusion eines Gemisches von Fentanyl 0,072 mg/kg, Alloferin 0,02 mg/kg und Dihydrobenzpyridyl 0,25 mg/kg x min erhalten. Die Tiere werden intubiert und mit einem Gemisch aus O₂/N₂O 1/5 mit einer Engström Atempumpe mit 16 Atemzügen pro min und einem Volumen von 18-24 ml/kg beatmet. Die Körpertemperatur wird bei 38°C ± 0,1°C gehalten. Der arterielle Blutdruck wird über einen Katheder in der Femoralarterie gemessen. Es wird eine Thorakotomie auf der linken Seite am fünften Intercostalraum durchgeführt. Die Lunge wird zurückgelegt, fixiert und das Pericard eingeschnitten. Ein proximaler Abschnitt der LAD distal zur ersten diagonalen Verzweigung wird freipräpariert und ein kalibrierter elektromagnetischer Flussmesskopf (Gould Statham, model SP7515) um das Gefäß gelegt und mit einem Flussmessgerät (Statham, model SP-2202) verbunden. Ein mechanischer Okkluder wird distal zum Flussmesskopf so angebracht, dass keine Verzweigungen zwischen Flussmesskopf und Okkluder liegen.

Blutentnahmen und Substanzgaben werden durch einen Katheder in der Femoralvene durchgeführt. Ein peripheres EKG wird mit subcutan verankerten Nadeln abgeleitet. Ein MikrotipDruckmanometer (Millarmodel PC-350) wird durch den linken Vorhof geschoben um den linksventrikulären Druck zu messen. Die Messung der Herzfrequenz wird über die R-Zacke des EKGs getriggert. Die hämodynamischen Parameter und der Koronarfluss werden während des gesamten Versuchs über einen Vielfachschreiber aufgezeichnet.

Eine Okklusion von vier Minuten verursacht eine reaktive Hyperämie. Man misst die Differenz zwischen dem Koronarfluss unter Kontrollbedingungen und dem Maximalfluss während der reaktiven Hyperämie. Die Zeit, die benötigt wird, um die Hälfte dieses Maximalflusses im Abfall zu erreichen, ist ein geeigneter Parameter, um die reaktive Hyperämie zu beurteilen.

Nach einer Stabilisierungszeit von einer Stunde wird das Experiment mit einer vierminütigen Okklusion begonnen. Dreißig Minuten später wird die Substanz gegeben (i.v.) und zwei Minuten später erneut occludiert. Die reaktive Hyperämie nach Verum und Placebo wird verglichen.

### 4. Messung der Plasmakonzentration von Adenosinaufnahme-Hemmern nach oraler Gabe bei Mäusen.

Testprinzip: Nach oraler Gabe wird den Mäusen Blut entnommen und die Konzentration des Wirkstoffs im Blut durch die funktionelle Hemmung der Adenosinaufnahme an Kaninchenerythrozyten gemessen.

Die Substanzen wurden in einer Dosierung von 10 mg/kg und einem Applikationsvolumen von 10 ml/kg mit der Schlundsonde appliziert. Als Lösungsmittel wurde Polyethylenglykol 400/Ethanol 9:1 verwendet. Nach einer Stunde wurden die Tiere narkotisiert und durch Herzpunktion ca. 0,5 bis 0,7 ml Blut entnommen. Das Blut wurde im 5-fachem Volumen Acetonitril gefällt, 30 Minuten im Eisbad gehalten und anschließend 5 Minuten bei 16.000 g in einer Eppendorf- Zentrifuge zentrifugiert. Der Überstand wurde bei Raumtemperatur in einer Speedvac zur Trockene eingedampft. Die getrockneten Proben wurden zuerst mit 20 µl DMSO benetzt und danach mit 1 ml 10mM 3-(N-Morpholino)propansulfonsäure-Puffer (MOPS) / 0,9 %ige wässrige Natriumchloridlösung pH 7,4 versetzt und 15 Minuten im Ultraschallbad gehalten. Danach wurde 5 Minuten bei 16.000 g zentrifugiert.

Es wurde jeweils 500 µl Extrakt; 200 µl Extrakt und 300 µl des o.g. Puffers; 100 µl Extrakt und 400 µl Puffer; 50 µl Extrakt und 450 µl Puffer mit einer Suspension von jeweils 500 µl Kaninchenerythrozyten versetzt. [Die Erythrozten wurden, wie unter Versuch 2 ("Hemmung der Adenosinaufnahme in Kaninchenerythrozyten") beschrieben, isoliert und auf das fünfzigfache des ursprünglichen Blutvolumens verdünnt]. Nach fünf Minuten wurde, wie unter Versuch 2 beschrieben, Adenosin zugesetzt und die Adenosinaufnahme gemessen. Aus der Inhibition der Adenosinaufnahme lässt sich die Konzentration des Hemmers in der Probe berechnen, da die Hemmwirkung des Adenosinaufnahme-Hemmers vorher durch eine Konzentrationsirkungskurve mit der bei Versuch 2 beschriebenen Methode ermittelt worden war.

### 5. Maus-Angiogenese-Modell

Um die Wirkung von Adenosinaufnahme-Hemmern auf die Collateralisierung und die Neovaskularisation zu prüfen, wurde ein Maus Modell zur Angiogenese entwickelt. Dabei wird der Maus eine Femoralarterie am oberen Ende des Oberschenkels ligiert. Dadurch wird eine chronische Ischämie der jeweiligen Hinterlaufs induziert. Der andere Hinterlauf dient als individuelle Kontrolle. Um einen Restfluss durch das ligierte Gefäß ausschließen zu können, werden zwei Ligaturen gelegt und das Gefäß zwischen diesen beiden durchtrennt. Wenige Tage nach dieser Operation wird die Behandlung gestartet.

Als Messparameter während des laufenden Versuches wird die Pfötchentemperatur beider Hinterläufe gemessen. Dabei weist der ischämische Hinterlauf aufgrund seiner schlechteren Durchblutung die niedrigere absolute Temperatur auf. Errechnet wird jeweils die Temperaturdifferenz zwischen den Pfoten der Hinterläufe. Diese individuelle Temperaturdifferenz wird in verschiedenen Behandlungsgruppen Dosisabhängig und gegenüber einer unbehandelten Kontrolle bestimmt. Adenosinaufnahme-Hemmer zeigen in diesem Modell eine signifikante Verbesserung der Durchblutung des ischämischen Hinterlaufs im Vergleich zu entsprechenden Kontrollen.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen. Neben den Wirkstoffen der Formel (I) können die Formulierungen auch andere pharmazeutische Wirkstoffe enthalten.

Die Formulierungen werden beispielsweise hergestellt durch Verstrecken der Wirkstoffe mit inerten, nichttoxischen, pharmazeutisch geeigneten Hilfsstoffen. Als Hilfsstoffe seien beispielsweise aufgeführt: Wasser, nichttoxische organische Lösungsmittel wie z.B. Paraffine, pflanzliche Öle (z.B. Sesamöl), Alkohole (z.B. Ethanol, Glycerin), Glykole (z.B. Polyethylenglykol), feste Trägerstoffe wie natürliche oder synthetische Gesteinsmehle (z.B. Talkum oder Silicate), Zucker (z.B. Milchzucker), Emulgiermittel, Dispergiermittel (z.B. Polyvinylpyrrolidon) und Gleitmittel (z.B. Magnesiumsulfat).

Die Applikation erfolgt in üblicher Weise, vorzugsweise oral, transdermal, parenteral, perlingual, intravenös; besonders bevorzugt oral oder intravenös.

Im Allgemeinen hat es sich als vorteilhaft erwiesen, bei intravenöser Applikation Mengen von etwa 0,0001 bis 10 mg/kg, vorzugsweise etwa 0,003 bis 1 mg/kg Körpergewicht zur Erzielung wirksamer Ergebnisse zu verabreichen. Bei oraler Applikation werden 0,1 bis 20 mg/kg, vorzugsweise 0,3 bis 10 mg/kg Körpergewicht verwendet.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muss. Es kann empfehlenswert sein, diese Menge in mehreren Einzelgaben über den Tag zu verteilen oder den Wirkstoff verzögert aus der Formulierung über einen längeren Zeitraum freizusetzen.

Die vorliegende Erfindung wird nachstehend anhand der folgenden bevorzugten Beispiele veranschaulicht, die die Erfindung jedoch keinesfalls beschränken.

Soweit nicht anders angegeben, beziehen sich alle Mengenangaben auf Gewichtsprozente; bei Lösungsmittelgemischen sind Volumenverhältnisse angeführt.

### B Herstellungsbeispiele

In den Beispielen werden die folgenden Abkürzungen verwendet:
DMF = N,N-Dimethylformamid
DMSO = Dimethylsulfoxid
TFA = Trifluoressigsäure
THF = Tetrahydrofuran
EDC = N-(3-Dimethylaminopropyl)-N'-cthylcarbodiimid-Hydrochlorid
HOBT =1-Hydroxybenzotriazol
DMAP = 4-Dimethylaminopyridin
TBDMS= tert.-Butyl-dimethylsilyl
BOC = tert.-Butyloxycarbonyl

### Ausgangsverbindungen

### Beispiel I

### (1R,2R)-2-(4-Brommethyl-phenyl)-cyclohezan-1-carbonsäure-tert.-butylester:

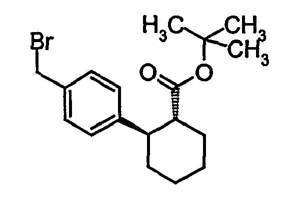

Das Zwischenprodukt wird in Analogie zur Vorschrift für das Racemat (US-A-5 395 840, Spalte 17) hergestellt. Zur Aufreinigung wird das erhaltene Gemisch mit Diethylether oder Diisopropylether verrührt.

### Beispiel II

### (2S)-2-Amino-4-(methylsulfonyl)butanamid Hydrochlorid

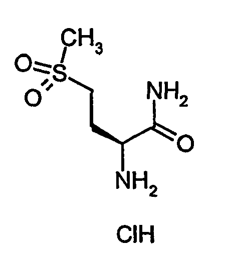

### a) N²-(tert.-Butoxycarbonyl)-L-methioninamid

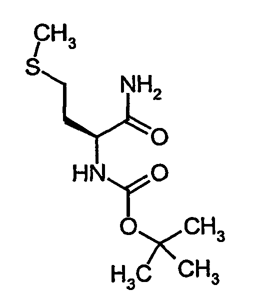

Zu 20 g (80,2 mmol) *N*-Boc-L-methionin in 200 ml THF werden unter Argon bei -15°C 8,12 g (80.2 mmol) Triethylamin hinzugegeben. Binnen 5 min werden 10,4 ml (80,2 mmol) Chlorameisensäureisobutylester zugetropft und die Reaktionsmischung wird 30 min bei -15°C gerührt. Anschließend werden 40 ml 2N Ammoniak-Lösung in Methanol hinzugegeben und 30 min in der Kälte gerührt. Das Reaktionsgemisch wird filtriert, das Filtrat im Vakuum eingeengt und der Rückstand mit 200 ml Wasser 1 Stunde ausgerührt. Der Feststoff wird abgesaugt, in 250 ml Dichlormethan gelöst, zweimal mit ges. Natriumhydrogencarbonat-Lösung gewaschen und das Solvens erneut im Vakuum entfernt. Der Rückstand wird mit 0,7 1 Petrolether ausgerührt, abgesaugt und im Hochvakuum getrocknet. Man erhält 12,86 g (64,6 % der Theorie) Produkt als kristalline Substanz.
R_{*f*}(Dichlormethan/Methanol 10:1) = 0,52.
MS (DCI, NH₃) = 249 [(M+H)⁺; 68 %]; 266 [(M+NH₄)⁺; 100 %].
¹H-NMR (200 MHz, DMSO-d6) δ [ppm]= 1,38 (s, 9H); 1,65-1,95 (m, 2H); 2,04 (s, 3H); 2,44 (br. t, 2H); 3,93 (dt, 1H); 6,88 (d, 1H); 6,99 (br. s, 1H); 7,25 (br. s, 1H).

### b) tert.-Butyl (1S)-1-(aminocarbonyl)-3-(methylsulfonyl)propylcarbamat

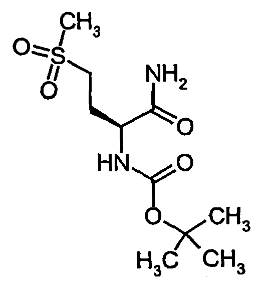

Zu 12,25 g (49,3 mmol) der Verbindung aus Beispiel II-a in 50 ml Dichlormethan und 15 ml Methanol werden bei 0°C portionsweise 29,9 g (98,7 mmol) 3-Chlorperbenzoesäure hinzugegeben. Nach 2 Stunden wird gesättigte Natriumhydrogensulfit-Lösung hinzugefügt und eine Stunde bei Raumtemperatur gerührt. Die Phasen werden getrennt, die wässrige Phase zweimal mit Dichlormethan extrahiert und die vereinten organischen Phasen mit gesättigter Natriumhydrogencarbonat-Lösung gewaschen. Nach dem Trocknen über Natriumsulfat und dem Entfernen des Solvens im Vakuum werden 2,36 g (9,4 %) Produkt isoliert. Daraufhin wird die Natriumhydrogencarbonat-Lösung zweimal mit Essigester extrahiert, die vereinten organischen. Phasen werden über Natriumsulfat getrocknet, das Solvens im Vakuum entfernt und dabei 7,38 g (46,6 %) Produkt erhalten, welches noch geringe Mengen Benzoesäure enthält. Das Einengen der Natriumhydrogencarbonat-Lösung und Ausschütteln des Rückstandes mit Wasser und Essigester, zweimalige Extraktion der wässrigen. Phase mit Essigester und Trocknung über Natriumsulfat ergibt weitere 0,65 g (4,4 %) Produkt. Die vereinten Produktfraktionen werden ohne weitere Aufreinigung weiter umgesetzt.
R_{*f*}(Dichlormethan/Methanol 10:1) = 0,49.
MS (ESI-pos.) = 281 [(M+H)⁺; 18 %]; 303 [(M+Na)⁺; 100 %]; 583 [(2M+Na)⁺, 50%].
¹H-NMR (200 MHz, DMSO-d6) δ [ppm]= 1,38 (s, 9H); 1,72-2,14 (m, 2H); 2,97 (s, 3H); 3,08 (m, 2H); 3,97 (m, 1H); 6,96 (d, 1H); 7,17 (br. s, 1H); 7,34 (br. s, 1H).

### c) (2S)-2-Amino-4-(methylsulfonyl)butanamid Hydrochlorid

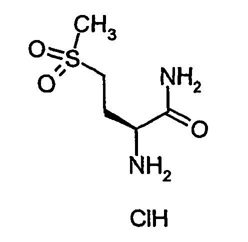

10,25 g (36,56 mmol) der Verbindung aus Beispiel II-b werden in 20 ml Dioxan gelöst und mit 50 ml 4N HCl in Dioxan 1 Stunde bei Raumtemperatur gerührt. Nach der Zugabe von weiteren 50 ml 4 N HCl in Dioxan wird bei Raumtemperatur über Nacht bis zur vollständigen Umsetzung gerührt. Der ausgefallene Feststoff wird abgesaugt und mit Petrolether gewaschen. Es werden 6,96 g (62,5 % der Theorie) Produkt als farbloser Feststoff isoliert.
MS (ESI-pos.) = 181 [(M+H)⁺; 100 %]; 203 [(M+Na)⁺; 18 %].
¹H-NMR (200 MHz, DMSO-d6) δ [ppm]= 2,19 (m, 2H); 3,04 (s, 3H); 3,22 (m, 2H); 3,89 (t, 1H); 7,67 (br.s, 1H); 8,08 (br. s, 1H); 8,38 (br. s, 3H).

### Allgemeine Vorschrift zur Alkylierung [A]:

In einem typischen Ansatz wird zu einer Suspension von Natriumhydrid (4,33 mmol) in trockenem DMF (6 ml) eine Lösung der Verbindung der Formel (III) (4,12 mmol) in trockenem DMF (6 mL) gegeben. Nach 30 min Rühren bei Raumtemperatur und 30 min bei 40°C wird eine Suspension der Verbindung der Formel (II) (4,12 mmol) in trockenem DMF (15 ml) zugegeben. Nach 24 Stunden Rühren bei Raumtemperatur wird das Rohgemisch in destilliertes Wasser gegeben (200 ml). Die milchige Emulsion wird mit 2 g Natriumchlorid versetzt und viermal mit je 40 ml Diethylether extrahiert. Die vereinigten organischen Phasen werden dreimal mit je 30 ml gesättigter Kochsalzlösung gewaschen und über Natriumsulfat getrocknet. Nach Chromatographie (Kieselgel, Cyclohexan:Essigester) wird das Produkt in 60 bis 96 % Ausbeute erhalten.

### Allgemeine Vorschrift zur Esterspaltung [B]:

In einem typischen Ansatz wird bei Raumtemperatur eine Lösung aus Ester der Formel (IV) (T= tert.-Bu; 24,5 mmol) in 46 ml Dichlormethan mit 23 ml Trifluoressigsäure versetzt und 16 Stunden bei Raumtemperatur gerührt. Das Lösungsmittel wird im Vakuum abgezogen und der Rückstand in 150 ml Diethylether aufgenommen, mit 200 ml Waser versetzt und mit 1 N Natronlauge (ca. 70 ml) auf pH 12 eingestellt. Die Phasen werden getrennt und die wässrige Phase wird zweimal mit je 100 ml Diethylether gewaschen. Es wird mit 5 N Salzsäure auf PH 3 angesäuert und dreimal mit je 150 ml Dichlormethan extrahiert. Nach Trocknen der vereinigten organischen Phasen über Natriumsulfat und Einengen im Vakuum wird das Produkt in 90 bis 98 % Ausbeute erhalten.

### Allgemeine Vorschrift zur Amidbildung [C]:

In einem typischen Ansatz wird ein Gemisch aus der Säure der Formel (V) (3,96 mmol), 1-Hydroxybenzotriazol (3,96 mmol), EDC-Hydrochlorid (4,75 mmol) und 4-Dimethylaminopyridin (0,33 mmol) mit trockenem DMF (10 ml) versetzt. Es wird 5 Minuten bei Raumtemperatur gerührt und dann werden N-Methylmorpholin (11,9 mmol) und (*S*)-Phenylglycinamid-Hydrochlorid (4,75 mmol) zugegeben. Nach drei Tagen Rühren bei Raumtemperatur wird mit Reversed-Phase-HPLC chromatographiert und die erhaltene Produktfraktion anschließend lyophilisiert. Das gewünschte Produkt wird in 60 bis 90 % Ausbeute erhalten.

### Synthesebeispiele

### Beispiel 1-1

### N-{4-[(1R,2R)-2-({[(1S)-2-Amino-2-oxo-1-phenylethyl]amino}carbonyl)cyclohexyl]benzyl}-N-phenyl-4-morpholincarbonsäureamid:

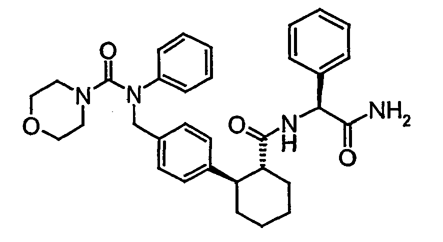

### a) N-Phenyl-4-morpholincarbonsäureamid:

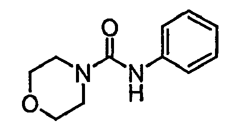

Eine Lösung von 7,44 g (85,4 mmol) Morpholin in 35 ml abs. Dichlormethan wird auf 0°C gekühlt. Es wird tropfenweise (innerhalb von 5 min) mit einer Lösung von 9,25 g (77,7 mmol) Phenylisocyanat in 15 ml abs. Dichlormethan versetzt. Man rührt 30 min bei 0°C und über Nacht bei Raumtemperatur. Dabei fällt das Produkt in Form weißer Kristalle aus. Die Kristalle werden abfiltriert, zweimal mit je 20 ml Diethylether gewaschen und im Hochvakuum getrocknet: 11,1 g weiße Kristalle (69 % der Theorie).
R_{*f*}(Dichlormethan/Methanol 20:1) = 0,38.
MS (DCI, NH₃) = 224 (M+NH4)⁺.
¹H-NMR (300 MHz, CDCl₃) δ[ppm]: 3,46 (4H, t), 3,72 (4H, t), 6,39 (1H, br. s), 7,05 (1H, tt), 7,23-7,38 (4H, m).

### b) (1R,2R)-2-(4-{[(4-Morpholinylcarbonyl)(phenyl)amino]methyl}phenyl)-cyclohexancarbonsäure-tert. -butylester:

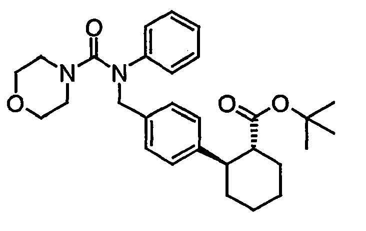

Zu einer Suspension von 173 mg (60 % in Mineralöl, 4,33 mmol) Natriumhydrid in 4 ml DMF wird eine Lösung von 850 mg (4,12 mmol) der Verbindung aus Beispiel 1-1a in 6 ml DMF getropft. Nach 30 min Rühren bei Raumtemperatur wird eine Suspension von 1,62 g (90 %ig, 4,12 mmol) (1*R*,2*R*)-2-(4-Brommethyl-phenyl)-cyclohexan-1-carbonsäure-tert.-butylester aus Beispiel I in 15 ml DMF zugetropft. Nach 24 Stunden Rühren bei Raumtemperatur wird das Rohgemisch in 200 ml dest. Wasser gegeben. Die milchige Emulsion wird mit 2 g Natriumchlorid versetzt und viermal mit je 40 ml Diethylether extrahiert. Die vereinigten organische Phasen werden dreimal mit je 30 ml gesättigter Kochsalzlösung gewaschen und über Natriumsulfat getrocknet. Das gelbe, ölige Rohprodukt wird durch Säulenchromatographie (Kieselgel (70-230 mesh), Gradient von Cyclohexan zu Cyclohexan/Essigester = 2:1) gereinigt. Man erhält 1,89 g (96 % der Theorie) eines farblosen Öls, das im Hochvakuum erstarrt.
R_{*f*}(Cyclohexan/Essigester 2:1) = 0,17.
MS (ESI) = 479 (M+H)⁺.
¹H-NMR (200 MHz, DMSO- d₆) δ[ppm]: 1,00 (9H, s), 1,20-1,53 (4H, m), 1,58-1,92 (4H, m), 2,30-2,60 (2H, m), 3,04-3,16 (4H, m), 3,33-3,43 (4H, m), 4,78 (2H, s), 6,98-7,32 (9H, m).

### c) (1R,2R)-2-(4-{[(4-Morpholinylcarbonyl)(phenyl)amino]methyl}phenyl)-cyclohexancarbonsäure:

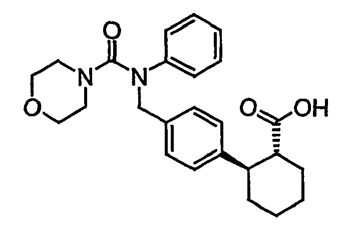

Eine Lösung von 11,75 g (24,5 mmol) der Verbindung aus Beispiel 1-1b in 46 ml Dichlormethan wird mit 23 ml Trifluoressigsäure versetzt und 16 Stunden bei Raumtemperatur gerührt. Das Lösungsmittel wird im Vakuum abdestilliert und der Rückstand jeweils zweimal in 10 ml Dichlormethan gelöst, mit 30 ml Cyclohexan versetzt und im Vakuum eingedampft.

Der Rückstand wird in 150 ml Diethylether gelöst, mit 200 ml Wasser versetzt und mit 70 ml 1N NaOH auf pH 12 eingestellt. Die Phasen werden getrennt und die wäßrige Phase zweimal mit je 100 ml Diethylether gewaschen. Es wird mit 5N Salzsäure auf pH = 3 angesäuert und dreimal mit je 150 ml Dichlormethan extrahiert. Es wird mit Natriumsulfat getrocknet und das Lösungsmittel im Vakuum abgezogen. Der Rückstand wird in 20 ml Diethylether gelöst und wieder eingedampft. Dabei erhält man 10,1 g (95 % der Theorie) des Produkts in Form eines weißen, festen Schaums.
R_{*f*}(Dichlormethan/Methanol 10:1) = 0,39.
MS (ESI) = 423 (M+H)⁺.
¹H-NMR (200 MHz, DMSO- d₆) δ[ppm]: 1,20-1,54 (4H, m), 1,58-1,83 (3H, m), 1,85-2,03 (1H, m), 2,40-2,50 (2H, m), 3,05-3,18 (4H, m), 3,30-3,44 (4H, m), 4,77 (2H, s), 7,00-7,34 (9H, m), 11,74 (1H, br. s).

### d) N-{4-[(1R,2R)-2-({[(1S)-2-Amino-2-oxo-1-phenylethyl]amino}carbonyl)-cyclohexyl]benzyl}-N-phenyl-4-morpholincarbonsäureamid:

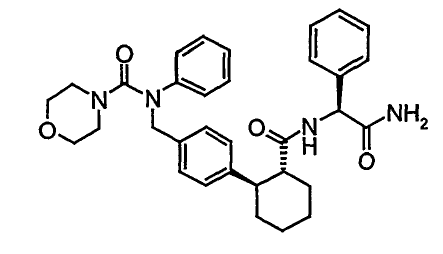

Ein Gemisch aus 1,67 g (3,96 mmol) der Verbindung aus Beispiel 1-1c, 535 mg (3,96 mmol) HOBT, 910 mg (4,75 mmol) EDC und 40 mg DMAP werden mit 10 ml abs. DMF versetzt. Man rührt 5 min bei Raumtemperatur bis sich eine klare Lösung gebildet hat. Dann werden 1,31 ml (1,20 g, 11,88 mmol) *N*-Methylmorpholin und 0,886 g (4,75 mmol) L-Phenylglycinamid-hydrochlorid zugegeben. Man läßt 3 Tage bei Raumtemperatur rühren und trennt dann direkt über RP-HPLC (C18 Gromsil, 250mm x 30 mm, 50 ml/min, Gradient Wasser/Acetonitril 90:10 → Wasser/Acetonitril 10:90 in 30 min, 4,5 ml Lösung Rohgemisch pro Trennung). Das Acetonitril wird im Vakuum entfernt wobei sich das Produkt als schwach rosafarbener, klebriger Feststoff abscheidet. Es wird eingefroren und über Nacht lyophilisiert. Man erhält 1,80 g (82 % der Theorie) des Produktes als weißen Feststoff.
R_{*f*}(Dichlormethan/Methanol 20:1) = 0,20.
MS (ESI) = 555 (M+H)⁺.
¹H-NMR (300 MHz, DMSO- d₆) δ[ppm]: 1,20-1,54 (4H, m), 1,61-1,87 (4H, m), 2,59-2,70 (1H, m), 2,75-2,86 (1H, m), 3,12 (4H, t), 3,38 (4H, t), 4,79 (2H, s), 5,17 (1H, d), 6,80-6,89 (2H, m), 7,02-7,15 (11H, m), 7,28 (2H, t), 7,58 (1H, br. s), 7,96 (1H, d).

### Beispiel 1-34

### (1R,2R)-2-(4-{[Acetyl(2-pyridinyl)amino]methyl}phenyl)-N-[(1S)-2-amino-2-oxo-1-phenylethyl]cyclohexancarboxamid:

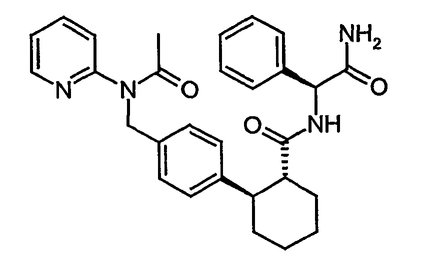

### a) (1R,2R)-2-(4-{[Acetyl(2-pyridinyl)amino]methyl}phenyl)cyclohexancarbonsäure-tert.-butylester:

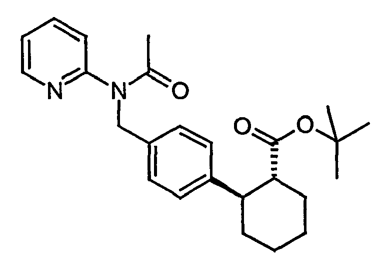

N-(2-Pyridinyl)acetamid (500mg) wird in trockenem DMF (25 mL) vorgelegt, bei 0°C mit Natriumhydrid (116 mg, 80 % in Öl) versetzt, 30 min bei Raumtemperatur und 30 min bei 40°C gerührt, auf 0°C abgekühlt und portionsweise mit der Verbindung aus Beispiel I (1,36 g) versetzt. Es wird über Nacht bei Raumtemperatur gerührt, anschließend bei 0°C mit Wasser hydrolysiert und mit Diethylether extrahiert. Die organischen Phasen werden mit Magnesiumsulfat getrocknet und dann eingeengt. Anschliessende Chromatographie (Kieselgel, Cyclohexan:Essigester 1:1 bis 0:1) liefert (1R,2R)-2-(4-{[Acetyl(2-pyridinyl)amino]methyl}phenyl)cyclohexancarbonsäure-tert.-butylester als farblosen Feststoff.
MS (ESI) = 409 (M+H)⁺.
¹H-NMR (300 MHz, DMSO-d₆): δ[ppm]: 1.0 (s, 9H), 1.2-1.49 (m, 5H), 1.62-1.77 (m, 3H), 1.81-1.9 (m, 1H), 2.02 (s, 3H), 2.35-2.45 (m, 1H), 5.02 (s, 2H), 7.05-7.13 (m, 4H), 7.2-7.27 (m, 1H), 7.37 (d, 1H), 7.48 (td, 1H), 8.43 (dd, 1H).

### b) (1R,2R)-2-(4-{[Acetyl(2-pyridinyl)amino]methyl}phenyl)cyclohezancarbonsäure

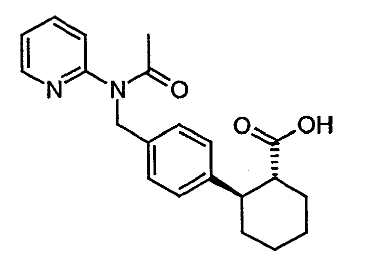

(1*R*,2*R*)-2-(4- {[Acetyl(2-pyridinyl)amino]methyl}phenyl)cyclohexancarbonsäure-tert.-butylester (0,44 g) wird in Dichlormethan (3,4 mL) und Trifluoressigsäure (3,4 mL) gelöst, 2 Stunden bei Raumtemperatur gerührt und anschliessend bei 0°C mit Natronlauge basisch gestellt. Die wässrige Phase wird mit Dichlormethan gewaschen, mit Salzsäure angesäuert und mit Dichlormethan extrahiert. Die organischen Extrakte werden über Natriumsulfat getrocknet und eingeengt. Es wird (1*R*,2*R*)-2-(4-{[Acetyl(2-pyridinyl)amino]methyl}phenyl)cyclohexancarbonsäure (445 mg) als gelbes zähflüssiges Öl erhalten.
MS (ESI) = 353 (M+H)⁺; 375 (M+Na)⁺.
¹H-NMR (300 MHz, DMSO-d₆): δ[ppm]: 1.21-1.5 (m, 4H), 1.62-1.8 (m, 3H), 1.89-1.98 (m, 1H), 2.0 (s, 3H), 2.4-2.5 (m, 1H), 2.55-2.7 (m, 1H), 5.0 (s, 2H), 7.05-7.13 (m, 4H), 7.24-7.3 (1H), 7.43 (d, 1H), 7.84 (td, 1H), 8.47 (dd, 1H), 11.6 (breites s, 1H).

### c) (1R,2R)-2-(4-{[Acetyl(2-pyridinyl)amino]methyl}phenyl)-N-[(1S)-2-amino-2-oxo-1-phenylethyl]cyclohexancarboxamid

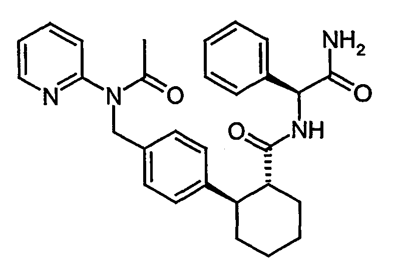

(1*R*,2*R*)-2-(4-{[Acetyl(2-pyridinyl)amino]methyl}phenyl)cyclohexancarbonsäure (0,44 g) wird in DMF (15 mL) suspendiert, mit (2*S*)-2-Amino-2-phenylethanamid (0,37 g), Triethylamin (0,68 mL), 1-Hydroxybenztriazol (0,18 g) und EDC-Hydrochlorid (0,27 g) versetzt und 2 Tage bei Raumtemperatur gerührt. Die Suspension wird mit Wasser verdünnt, mit Dichlormethan extrahiert, die organischen Phasen mit gesättigter Kochsalzlösung gewaschen und über Natriumsulfat getrocknet und eingeengt. Chromatographie (Kieselgel, Dichlormethan:Methanol:gesättigte wässrige Ammoniaklösung = 50:1:0,05 bis 20:1:0,05 liefert (1*R*,2*R*)-2-(4-{[Acetyl(2-pyridinyl)amino]methyl}phenyl)-N-[(1*S*)-2-amino-2-oxo-1-phenylethyl]cyclohexancarboxamid (0,39 g) als farblosen Feststoff.
R_{*f*}(Dichlormethan/Methanol/gesättigte wässrige Ammoniaklösung 40 : 2 : 0,1) = 0,43
MS (FSI) = 485 (M+H⁺); 507 (M+Na)⁺.
¹H-NMR (300MHz, DMSO-d₆): δ[ppm]: 1.23-1.52 (m, 4H), 1.65-1.86 (m, 4H), 2.01 (s, 3H), 2.59-2.84 (m, 2H), 5.04 (s, 2H), 5.15 (d, 1H), 6.78 (d, 2H), 6.97-7.11 (m, 8H), 7.24-7.29 (m, 1H), 7.43 (d, 1H), 7.57 (s, 1H), 7.80 (td, 1H), 7.93 (d, 1H), 8.45-8.48 (m, 1H).

Die in der folgenden Tabelle 1 aufgeführten Verbindungen werden in analoger Weise hergestellt:

**Tabelle 1**

| **Beispiel** | **Struktur** | **Retentionszeit (Methode)** |
|---|---|---|
| 1-2 | 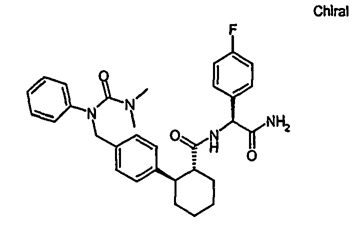 | 7,49 (D) |
| 1-3 | 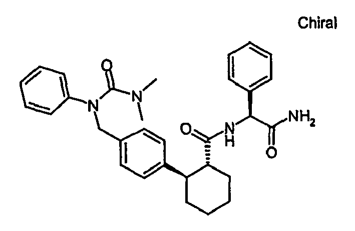 | 7,17 (D) |
| 1-4 | 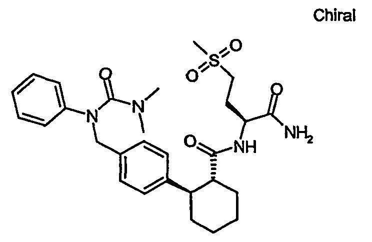 | 4,15 (A) |
| 1-5 | 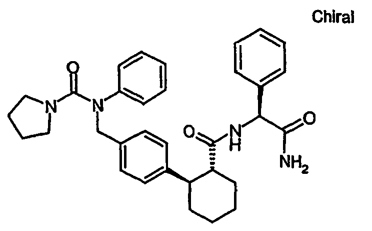 | 4,63 (A) |
| 1-6 | 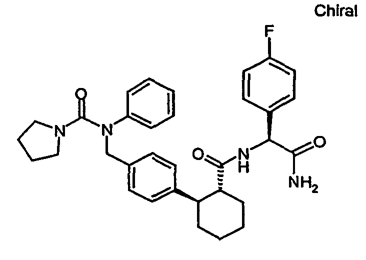 | 4,69 (A) |
| 1-7 | 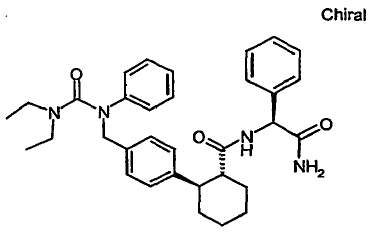 | 4,80 (A) |
| 1-8 | 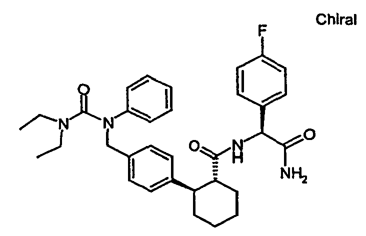 | 4,86 (A) |
| 1-9 | 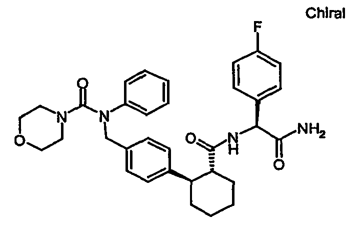 | 4,44 (A) |
| 1-10 | 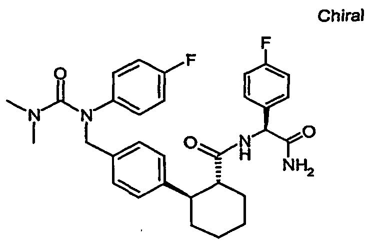 | 4,57 (A) |
| 1-11 | 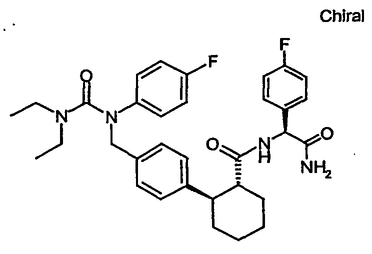 | 4,87 (A) |
| 1-12 | 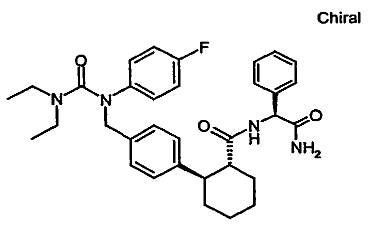 | 4,82 (A) |
| 1-13 | 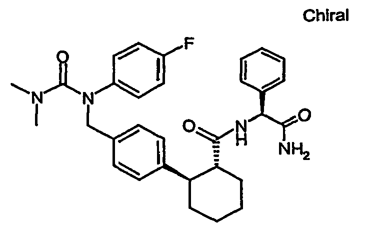 | 4,52 (A) |
| 1-14 | 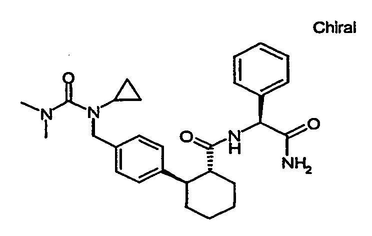 | 4,15 (A) |
| 1-15 | 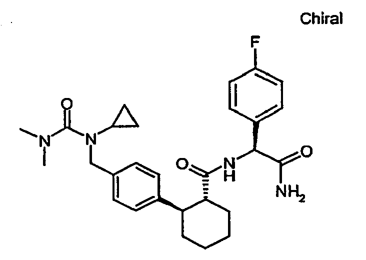 | 4,21 (A) |
| 1-16 | 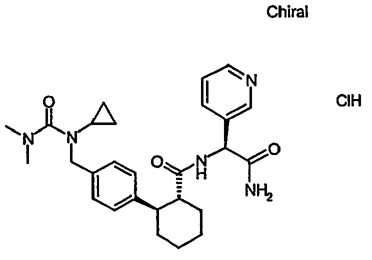 | 4,01 (A) |
| 1-17 | 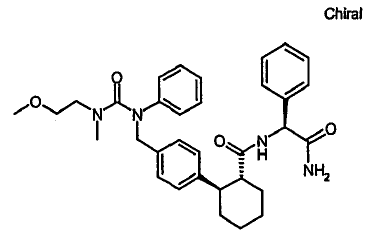 | 4,52 (A) |
| 1-18 | 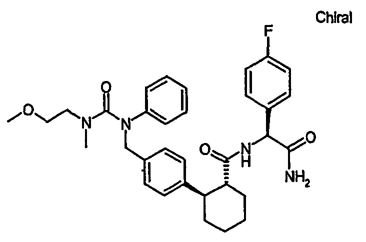 | 4,58 (A) |
| 1-19 | 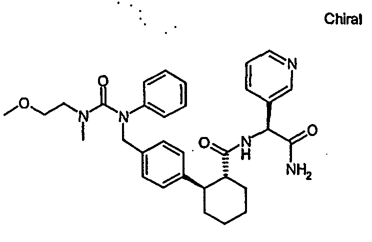 | 4,33 (A) |
| 1-20 | 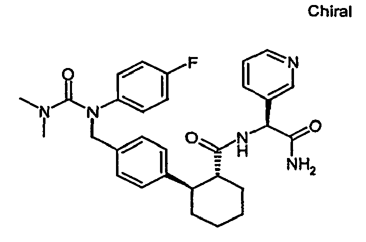 | 4,30 (A) |
| 1-21 | 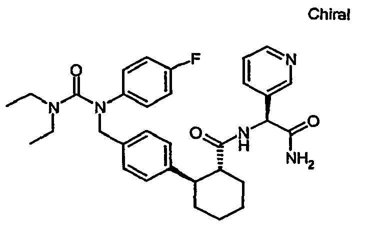 | 4,53 (A) |
| 1-22 | 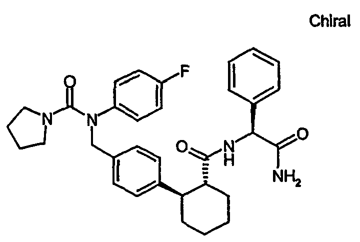 | 4,64 (A) |
| 1-23 | 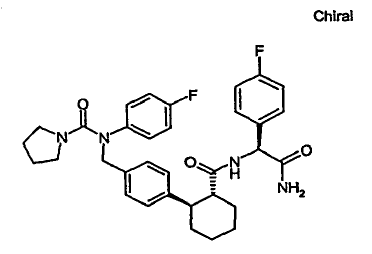 | 4,69 (A) |
| 1-24 | 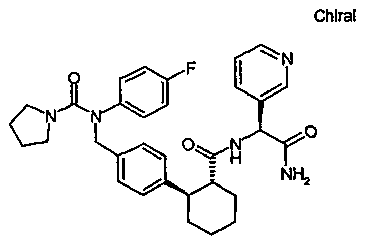 | 4,43 (B) |
| 1-25 | 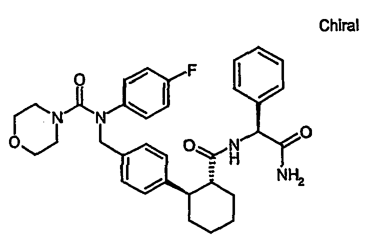 | 4,41 (A) |
| 1-26 | 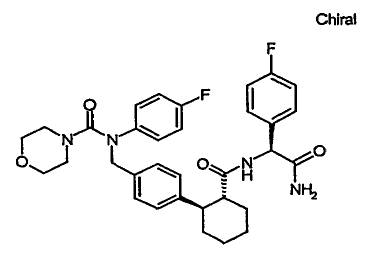 | 4,46 (A) |
| 1-27 | 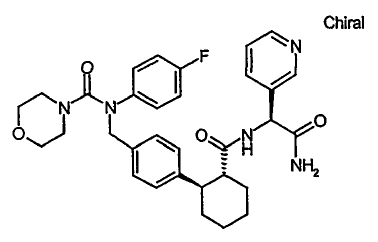 | 4,17 (B) |
| 1-28 | 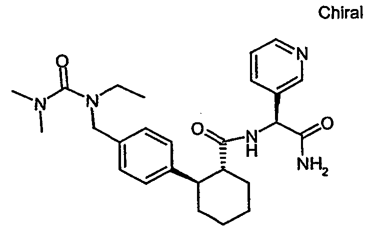 | 3,96 (A) |
| 1-29 | 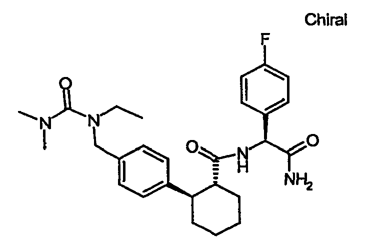 | 4,18 (A) |
| 1-30 | 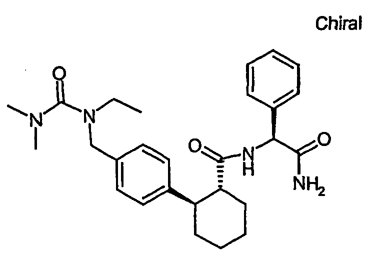 | 4,12 (A) |
| 1-31 | 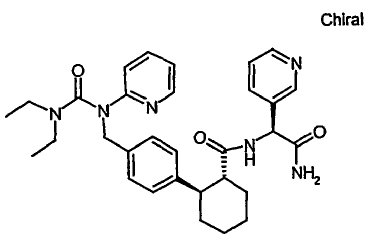 | 3,67 (A) |
| 1-32 | 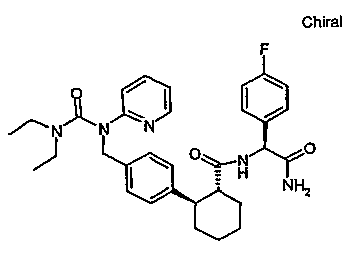 | 3,91 (A) |
| 1-33 | 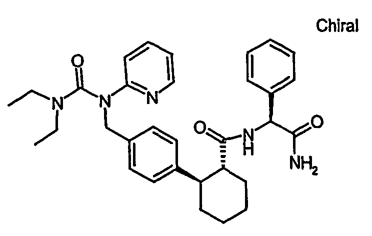 | 3,87 (A) |

### Beispiel 2-1

### (1R,2R)-N-[(1S)-2-Amino-1-(4-fluorphenyl)-2-oxoethyl]-2-(4-{[{[ethyl(2-hydroxyethyl)amino]carbonyl}(4-fluorphenyl)amino]methyl}phenyl)cyclohexancarbonsäureamid:

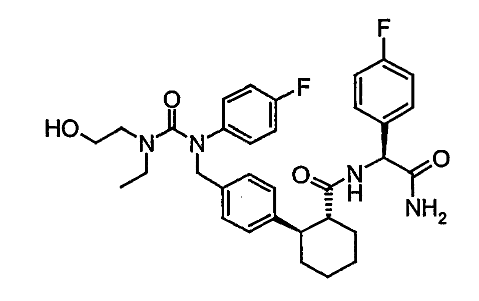

### a) N-Ethyl-N'-(4-fluorphenyl)-N-(2-hydroxyethyl)harnstoff:

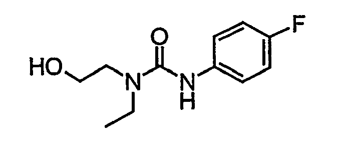

Eine Lösung von 720 mg (8,02 mmol) N-Ethylethanolamin in 4 ml Dichlormethan wird bei 0°C tropfenweise mit 1,00 g (7,29 mmol) 4-Fluorphenylisocyanat versetzt. Nach 10 min bei 0°C wird 2 Stunden bei Raumtemperatur gerührt. Die Lösung wird im Vakuum eingeengt, der Rückstand in 20 ml Dichlormethan gelöst und mit 400 mg Amberlyst^{®} 15 versetzt. Es wird 15 min bei Raumtemperatur gerührt, abfiltriert und im Vakuum eingeengt. Man erhält 1,50 g (91% der Theorie) des Produktes als gelbes Öl.
R_{*f*}(Dichlormethan/Methanol 40:1) = 0,29.
MS (ESI) = 227 (M+H)⁺.
¹H-NMR (400 MHz, DMSO- d₆) δ[ppm]: 1,07 (3H, t), 3,28-3,39 (4H, m), 3,56 (2H, t), 5,16 (1H, t), 7,0-7,09 (2H, m), 7,37-7,43 (2H, m), 8,44 (1H, br. s).

### b) N-(2-{[tert.-Butyl(dimethyl)silyl]oxy}ethyl)-N-ethyl-N'-(4-fluorphenyl)-harnstoff:

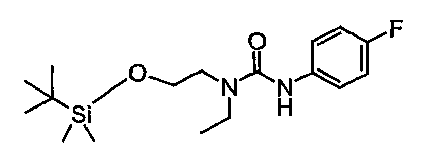

Eine Lösung von 1,41 g (6,23 mmol) der Verbindung aus Beispiel 2-1a in einem Gemisch aus 10 ml Dichlormethan und 1,5 ml abs. DMF wird mit 1,30 ml (9,35 mmol) Triethylamin und einer Lösung von 1,03 g (6,86 mmol) TBDMS-Chlorid in 5 ml Dichlormethan versetzt. Es wird 18 Stunden bei Raumtemperatur gerührt und mit 30 ml Dichlormethan verdünnt. Es wird dreimal mit je 30 ml Wasser gewaschen, mit Natriumsulfat getrocknet und im Vakuum eingeengt. Man erhält 2,00 g (94 % der Theorie) farbloses Öl.
R_{*f*} (Dichlormethan/Methanol 40:1) = 0,74.
MS (ESI) = 341 (M+H)⁺.
¹H-NMR (200 MHz, DMSO- d₆) δ[ppm]: 0,00 (6H, s), 0,81 (9H, s), 1,04 (3H, t), 3,27-3,41 (4H, m), 3,67 (2H, t), 6,94-7,09 (2H, m), 7,34-7,46 (2H, m), 8,15 (1H, br. s).

### c) (1R,2R)-2-{4-[4-Ethyl-2-(4-fluorphenyl)-8,8,9,9-tetramethyl-3-oxo-7-oxa-2,4-diaza-8-siladec-1-yl]phenyl}cyclohexancarbonsäure-tert.-butylester:

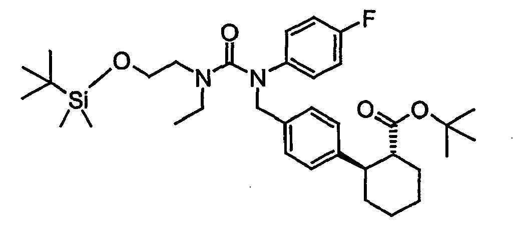

Zu einer Suspension von 54,6 mg (60 % in Mineralöl, 1,37 mmol) Natriumhydrid in 1 ml DMF wird eine Lösung von 443 mg (1,30 mmol) der Verbindung aus Beispiel 2-1b in 3 ml DMF getropft. Nach 45 min Rühren bei Raumtemperatur wird eine Suspension von 510 mg (90 %, 1,30 mmol) (1*R*,2*R*)-2-(4-Brommethyl-phenyl)-cyclohexan-1-carbonsäure-tert.-butylester aus Beispiel I in 3 ml DMF zugetropft. Nach 4 Stunden Rühren bei Raumtemperatur wird mit 50 ml Wasser verdünnt und dreimal mit je 30 ml Diethylether extrahiert. Die vereinigten organischen Phasen werden mit 100 ml gesättigter Kochsalz-Lösung gewaschen und über Natriumsulfat getrocknet. Das Rohprodukt wird durch Säulenchromatographie (Kieselgel (70-230 mesh), Gradient: von Cyclohexan zu Cyclohexan/Essigester 5:1) gereinigt. Man erhält 621 mg (78 % der Theorie) des Produktes als farbloses Öl.
R_{*f*} (Dichlormethan/Methanol 40:1) = 0,78.
MS (ESI) = 613 (M+H)⁺.
¹H-NMR (200 MHz, DMSO- d₆) δ[ppm]: 0,06 (6H, s), 0,83 (3H, t), 0,84 (9H, s), 0,99 (9H, s), 1,20-1,52 (4H, m), 1,59-1,92 (4H, m), 2,32-2,61 (2H, m), 3,06 (2H, q), 3,15 (3H, t), 3,51 (2H, t), 4,67 (2H, s), 7,00-7,22 (8H, m).

### d) (1R,2R)-2-(4-{[{[Ethyl(2-hydroxyethyl)amino]carbonyl}(4-fluorphenyl)-amino]methyl}phenyl)cyclohexancarbonsäure-tert.-butylester:

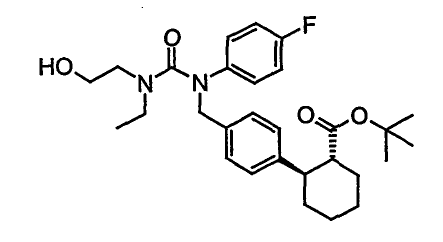

Zu einer Lösung von 246 mg (0,40 mmol) der Verbindung aus Beispiel 2-1c in 20 ml THF werden 345 µl (1,20 mmol) einer 1,1 M Lösung von Tetra-*n*-butylammoniumfluorid in THF gegeben. Man rührt 4 Stunden bei Raumtemperatur und verdünnt anschließend mit 100 ml Diethylether. Es wird dreimal mit je 25 ml einer halbgesättigten Kochsalzlösung und einmal mit 20 ml ges. Kochsalzlösung gewaschen. Die vereinigten Waschlösungen werden mit 20 ml Diethylether extrahiert und die vereinigten organischen Phasen mit Natriumsulfat getrocknet. Das Rohprodukt wird durch Säulenchromatographie (Kieselgel (70-230 mesh), Gradient: von Cyclohexan zu Cyclohexan/Essigester 1:1) gereinigt. Ausbeute: 201 mg farbloses Öl (95 % der Theorie)
R_{*f*}(Cyclohexan/Essigester 1:1) = 0,29.
MS (ESI) = 499 (M+H)⁺.
¹H-NMR (200 MHz, DMSO- d₆) δ[ppm]: 0,83 (3H, t), 1,00 (9H, s), 1,20-1,52 (4H, m), 1,60-1,92 (4H, m), 2,32-2,61 (2H, m), 2,91-3.17 (4H, m), 3,36 (2H, t), 4,61 (1H, t), 4,66 (2H, s), 7,00-7,22 (8H, m).

### e) (1R,2R)-2-(4-{[{[Ethyl(2-hydroxyethyl)amino]carbonyl}(4-fluorphenyl)-amino]methyl}phenyl)cyclohexancarbonsäure:

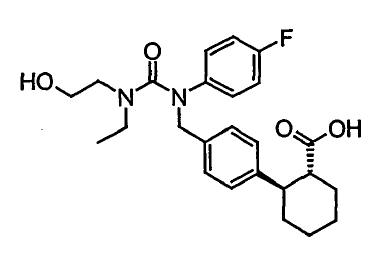

Eine Lösung von 200 mg (0,40 mmol) der Verbindung aus Beispiel 2-1d wird in 2 ml Dichlormethan gelöst und mit 1 ml Trifluoressigsäure versetzt. Man bewahrt die Lösung 16 Stunden bei 6°C auf und versetzt dann mit 15 ml 1 N Natriumhydroxidlösung und 20 ml Wasser. Es wird zweimal mit je 20 ml Diethylether gewaschen und mit 1 N Salzsäure auf pH 3-4 eingestellt. Man extrahiert dreimal mit je 30 ml Dichlormethan und trocknet mit Natriumsulfat. Der Rückstand wird in 4 ml Diethylether aufgenommen und wieder eingedampft. Dabei wird aus dem anfänglichen Öl ein weißer Hartschaum. Ausbeute: 146 mg (78 % der Theorie, 94 % Reinheit laut HPLC).
R_{*f*} (Dichlormethan/Methanol 40:1) = 0,44.
MS (ESI) = 443 (M+H)⁺.
¹H-NMR (200 MHz, DMSO- d₆) δ[ppm]: 0,83 (3H, t), 1,20-1,52 (4H, m), 1,60-1,82 (3H, m), 1,88-2,02 (1H, m), 2,40-2,72 (2H, m), 2,99-3,20 (4H, m), 3,34 (2H, t), 4,64 (2H, s), 7,03-7,20 (8H, m), 11,70 (1H, br. s)

### f) (1R,2R)-N-[(1S)-2-Amino-1-(4-fluorphenyl)-2-oxoethyl]-2-(4-{[{[ethyl(2-hydroxyethyl)amino]carbonyl}(4-fluorphenyl)amino]methyl}phenyl)-cyclohexancarbonsäureamid:

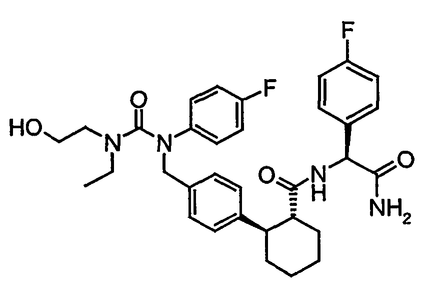

Ein Gemisch aus 44,3 mg (0,100 mmol) der Verbindung aus Beispiel 2-1e, 13,5 mg (0,100 mmol) HOBT, 23,0 mg (0,120 mmol) EDC und 1 mg DMAP werden mit 0,90 ml abs. DMF versetzt. Man rührt 5 min bei Raumtemperatur bis sich eine klare Lösung gebildet hat. Dann werden 22,0 µl (20,2 mg, 0,200 mmol) *N*-Methyl-morpholin und 30,7mg (0,150 mmol) L-4-Fluorphenylglycinamid-hydrochlorid zugegeben. Man lässt 3 Tage bei Raumtemperatur rühren und trennt dann direkt über RP-HPLC (C18 Gromsil, 50 x 20 mm, 25 ml/min, Gradient Wasser/Acetonitril 90:10 → Wasser/Acetonitril 10:90 in 8 min). Das Acetonitril wird im Vakuum entfernt wobei das Produkt in Form weißer Flocken ausfällt. Es wird eingefroren und über Nacht lyophilisiert. Man erhält 37,9 mg (64 % der Theorie) des Produktes als weißen Feststoff.
R_{*f*}(Dichlormethan/Methanol 10:1) = 0,37.
MS (ESI) = 593 (M+H)⁺.
¹H-NMR (300 MHz, DMSO- d₆) δ[ppm]: 0,82 (3H, t), 1,20-1,53 (4H, m), 1,61-1,87 (4H, m), 2,56-2,69 (1H, m), 2,75-2,87 (1H, m), 3,05 (2H, q), 3,13 (2H, t), 4,68 (2H, dd), 5,13-5,20 (1H, m), 6,79-6,87 (2H, m), 6,92 (2H, t), 7,02-7,18 (9H, m), 7,63 (1H, br s), 8,05 (1H, d).

### Beispiel 2-18

### (1R,2R)-N-[(1S)-2-Amino-1-phenyl-2-oxoethyl]-2-(4-{[{[ethyl(2-hydroxyethyl)-amino]carbonyl}(phenyl)amino]methyl}phenyl)cyclohezancarbonsäureamid:

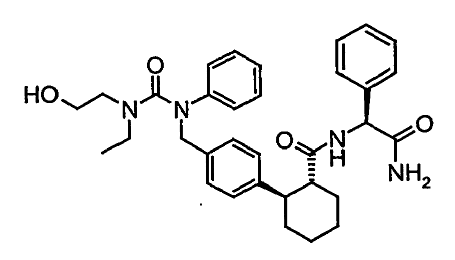

### a) N-Ethyl-N'-Phenyl-N-(2-hydrozyethyl)harnstoff:

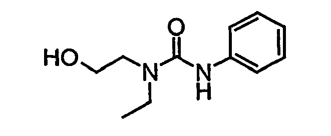

Eine Lösung von 820 mg (9,23 mmol) N-Ethylethanolamin in 4 ml Dichlormethan wird bei 0°C tropfenweise mit einer Lösung von 1,00 g (8,39 mmol) Phenylisocyanat in 2 ml Dichlormethan versetzt. Nach 10 min bei 0°C wird 16 Stunden bei Raumtemperatur gerührt. Die Lösung wird im Vakuum eingeengt und der feste Rückstand dreimal mit je 20 ml Diethylether gewaschen. Man erhält 1,71 g (98 % der Theorie) des Produktes als weißen Feststoff.
R_{*f*} (Dichlormethan/Methanol 20:1) = 0,17.
MS (ESI) = (208 M)⁺.
¹H-NMR (300 MHz, DMSO- d₆) δ[ppm]: 1,08 (3H, t), 3,28-3,39 (4H, m), 3,57 (2H, q), 5,17 (1H, t), 6,91 (1H, t), 7,22 (2H, t), 7,39 (2H, d), 8,44 (1H, br. s).

### b) N-(2-{[tert.-Butyl(dimethyl)silyl]oxy}ethyl)-N-ethyl-N'-phenylharnstoff:

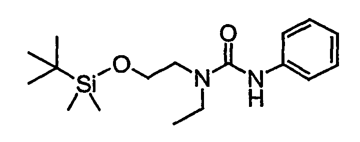

Eine Lösung von 1,69 g (8,12 mmol) der Verbindung aus Beispiel 2-18a in einem Gemisch aus 10 ml Dichlormethan und 3,0 ml abs. Dimethylformamid wird mit 1,70 ml (12,2 mmol) Triethylamin und einer Lösung von 1,35 g (8,93 mmol) TBDMS-Chlorid in 5 ml Dichlormethan versetzt. Es wird 18 Stunden bei Raumtemperatur gerührt und mit 100 ml Diethylether verdünnt. Es wird dreimal mit je 30 ml Wasser gewaschen, mit Natriumsulfat getrocknet und im Vakuum eingeengt. Man erhält 2,62 g (95 % der Theorie) farbloses Öl.
R_{*f*} (Dichlormethan/Methanol 20:1) = 0,67.
MS (ESI) = 323 (M+H)⁺.
¹H-NMR (200 MHz, DMSO- d₆) δ[ppm]: 0,04 (6H, s), 0,85 (9H, s), 1,08 (3H, t), 3,33-3,45 (4H, m), 3,72 (2H, t), 6,92 (1H, t), 7,22 (2H, t), 7,42 (2H, d), 8,13 (1H, br. s).

### c) (1R,2R)-2-{4-[4-Ethyl-2-phenyl-8,8,9,9-tetramethyl-3-oxo-7-oxa-2,4-diaza-8-siladec-1-yl]phenyl}cyclohexancarbonsäure-tert.-butylester:

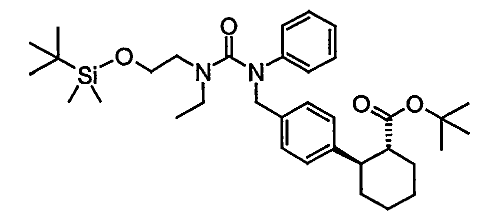

Zu einer Suspension von 54,6 mg (60 % in Mineralöl, 1,37 mmol) Natriumhydrid in 1 ml DMF wird eine Lösung von 419 mg (1,30 mmol) der Verbindung aus Beispiel 2-18b in 3 ml DMF getropft. Nach 15 min Rühren bei Raumtemperatur wird eine Suspension von 535 mg (85,8 %, 1,30 mmol) (1*R*,2*R*)-2-(4-Brommethyl-phenyl)-cyclohexan-1-carbonsäure-tert.-butylester aus Beispiel I in 4 ml DMF zugetropft. Nach 20 Stunden Rühren bei Raumtemperatur wird mit 50 ml Wasser verdünnt und dreimal mit je 30 ml Diethylether extrahiert. Die vereinigten organischen Phasen werden mit 100 ml gesättigter Kochsalz-Lösung gewaschen und über Natriumsulfat getrocknet. Das Rohprodukt wird durch Säulenchromatographie (Kieselgel (70-230 mesh), Dichlormethan/Ethanol 40:1) gereinigt. Man erhält 757 mg (91 % der Theorie) des Produktes als farbloses Öl.
R_{*f*}(Petrolether/Ethylacetat 4:1) = 0,57.
MS (ESI) = 595 (M+H)⁺.
¹H-NMR (200 MHz, DMSO- d₆) δ[ppm]: 0,00 (6H, s), 0,80-0,85 (12H, m), 0,99 (9H, s), 1,20-1,52 (4H, m), 1,59-1,92 (4H, m), 2,32-2,61 (2H, m), 3,06 (2H, q), 3,15 (3H, t), 3,51 (2H, t), 4,67 (2H, s), 7,00-7,27 (9H, m).

### d) (1R,2R)-2-(4-{[{[Ethyl(2-hydroxyethyl)amino]carbonyl}(phenyl)-amino]methyl}phenyl)cyclohexancarbonsäure-tert.-butylester:

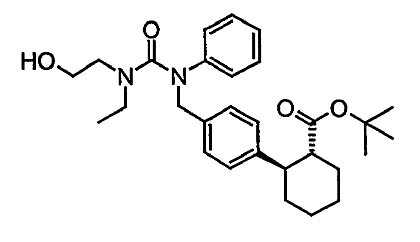

Zu einer Lösung von 724 mg (1,22 mmol) der Verbindung aus Beispiel 2-18c in 20 ml THF werden 350 µl (1,22 mmol) einer 1,1 M Lösung von Tetra-*n*-butylammoniumfluorid in THF gegeben. Man rührt 30 min bei Raumtemperatur und verdünnt anschließend mit 100 ml Diethylether. Es wird dreimal mit je 25 ml einer halbgesättigten Kochsalzlösung und einmal mit 20 ml gesättigter Kochsalzlösung gewaschen. Die vereinigten Waschlösungen werden mit 20 ml Diethylether extrahiert und die vereinigten organischen Phasen mit Natriumsulfat getrocknet. Ausbeute: 747 mg farbloses Öl (enthält noch tert.-Butyl(dimethyl)silylfluorid). Zur Charakterisierung wurde eine kleine Menge durch Säulenchromatographie (Kieselgel (70-230 mesh), Gradient: von Cyclohexan zu Cyclohexan/Essigester 1:1) aufgereinigt.
R_{*f*}(Cyclohexan/Essigester 1:1) = 0,4.
MS (ESI) = 481 (M+H)⁺.
¹H-NMR (200 MHz, DMSO- d₆) δ[ppm]: 0,83 (3H, t), 1,00 (9H, s), 1,20-1,52 (4H, m), 1,60-1,92 (4H, m), 2,35-2,61 (2H, m), 2,95-3,17 (4H, m), 3,29-3,43 (2H, m), 4,59 (1H, t), 4,68 (2H, s), 6,98-7,29 (9H, m).

### e) (1R,2R)-2-(4-{[{[Ethyl(2-hydroxyethyl)amino]carbonyl}(phenyl)-amino]methyl}phenyl)cyclohexancarbonsäure:

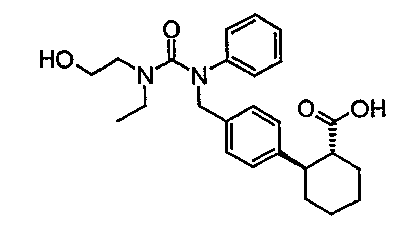

Eine Lösung von 724 mg (1,51 mmol) der Verbindung aus Beispiel 2-18d wird in 2 ml Dichlormethan gelöst und mit 1 ml Trifluoressigsäure versetzt. Man bewahrt die Lösung 5 Stunden bei Raumtemperatur auf und versetzt dann mit 15 ml 1 N Natriumhydroxidlösung und 20 ml Wasser. Es wird zweimal mit je 20 ml Diethylether gewaschen und mit 1 N Salzsäure auf pH 3-4 eingestellt. Man extrahiert dreimal mit je 30 ml Dichlormethan und trocknet mit Natriumsulfat. Der Rückstand wird in 4 ml Diethylether aufgenommen und wieder eingedampft. Dabei wird aus dem anfänglichen Öl ein weißer Hartschaum. Ausbeute: 276 mg (43 % der Theorie, 99 % Reinheit laut HPLC).
R_{*f*}(Dichlormethan/Methanol 10:1) = 0,35.
MS (ESI) = 425 (M+H)⁺.
¹H-NMR (200 MHz, DMSO- d₆) δ[ppm]: 0,83 (3H, t), 1,20-1,55 (4H, m), 1,59-1,82 (3H, m), 1,88-2,02 (1H, m), 2,40-2,72 (2H, m), 2,99-3,20 (4H, m), 3,25-3,50 (2H, m), 4,68 (2H, s), 7,00-7,32 (9H, m), 11,74 (1H, br. s)

### f) (1R,2R)-N-[(1S)-2-Amino-1-phenyl-2-oxoethyl]-2-(4-{[{[ethyl(2-hydroxyethyl)amino]carbonyl}(phenyl)amino]methyl}phenyl)cyclohexancarbonsäureamid:

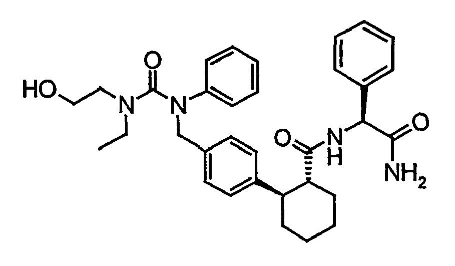

Ein Gemisch aus 42,5 mg (0,100 mmol) der Verbindung aus Beispiel 2-18e, 13,5 mg (0,100 mmol) HOBT, 23,0 mg (0,120 mmol) EDC und 1 mg DMAP werden mit 0,90 ml abs. DMF versetzt. Man rührt 5 min bei Raumtemperatur bis sich eine klare Lösung gebildet hat. Dann werden 22,0 µl (20,2 mg, 0,200 mmol) *N*-Methylmorpholin und 28 mg (0,150 mmol) L-Phenylglycinamid-hydrochlorid zugegeben. Man läßt 3 Tage bei Raumtemperatur rühren und trennt dann direkt über RP-HPLC (C18 Gromsil, 50 x 20 mm, 25 ml/min, Gradient Wasser/Acetonitril 90:10 → Wasser/Acetonitril 10:90 in 8 min). Das Acetonitril wird im Vakuum entfernt wobei das Produkt in Form weißer Flocken ausfällt. Es wird eingefroren und über Nacht lyophilisiert. Man erhält 37,6 mg (62 % der Theorie) des Produktes als weißen Feststoff.
R_{*f*}(Dichlormethan/Methanol 10:1) = 0,44.
MS (ESI) = 557 (M+H)⁺.
¹H-NMR (200 MHz, DMSO- d₆) δ[ppm]: 0,84 (3H, t), 1,19-1,58 (4H, m), 1,61-1;88 (4H, m), 2,56-2,90 (2H, m), 3,00-3,23 (4H, m), 3,25-3,50 (2H, m), 4,65 (1H, t), 4,72 (2H, s), 5,17 (1H, d), 6,79-6,87 (2H, m), 6,95-7,30 (13H, m), 7,65 (1H, br s), 8,02 (1H, d).

Die in der folgenden Tabelle 2 aufgeführten Verbindungen werden in analoger Weise hergestellt, wobei zusätzlich zu den in der Tabelle angegebenen Daten weitere spektroskopische Daten einzelner Verbindungen vorab aufgeführt sind:
**(*S*)-N-{{(1*R*,2*R*)-2-(4-{[{[Bis(2-hydroxyethyl)amino]carbonyl}(phenyl)amino]-methyl}phenyl)cyclohex-1-yl}carbonyl}-phenylglycinamid (Beispiel 2-3)**
   MS (ESI) = 573 (M+H)⁺.
   ¹H-NMR (200 MHz, DMSO- d₆) δ[ppm]: 1,21-1,60 (4H, m), 1,61-1,96 (4H, m), 2,60-2,93 (2H, m), 3,19.(4H, t), 3,39 (4H, t), 4,76 (2H, s), 5,15-5,25 (1H, m), 6,82-6,93 (2H, m), 6,90-7,35 (13H, m), 7,66 (1H, br. s), 8,04 (1H, d).
**(*S*)-N-{{(1*R*,2*R*)-2-(4-{[{[2-Hydroxyethylamino]carbonyl}(phenyl)amino]-methyl}phenyl)cyclohex-1-yl}carbonyl}-phenylglycinamid** (Beispiel 2-17)
   MS (ESI) = 551 (M+H)⁺.
   ¹H-NMR (300 MHz, DMSO- d₆) δ[ppm]: 1,20-1,55 (4H, m), 1,62-1,89 (4H, m),
   2,60-2,70 (1H, m), 2,77-2,86 (1H, m), 3,12 (2H, q) 3,32-3.40 (2H, m), 4,59 (1H, t), 4,80 (1H, s), 5,17 (1H, d), 5,66 (1H, t), 6,76-6,83 (2H, m), 6,97-7,23 (11H, m), 7,33 (2H, t), 7,65 (1H, br. s), 8,02 (1H, d).

**Tabelle 2**

| **Beispiel** | **Struktur** | **Retentionszeit (Methode)** |
|---|---|---|
| 2-2 | 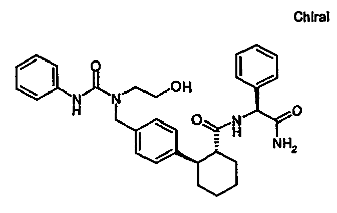 | 4,18 (A) |
| 2-3 | 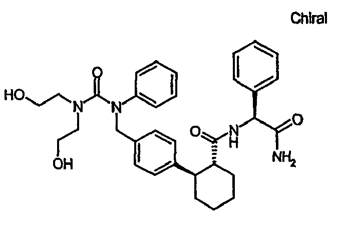 | 3,97 (A) |
| 2-4 | 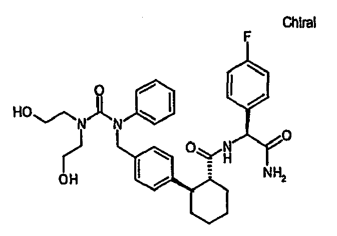 | 4,01 (A) |
| 2-5 | 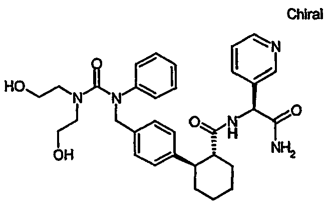 | 3,89 (A) |
| 2-6 | 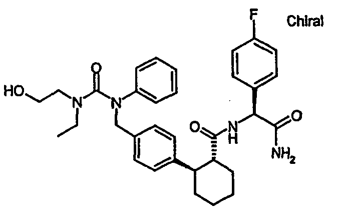 | 4,36 (A) |
| 2-7 | 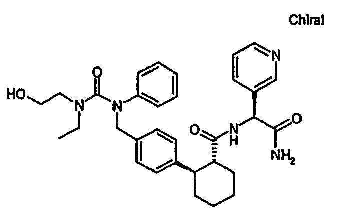 | 4,17 (A) |
| 2-8 | 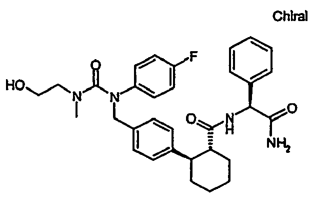 | 4,24 (A) |
| 2-9 | 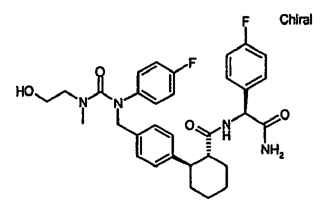 | 4,29 (A) |
| 2-10 | 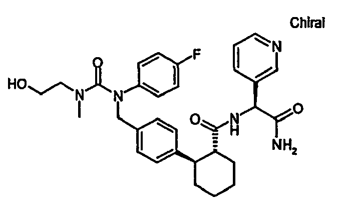 | 4,12 (B) |
| 2-11 | 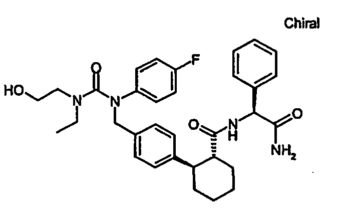 | 4,36 (A) |
| 2-12 | 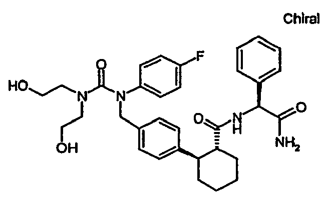 | 3,98 (A) |
| 2-13 | 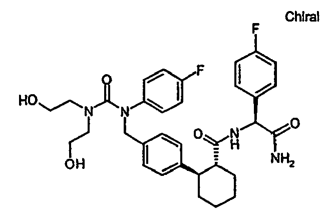 | 4,03 (A) |
| 2-14 | 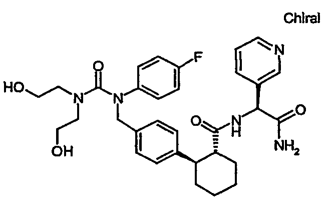 | 3,90 (A) |
| 2-15 | 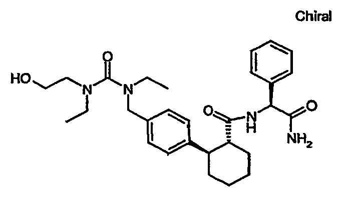 | 4,00 (A) |
| 2-16 | 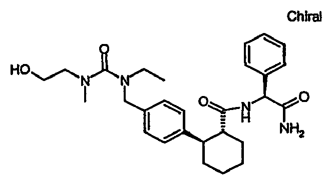 | 3,90 (A) |
| 2-17 | 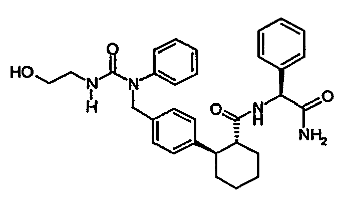 | 3,92 (A) |

### Beispiel 3-1

### 4-[(1R,2R)-2-({[(1S)-2-Amino-1-(4-fluorphenyl)-2-oxoethyl]amino}carbonyl)-cyclohexyl]benzyl 4-hydroxy-1-piperidincarbamat:

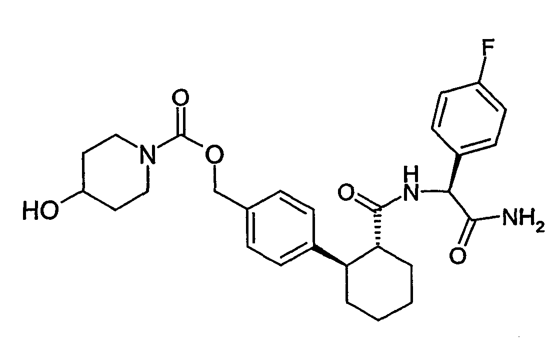

### a) (1R,2R)-2-{4-[(acetyloxy)methyl]phenyl}cyclohexancarbonsäure-tert.-butylester:

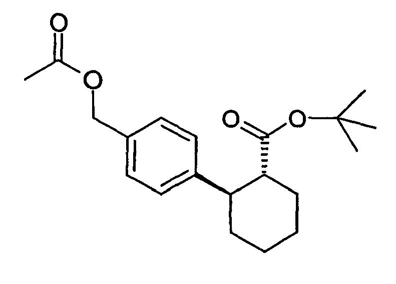

Einen Suspension von (1*R*,2*R*)-2-(4-Brommethyl-phenyl)-cyclohexan-1-carbonsäure-tert.-butylester aus Beispiel I (3 g, 8,49 mmol), Kaliumacetat (1,83 g, 18,68 mmol) und 18-Krone-6 (134,7 mg, 0,51 mmol) in Acetonitril (15 ml) wird 24 Stunden bei 50°C und weitere 16 Stunden bei 60°C erhitzt. Die Reaktionsmischung wird anschließend im Vakuum eingeengt und mit Wasser/Methylenchlorid extrahiert. Die organische Phase wird mit gesättigter Natriumchlorid-Lösung gewaschen und über Natriumsulfat getrocknet. Nach Konzentration des Rohproduktes im Vakuum und Chromatographie an Kieselgel (Eluens: Cyclohexan/Essigester = 30:1 bis 8:1) werden 2,7 g (95,6 %) Produkt als farbloser Feststoff erhalten.
MS (ESI+): 350.4 (M+NH₄)⁺
¹H-NMR (DMSO- d₆): 1.05 (9 H, s); 1.30-1.55 (4 H, m); 1.65-1.95 (4 H, m); 2.02 (3 H, s); 2.40-2.68 (2 H, m); 5.02 (2 H, s); 7.15-7.28 (4 H, m).

### b) (1R,2R)-2-{4-[(Acetyloxy)methyl]phenyl}cyclohexancarbonsäure:

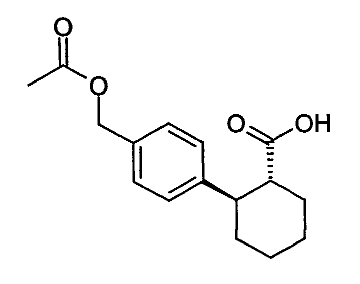

Eine Lösung der Verbindung aus Beispiel 3-1a (2,7 g, 8,12 mmol) in Dichlormethan (15 ml) und Trifluoressigsäure (7,5 ml) wird 2 Stunden bei Raumtemperatur gerührt. Nach Konzentration des Gemisches im Vakuum, Extraktion mit Methylenchlorid/-Wasser und anschließend zweimal mit gesättigter Kochsalz-Lösung werden nach Konzentration der organischen Phase im Vakuum 2,2 g (94 %) Produkt als erstarrtes Öl erhalten.
MS (ESI+): 294.3 (M+NH₄)⁺
¹H-NMR (DMSO- d₆): 1.30-1.55 (4 H, m); 1.65-2.02 (4 H, m); 2.03 (3 H, s); 2.40-2.75 (2 H, m); 5.00 (2 H, s); 7.15-7.28 (4 H, m); 11.71 (1 H, s).

### c) 4-[(1R,2R)-2-({[(1S)-2-Amino-1-(4-fluorphenyl)-2-oxoethyl]amino}-carbonyl)cyclohexyl]benzylacetat:

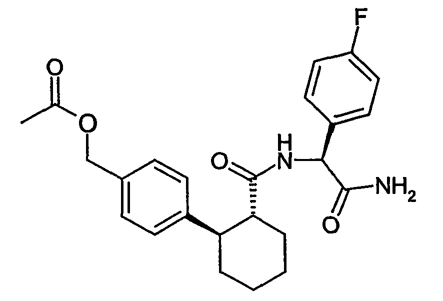

Zu einer Lösung der Verbindung aus Beispiel 3-1b (2,2 g, 7,96 mmol) in DMF (80 ml) werden 1-Hydroxybenzotriazol (1,18 g, 8,76 mmol) und EDC (1,60 g, 8,36 mmol) gegeben und 10 min bei Raumtemperatur gerührt. Anschließend werden N-Methylmorpholin (3,50 ml, 31,85 mmol), (+)-(*S*)-4-Fluorphenylglycinamid (1,63 g, 7,96 mmol) und eine Spatelspitze DMAP hinzugegeben und über Nacht bei Raumtemperatur gerührt. Nach Zugabe von Wasser (350 ml) wird 1 Stunde bei Raumtemperatur nachgerührt und die Mischung eisgekühlt. Die Titelverbindung wird anschließend abfiltriert, mit Wasser und Diethylether nachgewaschen und getrocknet. Nach Trocknung im Vakuum (200 mbar, 50°C, 16 h) erhält man 2,70 g (74,8 %) des Produktes als farblosen Feststoff.
MS (ESI+): 427.0 (M+H)⁺
¹H-NMR (DMSO- d₆): 1.25-1.55 (4 H, m); 1.65-1.90 (4 H, m); 2.06 (3 H, s); 2.64-2.71 (1 H, m); 2.80-2.90 (1 H, m); 5.02 (2 H, s); 5.20 (1 H, d); 6.75-6.94 (4 H, m); 7.15-7.22 (5 H, m); 7.67 (1 H, s); 8.05 (1 H, d).

### d) (1R,2R)-N-[(1S)-2-Amino-1-(4-fluorphenyl)-2-oxoethyl]-2-[4-(hydroxymethyl)phenyl]cyclohexancarbonsäureamid:

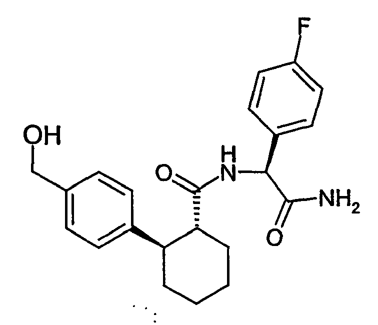

Eine Suspension der Verbindung aus Beispiel 3-1c (2,70 g, 6,61 mmol) in Ammoniaklösung (2M in Methanol, 50 ml) wird über Nacht bei Raumtemperatur gerührt. Nach Konzentration des Gemisches im Vakuum wird das Produkt 1 Stunde mit Diethylether (50 ml) ausgerührt, anschließend eisgekühlt und abfiltriert. Nach Trocknung im Vakuum (200 mbar, 50°C, 16 h) erhält man das Produkt als farblosen Feststoff (2,50 g, 98,4 %).
MS (ESI+): 385.5 (M+H)⁺
¹H-NMR (DMSO- d₆): 1.25-1.55 (4 H, m); 1.65-1.88 (4 H, m); 2.64-2.69 (1 H, m); 2.77-2.85 (1 H, m); 4.45 (2 H, s); 5.17 (1 H, d); 6.70-'6.76 (2 H, m); 6.87-6.94 (2 H, m); 7.10-7.17 (5 H, m); 7.65 (1 H, s); 7.98 (1 H, d).

### e) 4-[(1R,2R)-2-({[(1S)-2-Amino-1-(4-fluorphenyl)-2-oxoethyl]amino}-carbonyl)cyclohexyl]benzyl 4-hydroxy-1-piperidincarbamat:

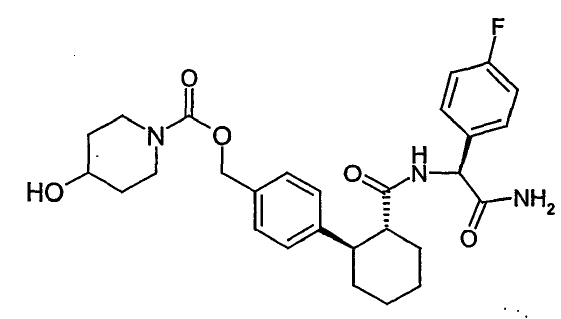

Zu einer Lösung der Verbindung aus Beispiel 3-1d (150,0 mg, 0,39 mmol) in DMF (5 ml) werden Triethylamin (0,16 ml, 1,17 mmol) und Disuccinimidylcarbonat (149,9 mg, 0,59 mmol) gegeben und 5 Stunden bei Raumtemperatur gerührt. Anschließend wird 4-Hydroxypiperidin (157,8 mg, 1,56 mmol) hinzugegeben und 12 Stunden bei Raumtemperatur gerührt. Nach Filtration wird die Lösung direkt mittels präparativer HPLC (Säule: Kromasil 100 C 18,5 µm, 250 x 40 mm; Eluent: Methanol/Wasser; Fluss: 25 ml/min; UV-Detektion bei 210 nm) aufgetrennt. Man erhält nach Konzentration im Vakuum 93,7 mg (45,6 %) des Produktes als farblosen Feststoff.
MS (ESI+): 534.2 (M+Na)⁺
¹H-NMR (DMSO- d₆): 1.15-1.95 (12 H, m); 2.55-2.95 (2 H, m); 2.95-3.16 (2 H, m); 3.55-3.80 (3 H, m); 4.72 (1 H, d); 5.02 (2 H, s); 5.19 (1 H, d); 6.70-6.95 (4 H, m); 7.10-7.25 (5 H, m); 7.70 (1 H, br.s); 8.09 (1 H, d).

### Beispiel 3-23

### 4-[(1R,2R)-2-({[(1S)-2-Amino-1-(4-fluorphenyl)-2-oxoethyl]amino}carbonyl)-cyclohexyl]benzyl 4-(2-hydroxyethyl)-1-piperazinecarbamat:

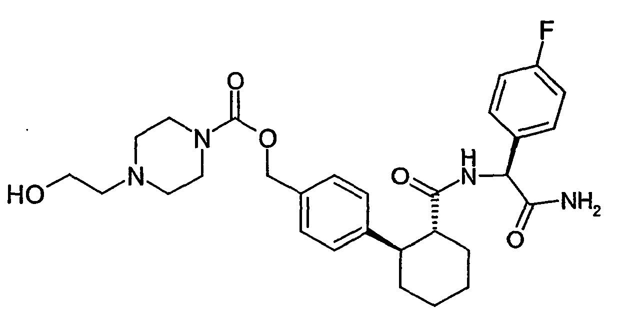

Zu einer Lösung der Verbindung aus Beispiel 3-1d (55 mg, 0,14 mmol) in DMF (2 ml) werden Triethylamin (0,06 ml, 0,43 mmol) und Disuccinimidylcarbonat (73,3 mg, 0,29 mmol) gegeben und über Nacht bei Raumtemperatur gerührt, Die Reaktionsmischung wird mit Methylenchlorid (ca. 10 ml) versetzt und 3 x mit wenig gesättigter Ammoniumchloridlösung gewaschen. Anschließend wird die organische Phase über Natriumsulfat getrocknet und nach Filtration im Vakuum eingeengt. Die so erhaltenen Rohmischung wird zu einer Mischung aus *N*-(2-Hydroxyethyl)-piperazin (74,3 mg, 0,57 mmol) und einer Spatelspitze DMAP gegeben und 12 Stunden bei Raumtemperatur gerührt. Nach Filtration wird die Lösung direkt mittels präparativer HPLC (Säule: Kromasil 100 C 18,5 µm, 250 x 40 mm; Eluent: Acetonitril/Wasser; Fluß: 25 ml/min; UV-Detektion bei 210 nm) aufgetrennt. Man erhält nach Konzentration im Vakuum 25 mg (29,8 %) des Produktes als farblosen Feststoff.
MS (ESI+): 541.3 (M+H)⁺
¹H-NMR (DMSO- d₆): 1.20-1.60 (4 H, m); 1.65-1.90 (4 H, m); 2.25-2.60 (8 H, m); 2.60-2.90 (2 H, m); 3.25-3.54 (4 H, m); 4.38 (1 H, t); 5.03 (2 H, s); 5.19 (1 H, d); 6.72-6.95 (4 H, m); 7.08-7.24 (5 H, m); 7.62 (1 H, s); 8.02 (1 H, d).

### Beispiel 3-36

### 4-[(1R,2R)-2-({[(1S)-2-Amino-1-phenyl-2-oxoethyl]amino}carbonyl)-cyclohexyl]benzyl 4-(2-hydroxyethyl)-1-piperazinecarbamat:

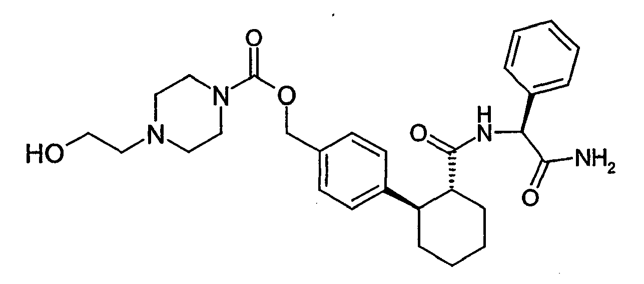

wird analog zu Beispiel 3-23 hergestellt, wobei anstelle von (+)-(*S*)-4-Fluorphenylglycinamid (+)-(*S*)-Phenylglycinamid eingesetzt wird. Die Aufreinigung erfolgt mittels präparativer HPLC (Säule: Waters Symmetry C 18, 7 µm, 300 x 19 mm; Eluent: Acetonitril/Wasser/2% Essigsäure; Fluß: 25 ml/min; UV-Detektion bei 230 nm). Man erhält nach Konzentration im Vakuum das Produkte in Form des essisauren Salzes, aus welchem durch Zugabe von Methylenchlorid und anschließender Extraktion mit einer 1:1 1 Mischung aus gesättigter Natriumchloridlösung und 2 molarer Natriumcarbonatlösung die Verbindung als farbloser Feststoff erhalten wird.
MS (ESI+): 523 [M+H]⁺
¹H-NMR (DMSO- d_{*6*}): 1.20-1.60 (4 H, m); 1.65-1.92 (4 H, m); 2.27-2.60 (8 H, m); 2.60-2.95 (2 H, m); 3.27-3.55 (4 H, m); 4.41 (1 H, t); 5.03 (2 H, s); 5.19 (1 H, d); 6.70-6.85 (2 H, m); 7.0-7.26 (8 H, m); 7.66 (1 H, s); 8.02 (1 H, d).

Die in der folgenden Tabelle 3 aufgeführten Verbindungen werden in analoger Weise hergestellt:

**Tabelle 3**

| **Beispiel** | **Struktur** | **Retentionszeit (Methode)** |
|---|---|---|
| 3-2 | 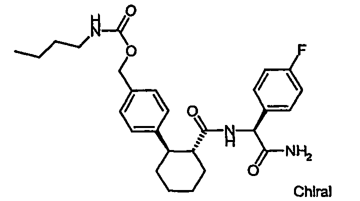 | 4,02 (C) |
| 3-3 | 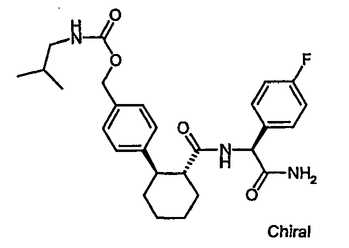 | 4,00 (C) |
| 3-4 | 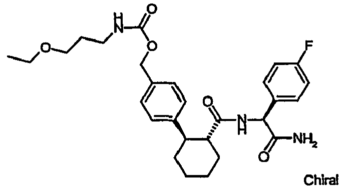 | 3,73 (C) |
| 3-5 | 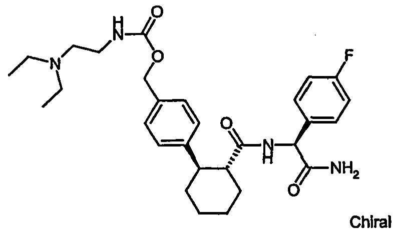 | 2,54 (C) |
| 3-6 | 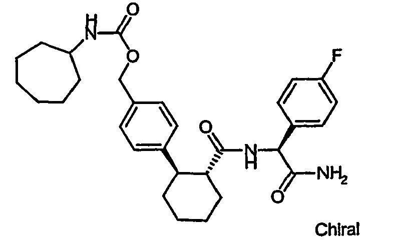 | 4,39 (C) |
| 3-7 | 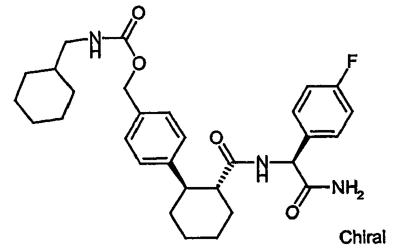 | 4,42 (C) |
| 3-8 | 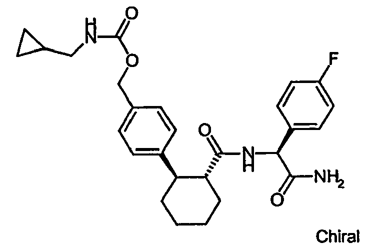 | 3,86 (C) |
| 3-9 | 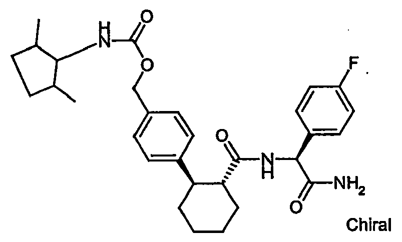 | 4,38 (C) |
| 3-10 | 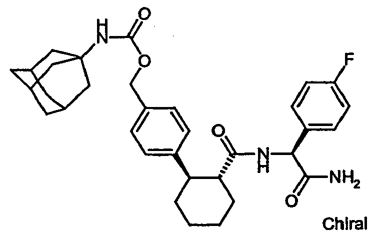 | 4,47 (C) |
| 3-11 | 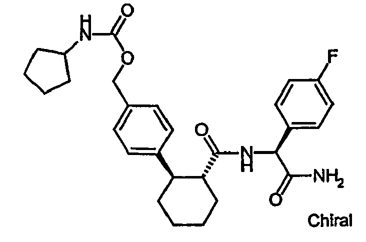 | 4,04 (C) |
| 3-12 | 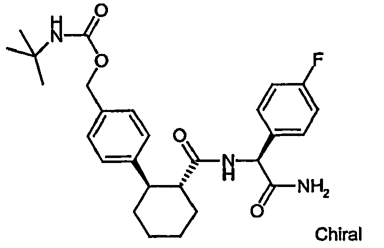 | 4,08 (C) |
| 3-13 | 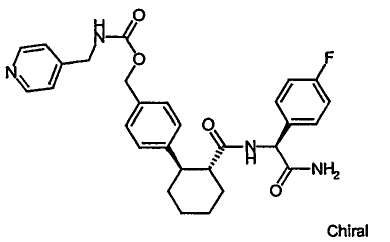 | 2,58 (C) |
| 3-14 | 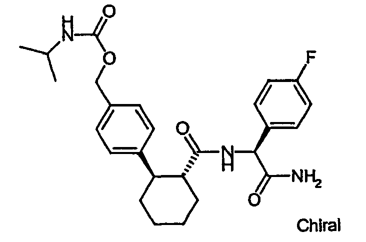 | 3,80 (C) |
| 3-15 | 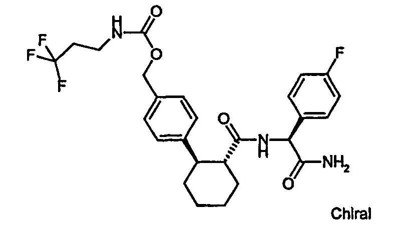 | 3,91 (C) |
| 3-16 | 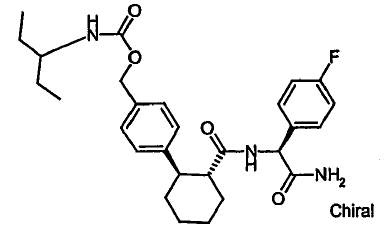 | 4,14 (C) |
| 3-17 | 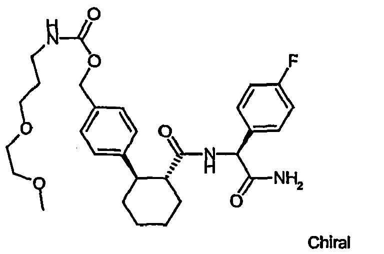 | 3,54 (C) |
| 3-18 | 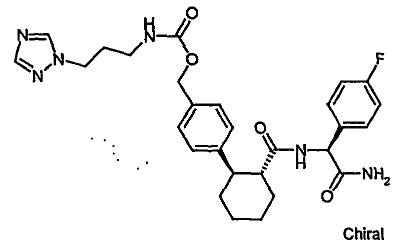 | 3,19 (C) |
| 3-19 | 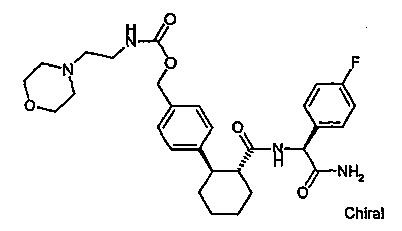 | 2,45 (C) |
| 3-20 | 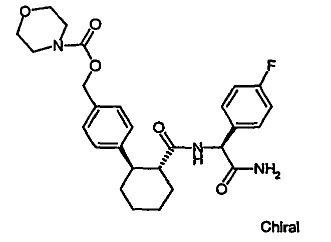 | 3,56 (C) |
| 3-21 | 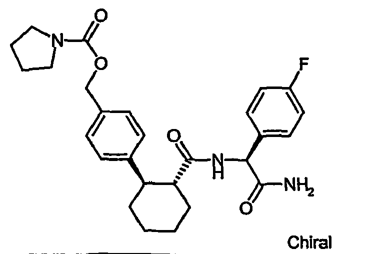 | 3,82 (C) |
| 3-22 | 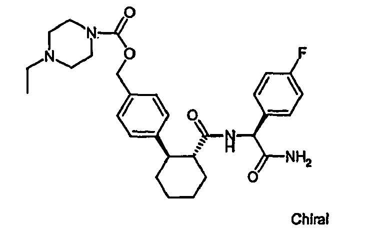 | 2,51 (C) |
| 3-23 | 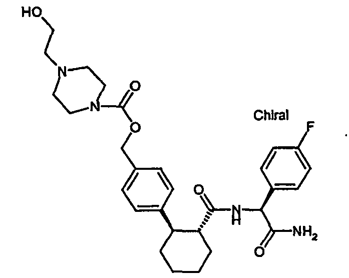 | 3,66 (A) |
| 3-24 | 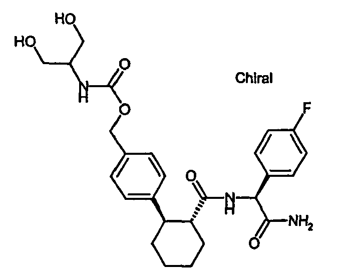 | 3,57 (A) |
| 3-25 | 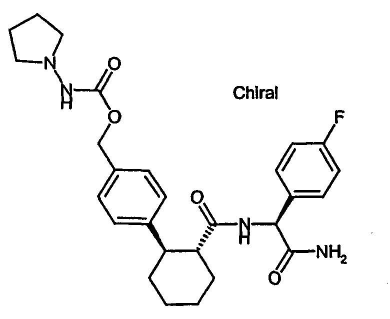 | 3,68 (A) |
| 3-26 | 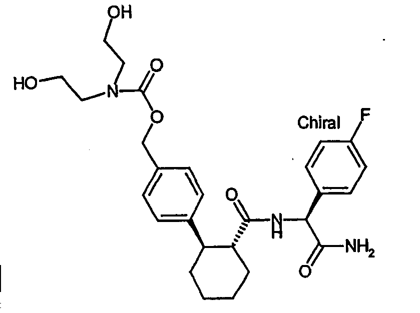 | 3,64 (A) |
| 3-27 | 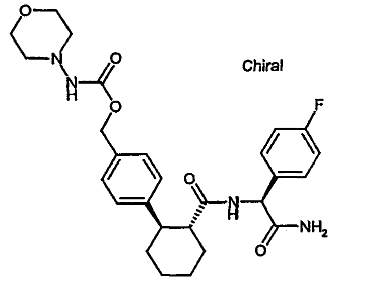 | 3,77 (A) |
| 3-28 | 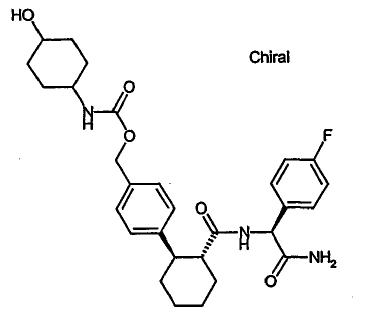 | 3,86 (A) |
| 3-29 | 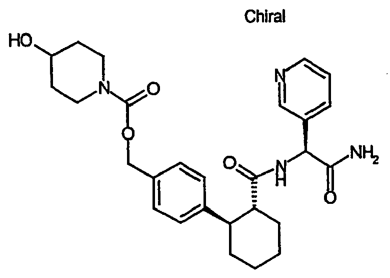 | 2,64 (C) |
| 3-30 | 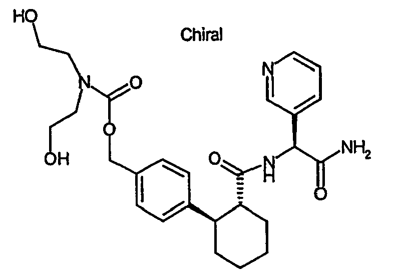 | 2,40 (C) |
| 3-31 | 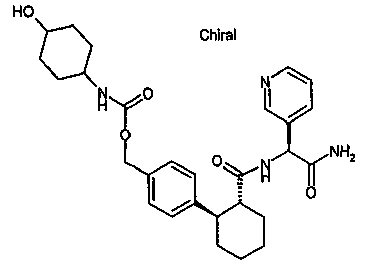 | 2,63 (C) |
| 3-32 | 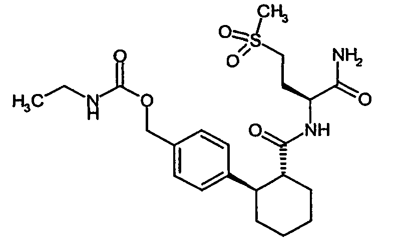 | 3,71 (E) |
| 3-33 | 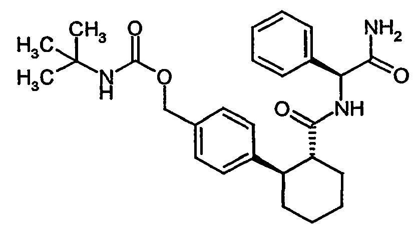 | 4,35 (E) |
| 3-34 | 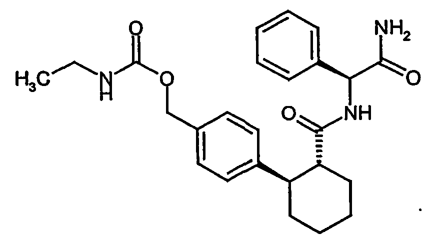 | 4,00 (E) |
| 3-35 | 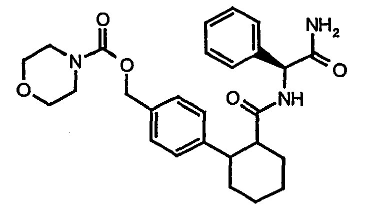 | R_{f} (CH₂Cl₂:MeOH = 20:1) 0,35 |

### Beispiel 4-1

### Benzyl-(4-[2-({[(1S)-2-amino-2-oxo-1-phenylethyl]amino}carbonyl)cyclohexyl]-benzyl)-carbamat:

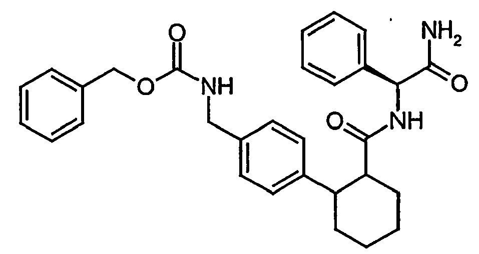

### a) 2-[4-({bis[(Benzyloxy)carbonyl]amino}methyl)phenyl]cyclohexancarbonsäure-tert.-butylester:

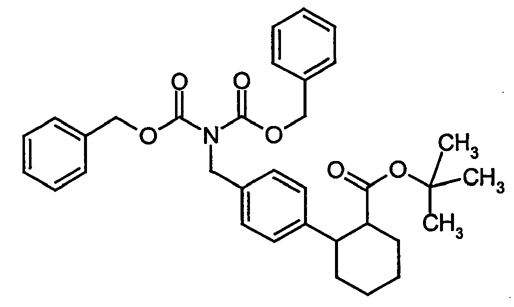

wird analog der allgemeinen Vorschrift A aus racemischem trans-2-(4-Brommethylphenyl)-cyclohexan-1-carbonsäure-tert.-butylester nach Beispiel I und Bis[(benzyloxy)carbonyl]amin (U. Ragnarsson et al., Synthesis, 1988, 992) in Gegenwart von NaH in DMF erhalten.

### b) 2-[4-({(Benzyloxy)carbonylamino}methyl)phenyl]cyclohexancarbonsäure:

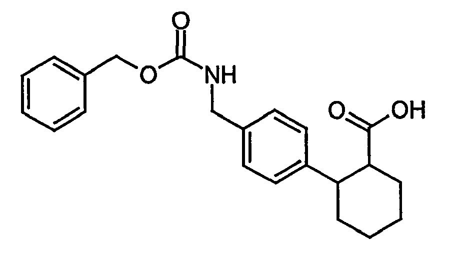

Der Ester aus Beispiel 4-1a (0,36 mmol) wird in Dichlormethan (5ml) gelöst, mit Trifluoressigsäure (5 ml) versetzt und 2 Stunden bei Raumtemperatur gerührt. Zur Aufarbeitung wird bei 0°C mit 2M Natronlauge neutralisiert, mit Dichlormethan extrahiert und die organische Phase wird über Magnesiumsulfat getrocknet und eingeengt. Der Rückstand wird chromatographiert (Kieselgel; Cyclohexan:Essigester: Essigsäure 3:1:0.1), 91,2 mg Säure werden erhalten.
R_{*f*} (Cyclohexan:Essigester:Essigsäure 3:1:0.2) = 0,21

### c) Benzyl-(4-[2-({[(1S)-2-amino-2-oxo-1-phenylethyl]amino}carbonyl)-cyclohexyl]benzyl)carbamat:

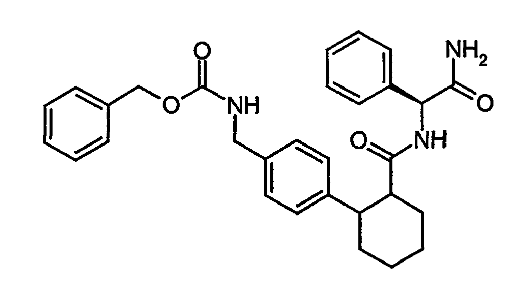

wird analog zur allgemeinen Vorschrift C aus der Säure nach Beispiel 4-1b und (*S*)-Phenylglycinamid-Hydrochlorid hergestellt. Es wird ein Gemisch der trans-Diastereomeren erhalten.
R_{*f*}(Methylenchlorid/Methanol 20:1) = 0,32.

### Beispiel 5-1

### N-{4-[2-({[(1S)-2-amino-2-oxo-1-phenylethyl]amino}carbonyl)cyclohexyl]-benzyl}-4-fluorbenzamid:

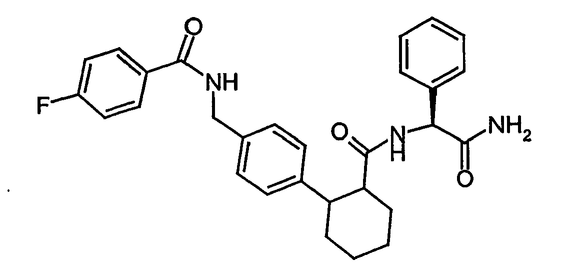

### a) 2-{4-[(1,3-Dioxo-1,3-dihydro-2H-isoindol-2-yl)methyl]phenyl}cyclohexancarbonsäure-tert.-butylester:

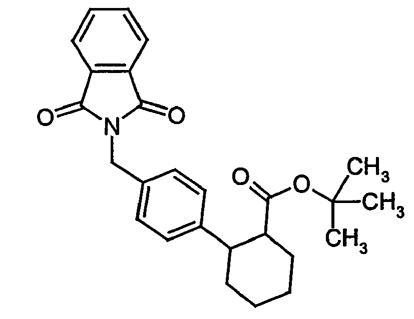

Racemischer trans-2-(4-Brommethyl-phenyl)-cyclohexan-1-carbonsäure-tert.-butylester nach Beispiel I (6,3 mmol) wird in DMF (30 ml) vorgelegt und mit Kaliumphthalimid (6 mmol) versetzt. Nach 5 min bei Raumtemperatur wird für 20 Stunden auf 50°C erhitzt. Nach Zugabe von Wasser, Extraktion mit Ether und Flashchromatographie über Kieselgel (Dichlormethan:Cyclohexan 1:1 → Dichlormethan) werden 1,66 g eines leicht gelblichen Feststoffes erhalten.
R_{*f*}(Methylenchlorid) = 0,2

### b)-c) Die Esterspaltung und die anschließende Amidbildung erfolgen analog der allgemeinen Vorschriften B und C

### d) (2S)-N-[2-(4-Aminomethyl-phenyl)-cyclohexyl-1-carbonyl]-phenylglycinamid:

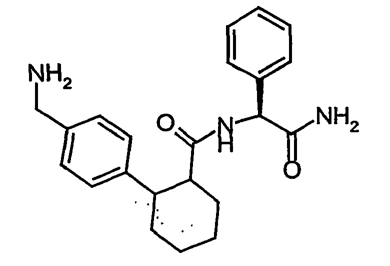

Zu einer Suspension des Phthalimids aus Beispiel 5-1c (0,5 mmol) in Ethanol (10 ml) wird Hydrazinhydrat (7,6 mmol) gegeben und 3 Tage bei Raumtemperatur gerührt. Nach Zugabe von 1M HCl bis pH=2 wird eingeengt, zwischen Dichlormethan und 10 %iger Natriumhydrogencarbonatlösung verteilt, die organische Phase über Natriumsulfat getrocknet und eingeengt. Chromatoraphie (Kieselgel, Dichloromethan: Methanol: Ammoniak 100:10:1) liefert 105 mg (51% Ausbeute) Diastereomerengemisch als gelblichen Feststoff.
R_{*f*}(Dichloromethan:Methanol:Ammoniak 100:10:1) = 0,13 bzw. 0,10
MS(DCI, NH₃)=510 (M+H⁺).
¹H-NMR(DMSO- d₆): A: 1.25-1.4 (4 H, m);); 1.7-1.85 (4 H, m); 2.55-2.8 (2 H, m); 3.3 (2 H, br s); 3.7 (2 H, s); 5.1 (1 H, d); 6.85 (1 H, s); 6.95 (1 H, s); 7.1-7.3 (9 H, m); 8.15 (1 H, d); B: 1.35-1.55 (4 H, m);); 1.65-1.9 (4 H, m); 2.2 (2 H, br s); 2.6-2.7 (1 H, m); 2.8 (1 H, td); 3.7 (2 H, s); 5.2 (1 H, d); 6.85 (2 H, d); 7.05-7.2 (8 H, m); 7.6 (1 H, s); 7.95 (1 H, d).

### e) N-{4-[2-({[(1S)-2-amino-2-oxo-1-phenylethyl]amino}carbonyl)-cyclohexyl]benzyl}-4-fluorbenzamid:

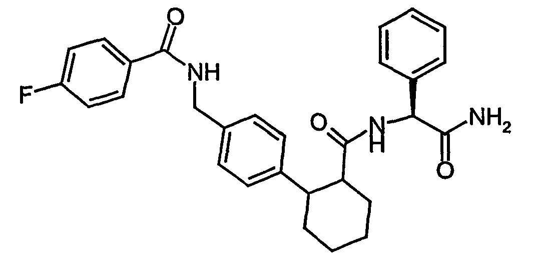

Das Amin aus Beispiel 5-1d (0,274 mmol) wird zusammen mit Triethylamin (0,82 mmol) in Dichlormethan (3 ml) gelöst und mit 4-Fluorbenzoesäureanhydrid (0,3 mmol) versetzt. Es wird bei Raumtemperatur gerührt, bis sich eine gelartige Konsistenz bildet (ca. 5 min). Dann wird Methanol zugegeben, bis alles gelöst ist, die Lösung wird dann auf Kieselgel aufgezogen und das Produkt mit Dichlormethan/Methanol 10:1 eluiert. Es werden 101 mg des gewünschten Produkts erhalten.
R_{*f*}(Dichloromethan:Methanol 10:1) = 0,24

Die in der folgenden Tabelle 4 aufgeführten Verbindungen werden in analoger Weise hergestellt:

**Tabelle 4**

| | | |
|---|---|---|
| **Beispiel** | **Struktur** | **R**_{**f**}**-Wert (CH**_{**2**}**Cl**_{**2**} **: MeOH : NH**_{**3**} **aq)** |
| 5-2 | 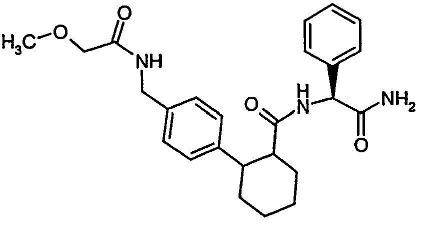 | 0,22 |
| | | (10:1:0) |
| 5-3 | 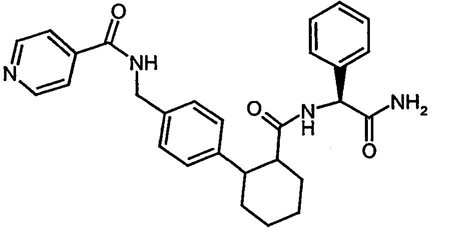 | 0,38 |
| | | (10:1:0) |
| 5-4 | 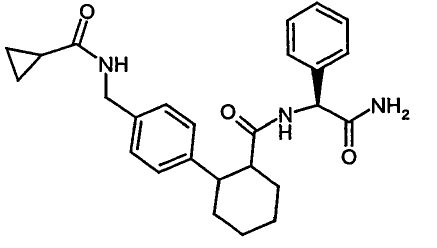 | 0,42/0,4 |
| | | (10:1:0) |

### Beispiel 6-1

### (1R,2R)-N-[(1R)-2-Amino-1-(4-fluorphenyl)-2-oxoethyl]-2-{4-[3-(cyclopropyl-amino)-3-oxopropyl]phenyl}cyclohexancarbonsäureamid:

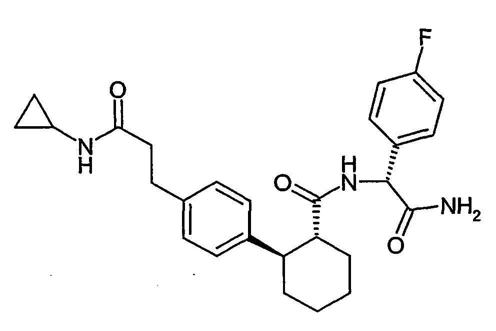

### a) 2-{4-[(1R,2R)-2-(tert.-Butoxycarbonyl)cyclohexyl]benzyl}malonsäure-dimethylester:

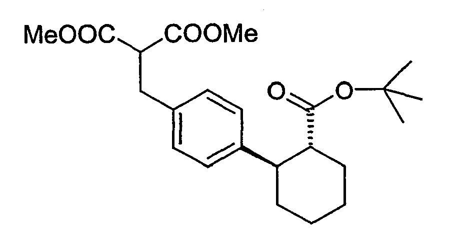

Zu einer Suspension aus NaH (60%ig in Mineralöl, 0,62 g, 15,57 mmol) in THF (50 ml) wird Malonsäuredimethylester (1,86 ml, 16,28 mmol) gegeben und 15 min bei Raumtemperatur gerührt. Die so erhaltene Lösung wird zu einer Lösung von (1*R*,2*R*)-2-(4-Brommethyl-phenyl)-cyclohexan-1-carbonsäure-tert.-butylester aus Beispiel I (5 g, 14,15 mmol) in THF (50 ml) gegeben und über Nacht bei Raumtemperatur gerührt. Die Mischung wird anschließend mit Wasser (200 ml) und Ethylacetat (500 ml) versetzt, ausgeschüttelt und die organische Phase mit gesättigter Ammoniumchlorid- und Kochsalz-Lösung gewaschen. Nach Trocknung der organischen Phase über Natriumsulfat, Filtration, Konzentration im Vakuum und Chromatographie an Kieselgel (Eluens: Cyclohexan/Ethylacetat = 6:1) werden 5 g (87 %) Produkt als farblose Flüssigkeit erhalten.
MS (DCI): 422.4 (M+NH₄)⁺
¹H-NMR (DMSO- d₆): 1.15 (9 H, s); 1.30-1.50 (4 H, m); 1.65-1.95 (4 H, m); 2.35-2.65 (2 H, m); 3.03 (2 H, d); 3.60 (6 H, s); 3.81 (1 H, t); 6.95-7.15 (4 H, m).

### b) (1R,2R)-2-{4-[3-Methoxy-2-(methoxycarbonyl)oxopropyl]phenyl}cyclohexancarbonsäure:

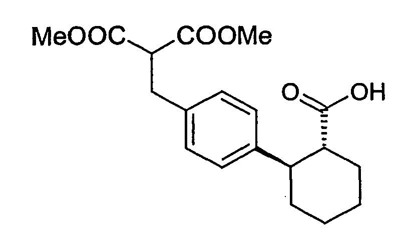

Zu einer Lösung der Verbindung aus Beispiel 6-1a (5 g, 12,36 mmol) in Dichlormethan (130,7 ml) wird unter Eiskühlung Trifluoressigsäure (57,2 ml) hinzugegeben und anschließend 5 Stunden bei Raumtemperatur gerührt. Nach Konzentration des Gemisches im Vakuum und Chromatographie an Kieselgel (Eluens: Dichlormethan/Methanol = 60:1 bis 20:1) werden 3,2 g (74 %) Produkt als farbloser Schaum erhalten.
MS (DCI): 366.1 (M+NH₄)⁺
¹H-NMR (DMSO- d₆): 1.30-1.50 (4 H, m); 1.65-1.70 (3 H, m); 1.90-2.00 (1 H, m); 2.45-2.55 (1 H, m); 2.60-2.70 (1 H, m); 3.03 (2 H, d); 3.60 (6 H, 2 s); 3.83 (1 H, t); 7.10 (4 H, q).

### c) 2-{4-[(1R,2R)-2-({[(1S)-2-Amino-1-(4-fluorphenyl)-2-oxoethyl]amino}-carbonyl)cyclohexyl]benzyl}malonsäuredimethylester:

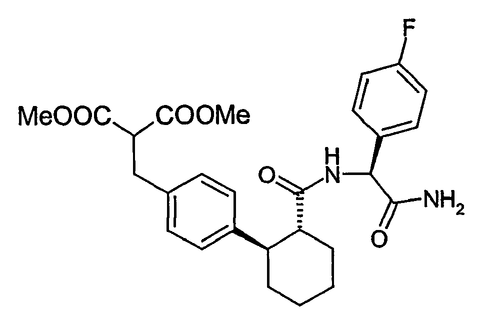

Zu einer Lösung der Verbindung aus Beispiel 6-1b (0.89 g, 2.54 mmol) in DMF (20 ml) werden 1-Hydroxybenzotriazol (0,38 g, 2,80 mmol) und EDC (0,56 g, 2,92 mmol) gegeben und 10 min bei Raumtemperatur gerührt. Anschließend werden N-Methylmorpholin (1,40 ml, 12,72 mmol), (+)-(*S*)-4-Fluorphenylglycinamid (0,52 g, 2,54 mmol) und eine Spatelspitze DMAP hinzugegeben und über Nacht bei Raumtemperatur gerührt. Nach Zugabe von Wasser (50 ml) wird 1 Stunde bei Raumtemperatur nachgerührt. Die Titelverbindung wird anschließend abfiltriert und mit Wasser und Diethylether nachgewaschen und getrocknet. Man erhält 1,17 g (92 %) eines farblosen Feststoffes.
MS (ESI+): 499.3 (M+H⁺)
¹H-NMR (DMSO- d₆): 1.20-1.90 (8 H, m); 2.55-2.90 (2 H, m); 3.03 (2 H, d); 3.60 (6 H, 2 s); 3.81 (1 H, t); 5.16 (1 H, d); 6.80-7.20 (9 H, m); 7.64 (1 H, br.s); 8.08 (1 H, d).

### d) 3-{4-[(1R,2R)-2-({[2-Amino-1-(4-fluorphenyl)-2-oxoethyl]amino}-carbonyl)cyclohexyl]phenyl}propancarbonsäure (Epimerengemisch bzw. R,R,R-Diastereomer)

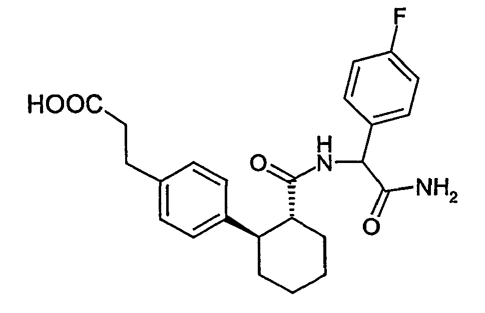

Zu einer Suspension der Verbindung aus Beispiel 6-1c (1,17 g, 2,35 mmol) in Methanol (10 ml) und Wasser (40 ml) wird Lithiumhydroxid (0,28 g, 11,73 mmol) gegeben und 1 Stunde bei 50°C gerührt. Anschließend wird das Methanol abdestilliert und der Ansatz mit 2 N Salzsäure auf pH 2 angesäuert. Der so erhaltene Rückstand wird mit Methylenchlorid/Methanol extrahiert und im Vakuum eingeengt. Der Rückstand wird anschließend in Dioxan (100 ml) aufgenommen und bei 120°C über Nacht unter Rückfluß erhitzt. Nach Einengen im Vakuum erhält man 508 mg (50,5 %) des Produktes (Epimerengemisch) als farbloses Öl. Durch Versetzten dieses Öls mit wenig Methylenchlorid/Methanol kristallisiert das reine (*R*,*R*,*R*)-Diastereomer (102 mg, 13 %) aus der Lösung als farbloser Feststoff aus.
MS (Epimerengemisch, ESI+): 427.3 (M+H)⁺
¹H-NMR [*R,R,R*-Diastereomer] (DMSO- d₆): 1.20-1.45 (4 H, m); 1.60-1.90 (4 H, m); 2.45-2.60 (von DMSO-Signal überlagert); 2.60-2.90 (4 H, m); 5.12 (1 H, d); 6.80-7.35 (10 H, m); 8.18 (1 H, d); 12.15 (1 H, br.s).

### e) (1R,2R)-N-[2-Amino-1-(4-fluorphenyl)-2-oxoethyl]-2-{4-[3-(cyclopropylamino)-3-oxopropyl]phenyl}cyclohexancarbonsäureamid (R,R,R-Diastereomer bzw. R,R,S-Diastereomer):

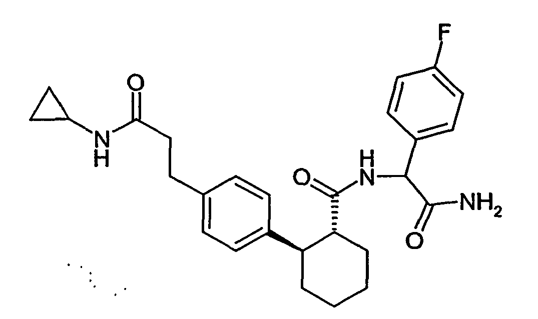

Zu einer Lösung der Verbindung aus Beispiel 6-1d (*R*,*R*,*R*-Diastereomer, 25,0 mg, 0,059 mmol) in DMF (1,5 ml) werden 1-Hydroxybenzotriazol (8,7 mg, 0,064 mmol) und EDC (12,9 mg, 0,067 mmol) gegeben und 10 min bei Raumtemperatur gerührt. Anschließend werden N-Methylmorpholin (0,016 ml, 0,147 mmol), Cyclopropylamin (8,4 mg, 0,147 mmol) und eine Spatelspitze DMAP hinzugegeben und über Nacht bei Raumtemperatur gerührt. Nach Einengen des Gemisches im Vakuum wird das Rohprodukt in Methylenchlorid aufgenommen, mit Wasser gewaschen und über Natriumsulfat getrocknet. Nach Konzentration des Rohproduktes im Vakuum und Chromatographie an Kieselgel (Eluens: Dichlormethan/Methanol = 20:1 bis 10:1) werden 25,0 mg (92 %) Produkt als farbloser Feststoff erhalten.
MS (ESI+): 466.3 (M+H)⁺
¹H-NMR (DMSO- d₆): 0.30-0.65 (4 H, m); 1.20-1.40 (4 H, m); 1.65-1.80 (4 H, m); 2.25-2.35 (2 H, m); 2.45-2.80 (5 H, m); 5.16 (1 H, d); 6.90-7.35 (10 H, m); 7.85 (1 H, d); 8.15 (1 H, d).

Die Herstellung des entsprechenden (*R*,*R*,*S*)-Diastereomers gelingt analog, wobei als Ausgangsmaterial die Verbindung aus Beispiel 6-1d (Epimerengemisch) eingesetzt wird. Man erhält hierbei das Produkt als Epimerengemisch, welches durch präparative HPLC-Chromatographie (Säule: Kromasil 100 C 18, 5 µm, 50 x 20 mm; Eluent: Acetonitril/Wasser; Fluß: 10 ml/min; UV-Detektion bei 254 nm) in die reinen Epimeren [(*R,R,S*)*-* und (*R*,*R*,*R*)-Diastereomere] aufgetrennt wird.

### Beispiel 6-2

### (1R,2R)-N-[2-Amino-1-(4-fluorphenyl)-2-oxoethyl]-2-(4-{3-[bis(2-methoxyethyl)-amino]-3-oxopropyl}phenyl)cyclohexancarbonsäureamid

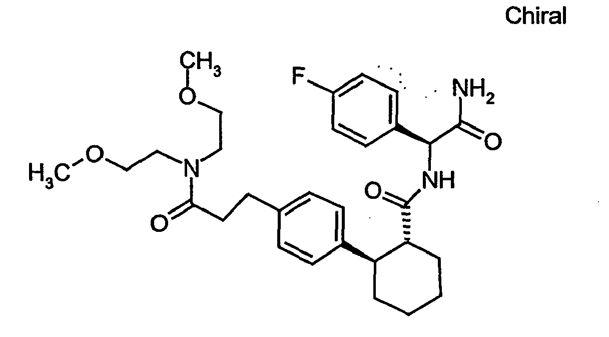

(1*R*,2*R*)-N-[2-Amino-1-(4-fluorphenyl)-2-oxoethyl]-2-(4-{3-[bis(2-methoxyethyl)-amino]-3-oxopropyl}phenyl)cyclohexancarbonsäureamid wird analog der unter Beispiel 6-1 beschriebenen Reaktionsfolge hergestellt. Rt-Zeit (Methode C) = 3,57

Die in den vorstehenden Beispielen und Tabellen aufgeführten HPLC-Retentionszeiten beziehen sich auf die folgenden HPLC-Methoden
- A:: Eluent A: = 1 % HClO₄ in Wasser, B = Acetonitril, Gradient: 0,5 min 98 % A, 4,5 min 10 % A, 6,5 min 10 % A, 6,7 min 98 % A, 7,5 min 98 % A, Kromasil 100 C18, 60x2 mm, 0,75 ml/min, 210 nm, 30°C
- B:: Eluent A: = 1 % HClO₄ in Wasser, B = Acetonitril, Gradient: 0,5 min 98 % A, 4,5 min 10 % A, 9,0 min 10 % A, 9,2 min 98 % A, 10,0 min 98 % A, Kromasil 100 C18, 60x2 mm, 0,75 ml/min, 210 nm, 30°C
- C:: Eluent A: = 0,1 % Ameisensäure in Wasser, B = 0,1 % Ameisensäure in Acetonitril, Gradient: 0 min 90 % A, 4 min 10 % A, 6,1 min 90 % A, Symmetry C18,50x2,1 mm, 0,5 ml/min, 210 nm, 30°C
- D:: Eluent A: = 0,01 M Phosphorsäure in Wasser, B = Acetonitril, Gradient: 1 min 90 % A, 9 min 10 % A, 13 min 10 % A, 13.5 min 90 % A, 15 min, 90 % A, Kromasil 100 C18, 125x2 mm, 210 nm, 30°C
- E:: Eluent A: = 0,5 % HClO₄ in Wasser, B = Acetonitril, Gradient: 0,5 min 98 % A, 4,5 min 10 % A, 6,5 min 10 % A, 6,7 min 98 % A, 7,5 min 98 % A, Kromasil 100 C18, 60x2 mm, 0,75 ml/min, 210 nm, 30°C

## Patentansprüche

1. Verbindungen der Formel (I) 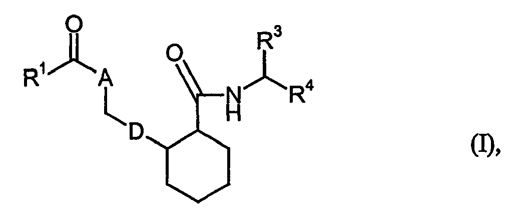 worin
D einen Rest 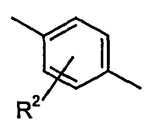 oder 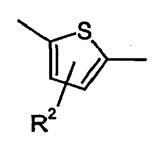 bedeutet,
worin
R² Wasserstoff, Halogen, Hydroxy, Carboxyl, Cyano, Nitro, Trifluormethyl, Trifluormethoxy, (C₁-C₆)-Alkyl, (C₁-C₆)-Alkoxy oder (C₁-C₆)-Alkoxycarbonyl bedeutet,
A ein Sauerstoffatom oder eine Gruppe der Formel N-R⁵ oder CH-R⁶ bedeutet,
worin
R⁵ Wasserstoff, (C₁-C₆)-Alkyl, (C₃-C₇)-Cycloalkyl, wobei Alkyl und Cycloalkyl ihrerseits bis zu dreifach unabhängig voneinander durch Hydroxy oder Mono- oder Di-(C₁-C₆)-alkylamino substituiert sein können, (C₆-C₁₀)-Aryl, 5- bis 10-gliedriges Heteroaryl mit bis zu drei Heteroatomen aus der Reihe N, O und/oder S oder 5- bis 6-gliedriges Heterocyclyl mit bis zu drei Heteroatomen aus der Reihe N, O und/oder S bedeutet, wobei Aryl, Heteroaryl und Heterocyclyl ihrerseits bis zu dreifach unabhängig voneinander durch Halogen, Hydroxy, Cyano, Nitro, Trifluormethyl, Trifluormethoxy, (C₁-C₆)-Alkyl, (C₁-C₆)-Alkoxy, (C₁-C₆)-Alkoxycarbonyl oder Mono- oder Di-(C₁-C₆)-alkylamino substituiert sein können,
R⁶ Wasserstoff, (C₁-C₆)-Alkoxycarbonyl oder Carboxyl bedeutet,
R¹ Wasserstoff, (C₁-C₆)-Alkyl, das seinerseits durch Hydroxy oder (C₁-C₄)-Alkoxy substituiert sein kann, (C₃-C₇)-Cycloalkyl, (C₆-C₁₀)-Aryl, 5- bis 10-gliedriges Heteroaryl mit bis zu zwei Heteroatomen aus der Reihe N, O und/oder S, worin Aryl und Heteroaryl ihrerseits unabhängig voneinander durch Halogen substituiert sein können, oder einen Rest der Formel -NR⁷R⁸ oder -OR⁹ bedeutet,
worin
R⁷ und R⁸ unabhängig voneinander Wasserstoff, (C₆-C₁₀)-Aryl, Adamantyl, (C₁-C₈)-Alkyl, dessen Kette durch ein oder zwei Sauerstoffatome unterbrochen sein kann und das bis zu dreifach unabhängig voneinander durch Hydroxy, Phenyl, Trifluormethyl, (C₃-C₈)-Cycloalkyl, (C₁-C₆)-Alkoxy, Mono-oder Di-(C₁-C₆)-alkylamino, 5- oder 6-gliedriges Heterocyclyl mit bis zu drei Heteroatomen aus der Reihe N, O und/oder S oder durch 5- bis 10-gliedriges Heteroaryl mit bis zu drei Heteroatomen aus der Reihe N, O und/oder S substituiert sein kann, (C₃-C₈)-Cycloalkyl, das bis zu dreifach unabhängig voneinander durch (C₁-C₄)-Alkyl, Hydroxy oder Oxo substituiert sein kann, oder 5- oder 6-gliedriges Heterocyclyl mit bis zu zwei Heteroatomen aus der Reihe N, O und/oder S, wobei N durch Wasserstoff oder (C₁-C₄)-Alkyl substituiert ist, bedeuten,
oder
R⁷ und R⁸ gemeinsam mit dem Stickstoffatom, an das sie gebunden sind, einen 4- bis 7-gliedrigen gesättigten Heterocyclus bilden, der bis zu zwei weitere Heteroatome aus der Reihe N, O und/oder S enthalten kann und gegebenenfalls substituiert ist durch Hydroxy, Oxo oder (C₁-C₆)-Alkyl, welches seinerseits durch Hydroxy substituiert sein kann,
und
R⁹ (C₆-C₁₀)-Aryl, Adamantyl, (C₁-C₈)-Alkyl, dessen Kette durch ein oder zwei Sauerstoffatome unterbrochen sein kann und das bis zu dreifach unabhängig voneinander durch Hydroxy, Phenyl, Trifluormethyl, (C₃-C₈)-Cycloalkyl, (C₁-C₆)-Alkoxy, Mono- oder Di-(C₁-C₆)-alkylamino, 5- oder 6-gliedriges Heterocyclyl mit bis zu drei Heteroatomen aus der Reihe N, O und/oder S oder durch 5- bis 10-gliedriges Heteroaryl mit bis zu drei Heteroatomen aus der Reihe N, O und/oder S substituiert sein kann, (C₃-C₈)-Cycloalkyl, das bis zu dreifach unabhängig voneinander durch (C₁-C₄)-Alkyl, Hydroxy oder Oxo substituiert sein kann, oder 5- oder 6-gliedriges Heterocyclyl mit bis zu zwei Heteroatomen aus der Reihe N, O und/oder S, wobei N durch Wasserstoff oder (C₁-C₄)-Alkyl substituiert ist, bedeuten,
R³ (C₁-C₈)-Alkyl, dessen Kette durch ein Schwefel- oder Sauerstoffatom oder eine S(O)- oder SO₂-Gruppe unterbrochen sein kann, Phenyl, Benzyl oder 5- oder 6-gliedriges Heteroaryl mit bis zu zwei Heteroatomen aus der Reihe N, O und/oder S bedeutet, worin Phenyl, Benzyl und Heteroaryl bis zu dreifach unabhängig voneinander durch Halogen, Trifluormethyl, Cyano, Nitro, Hydroxy, (C₁-C₆)-Alkyl oder (C₁-C₆)-Alkoxy substituiert sein können,
und
R⁴ einen Rest der Formel-C(O)-NR¹⁰R¹¹ bedeutet,
worin
R¹⁰ und R¹¹ unabhängig voneinander Wasserstoff oder (C₁-C₆)-Alkyl bedeuten,
und ihre Salze, Hydrate, Hydrate der Salze und Solvate.

2. Verbindungen nach Anspruch 1,
worin
D einen Rest 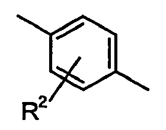 bedeutet,
worin
R² Wasserstoff, Halogen, Hydroxy, Carboxyl, Cyano, Nitro, Trifluormethyl, Trifluormethoxy, (C₁-C₆)-Alkyl, (C₁-C₆)-Alkoxy oder (C₁-C₆)-Alkoxycarbonyl bedeutet;
A ein Sauerstoffatom oder eine Gruppe der Formel N-R⁵ oder CH-R⁶ bedeutet,
worin
R⁵ Wasserstoff, (C₁-C₆)-Alkyl, (C₃-C₇)-Cycloalkyl, wobei Alkyl und Cycloalkyl ihrerseits bis zu dreifach unabhängig voneinander durch Hydroxy oder Mono- oder Di-(C₁-C₆)-alkylamino substituiert sein können, (C₆-C₁₀)-Aryl, 5- bis 10-gliedriges Heteroaryl mit bis zu drei Heteroatomen aus der Reihe N, O und/oder S oder 5- bis 6-gliedriges Heterocyclyl mit bis zu drei Heteroatomen aus der Reihe N, O und/oder S bedeutet, wobei Aryl, Heteroaryl und Heterocyclyl ihrerseits bis zu dreifach unabhängig voneinander durch Halogen, Hydroxy, Cyano, Nitro, Trifluormethyl, Trifluormethoxy, (C₁-C₆)-Alkyl, (C₁-C₆)-Alkoxy, (C₁-C₆)-Alkoxycarbonyl oder Mono- oder Di-(C₁-C₆)-alkylamino substituiert sein können,
R⁶ Wasserstoff, (C₁-C₆)-Alkoxycarbonyl oder Carboxyl bedeutet,
R¹ Wasserstoff, (C₁-C₆)-Alkyl, das seinerseits durch Hydroxy oder (C₁-C₄)-Alkoxy substituiert sein kann, (C₃-C₇)-Cycloalkyl, (C₆-C₁₀)-Aryl, 5- bis 10-gliedriges Heteroaryl mit bis zu zwei Heteroatomen aus der Reihe N, O und/oder S, worin Aryl und Heteroaryl ihrerseits unabhängig voneinander durch Halogen substituiert sein können, oder einen Rest der Formel -NR⁷R⁸ oder -OR⁹ bedeutet,
worin
R⁷ und R⁸ unabhängig voneinander Wasserstoff, (C₆-C₁₀)-Aryl, Adamantyl, (C₁-C₈)-Alkyl, dessen Kette durch ein oder zwei Sauerstoffatome unterbrochen sein kann und das bis zu dreifach unabhängig voneinander durch Hydroxy, Phenyl, Trifluormethyl, (C₃-C₈)-Cycloalkyl, (C₁-C₆)-Alkoxy, Mono-oder Di-(C₁-C₆)-alkylamino, 5- oder 6-gliedriges Heterocyclyl mit bis zu drei Heteroatomen aus der Reihe N, O und/oder S oder durch 5- bis 10-gliedriges Heteroaryl mit bis zu drei Heteroatomen aus der Reihe N, O und/oder S substituiert sein kann, (C₃-C₈)-Cycloalkyl, das bis zu dreifach unabhängig voneinander durch (C₁-C₄)-Alkyl, Hydroxy oder Oxo substituiert sein kann, oder 5- oder 6-gliedriges Heterocyclyl mit bis zu zwei Heteroatomen aus der Reihe N, O und/oder S, wobei N durch Wasserstoff oder (C₁-C₄)-Alkyl substituiert ist, bedeuten,
oder
R⁷ und R⁸ gemeinsam mit dem Stickstoffatom, an das sie gebunden sind, einen 4- bis 7-gliedrigen gesättigten Heterocyclus bilden, der bis zu zwei weitere Heteroatome aus der Reihe N, O und/oder S enthalten kann und gegebenenfalls substituiert ist durch Hydroxy, Oxo oder (C₁-C₆)-Alkyl, welches seinerseits durch Hydroxy substituiert sein kann,
und R⁹ (C₆-C₁₀)-Aryl, Adamantyl, (C₁-C₈)-Alkyl, dessen Kette durch ein oder zwei Sauerstoffatome unterbrochen sein kann und das bis zu dreifach unabhängig voneinander durch Hydroxy, Phenyl, Trifluormethyl, (C₃-C₈)-Cycloalkyl, (C₁-C₆)-Alkoxy, Mono- oder Di-(C₁-C₆)-alkylamino, 5- oder 6-gliedriges Heterocyclyl mit bis zu drei Heteroatomen aus der Reihe N, O und/oder S oder durch 5- bis 10-gliedriges Heteroaryl mit bis zu drei Heteroatomen aus der Reihe N, O und/oder S substituiert sein kann, (C₃-C₈)-Cycloalkyl, das bis zu dreifach unabhängig voneinander durch (C₁-C₄)-Alkyl, Hydroxy oder Oxo substituiert sein kann, oder 5- oder 6-gliedriges Heterocyclyl mit bis zu zwei Heteroatomen aus der Reihe N, O und/oder S, wobei N durch Wasserstoff oder (C₁-C₄)-Alkyl substituiert ist, bedeuten,
R³ (C₁-C₈)-Alkyl, dessen Kette durch ein Schwefelatom oder eine S(O)-oder SO₂-Gruppe unterbrochen sein kann, Phenyl, Benzyl oder 5- oder 6-gliedriges Heteroaryl mit bis zu zwei Heteroatomen aus der Reihe N, O und/oder S bedeutet, worin Phenyl, Benzyl und Heteroaryl bis zu dreifach unabhängig voneinander durch Halogen, Trifluormethyl, Cyano, Nitro, Hydroxy, (C₁-C₆)-Alkyl oder (C₁-C₆)-Alkoxy substituiert sein können,
und
R⁴ einen Rest der Formel -C(O)-NR¹⁰R¹¹ bedeutet,
worin
R¹⁰ und R¹¹ unabhängig voneinander Wasserstoff oder (C₁-C₆)-Alkyl bedeuten,
und ihre Salze, Hydrate, Hydrate der Salze und Solvate.

3. Verbindungen nach Anspruch 1,
worin
D einen Rest 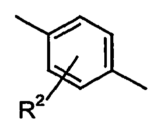 bedeutet,
worin
R² Wasserstoff, Chlor oder Fluor bedeutet,
A ein Sauerstoffatom oder eine Gruppe der Formel N-R⁵ bedeutet,
worin
R⁵ Wasserstoff, (C₁-C₆)-Alkyl, das seinerseits bis zu zweifach durch Hydroxy substituiert sein kann, (C₃-C₇)-Cycloalkyl, Phenyl oder 5- oder 6-gliedriges Heteroaryl mit bis zu drei Heteroatomen aus der Reihe N, O und/oder S bedeutet, wobei Phenyl und Heteroaryl ihrerseits bis zu zweifach unabhängig voneinander durch Halogen, Cyano, Trifluormethyl, Trifluormethoxy, (C₁-C₄)-Alkyl, (C₁-C₄)-Alkoxy oder Di-(C₁-C₄)-alkylamino substituiert sein können,
R¹ Wasserstoff, (C₁-C₆)-Alkyl, das seinerseits durch Hydroxy oder (C₁-C₄)-Alkoxy substituiert sein kann, (C₃-C₇)-Cycloalkyl, Phenyl, 5- bis 6-gliedriges Heteroaryl mit bis zu zwei Heteroatomen aus der Reihe N, O und/oder S, worin Phenyl und Heteroaryl ihrerseits unabhängig voneinander durch Halogen substituiert sein können, oder einen Rest der Formel -NR⁷R⁸ oder -OR⁹ bedeutet,
worin
R⁷ und R⁸ unabhängig voneinander Wasserstoff, Phenyl, Adamantyl, (C₁-C₆)-Alkyl, dessen Kette durch ein oder zwei Sauerstoffatome unterbrochen sein kann und das bis zu zweifach unabhängig voneinander durch Hydroxy, Phenyl, Trifluormethyl, (C₃-C₆)-Cycloalkyl, (C₁-C₄)-Alkoxy, Mono- oder Di-(C₁-C₄)-alkylamino, 5- bis 6-gliedriges Heterocyclyl mit bis zu zwei Heteroatomen aus der Reihe N und/oder O oder durch 5- bis 6-gliedriges Heteroaryl mit bis zu drei Heteroatomen aus der Reihe N, O und/oder S substituiert sein kann, (C₃-C₈)-Cycloalkyl, das bis zu zweifach durch Hydroxy substituiert sein kann, oder 5- bis 6-gliedriges Heterocyclyl mit bis zu zwei Heteroatomen aus der Reihe N, O und/oder S, wobei N durch Wasserstoff oder (C₁-C₄)-Alkyl substituiert ist, bedeuten,
oder
R⁷ und R⁸ gemeinsam mit dem Stickstoffatom, an das sie gebunden sind, einen 4- bis 7-gliedrigen gesättigten Heterocyclus bilden, der bis zu zwei weitere Heteroatome aus der Reihe N, O und/oder S enthalten kann und gegebenenfalls substituiert ist durch Hydroxy, Oxo oder (C₁-C₆)-Alkyl, welches seinerseits durch Hydroxy substituiert sein kann,
und
R⁹ Phenyl, Adamantyl, (C₁-C₆)-Alkyl, dessen Kette durch ein oder zwei Sauerstoffatome unterbrochen sein kann und das bis zu zweifach unabhängig voneinander durch Hydroxy, Phenyl, Trifluormethyl, (C₃-C₆)-Cycloalkyl, (C₁-C₃)-Alkoxy, Mono-oder Di-(C₁-C₄)-alkylamino, 5- bis 6-gliedriges Heterocyclyl mit bis zu zwei Heteroatomen aus der Reihe N und/oder O oder durch 5- bis 6-gliedriges Heteroaryl mit bis zu drei Heteroatomen aus der Reihe N, O und/oder S substituiert sein kann, (C₃-C₈)-Cycloalkyl, das bis zu zweifach durch Hydroxy substituiert sein kann, oder 5- bis 6-gliedriges Heterocyclyl mit bis zu zwei Heteroatomen aus der Reihe N, O und/oder S, wobei N durch Wasserstoff oder (C₁-C₄)-Alkyl substituiert ist, bedeuten,
R³ (C₁-C₈)-Alkyl, dessen Kette durch ein Schwefelatom oder eine S(O)-oder SO₂-Gruppe unterbrochen sein kann, Phenyl, Benzyl oder 5- bis 6-gliedriges Heteroaryl mit bis zu zwei Heteroatomen aus der Reihe N, O und/oder S bedeutet, worin Phenyl, Benzyl und Heteroaryl bis zu zweifach unabhängig voneinander durch Halogen, Trifluormethyl, Cyano, (C₁-C₃)-Alkyl, (C₁-C₃)-Alkoxy oder Hydroxy substituiert sein können,
und
R⁴ einen Rest der Formel -C(O)-NR¹⁰R¹¹ bedeutet,
worin
R¹⁰ und R¹¹ unabhängig voneinander Wasserstoff oder (C₁-C₆)-Alkyl bedeuten,
und ihre Salze, Hydrate, Hydrate der Salze und Solvate.

4. Verbindungen nach Anspruch 1,
worin
D einen Rest 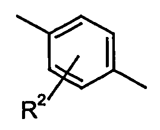 bedeutet,
worin
R² Wasserstoff bedeutet,
A ein Sauerstoffatom oder eine Gruppe der Formel N-R⁵ bedeutet,
worin
R⁵ Wasserstoff, (C₁-C₆)-Alkyl, das seinerseits bis zu zweifach durch Hydroxy substituiert sein kann, (C₃-C₇)-Cycloalkyl, Phenyl oder 5- bis 6-gliedriges Heteroaryl mit bis zu drei Heteroatomen aus der Reihe N, O und/oder S bedeutet, wobei Phenyl und Heteroaryl ihrerseits bis zu zweifach unabhängig voneinander durch Fluor, Chlor, Cyano, Trifluormethyl, Trifluormethoxy, (C₁-C₃)-Alkyl, (C₁-C₃)-Alkoxy oder Di-(C₁-C₃)-alkylamino substituiert sein können,
R¹ (C₁-C₄)-Alkyl oder einen Rest der Formel -NR⁷R⁸ bedeutet,
worin
R⁷ und R⁸ unabhängig voneinander Wasserstoff, Phenyl, Adamantyl, (C₁-C₄)-Alkyl, dessen Kette durch ein oder zwei Sauerstoffatome unterbrochen sein kann und das bis zu zweifach unabhängig voneinander durch Hydroxy, Phenyl, Trifluormethyl, (C₃-C₆)-Cycloalkyl, (C₁-C₃)-Alkoxy, Mono- oder Di-(C₁-C₃)-alkylamino, 5- bis 6-gliedriges Heterocyclyl mit bis zu zwei Heteroatomen aus der Reihe N und/oder O oder durch 5-bis 6-gliedriges Heteroaryl mit bis zu drei Heteroatomen aus der Reihe N, O und/oder S substituiert sein kann, (C₃-C₈)-Cycloalkyl, das bis zu zweifach durch Hydroxy substituiert sein kann, oder 5- bis 6-gliedriges Heterocyclyl mit bis zu zwei Heteroatomen aus der Reihe N, O und/oder S, wobei N durch Wasserstoff oder (C₁-C₄)-Alkyl substituiert ist, bedeuten,
oder
R⁷ und R⁸ gemeinsam mit dem Stickstoffatom, an das sie gebunden sind, einen 4- bis 7-gliedrigen gesättigten Heterocyclus bilden, der bis zu zwei weitere Heteroatome aus der Reihe N, O und/oder S enthalten kann und gegebenenfalls substituiert ist durch Hydroxy, Oxo oder (C₁-C₆)-Alkyl, welches seinerseits durch Hydroxy substituiert sein kann,
R³ (C₁-C₈)-Alkyl, dessen Kette durch ein Schwefelatom oder eine S(O)-oder SO₂-Grupp unterbrochen sein kann, Phenyl, Benzyl oder 5- bis 6-gliedriges Heteroaryl mit bis zu zwei Heteroatomen aus der Reihe N, O und/oder S bedeutet, worin Phenyl, Benzyl und Heteroaryl bis zu zweifach unabhängig voneinander durch Halogen, Trifluormethyl, Cyano, (C₁-C₃)-Alkyl, (C₁-C₃)-Alkoxy oder Hydroxy substituiert sein können,
und
R⁴ einen Rest der Formel -C(O)-NR¹⁰R¹¹ bedeutet,
worin
R¹⁰ und R¹¹ unabhängig voneinander Wasserstoff, Methyl oder Ethyl bedeuten,
und ihre Salze, Hydrate, Hydrate der Salze und Solvate.

5. Verbindungen nach Anspruch 1,
worin
D einen Rest 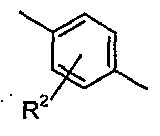 bedeutet,
worin
R² Wasserstoff bedeutet,
A ein Sauerstoffatom oder eine Gruppe der Formel N-R⁵ bedeutet,
worin
R⁵ (C₃-C₇)-Cycloalkyl, Phenyl, das seinerseits durch Fluor substituiert sein kann, oder Pyridyl bedeutet,
R¹ Methyl oder einen Rest der Formel -NR⁷R⁸ bedeutet,
worin
R⁷ und R⁸ unabhängig voneinander (C₁-C₄)- Alkyl, das ein- oder zweifach durch Hydroxy substituiert sein kann, bedeuten,
oder
R⁷ und R⁸ gemeinsam mit dem Stickstoffatom, an das sie gebunden sind, einen 5- bis 6-gliedrigen gesättigten Heterocyclus bilden, der ein weiteres Heteroatom O oder N enthalten kann, wobei N durch Wasserstoff oder (C₁-C₃)-Alkyl, das seinerseits durch Hydroxy substituiert sein kann, substituiert ist,
R³ Phenyl, das gegebenenfalls in para-Position durch Fluor substituiert sein kann, oder Pyridyl bedeutet,
und
R⁴ einen Rest der Formel -C(O)-NR¹⁰R¹¹ bedeutet,
worin
R¹⁰ und R¹¹ Wasserstoff bedeuten,
und ihre Salze, Hydrate, Hydrate der Salze und Solvate.

6. Verbindungen nach Anspruch 1, **gekennzeichnet durch** eine der folgenden stereochemischen Konfigurationen gemäß Formeln (Ia) bis (Id):

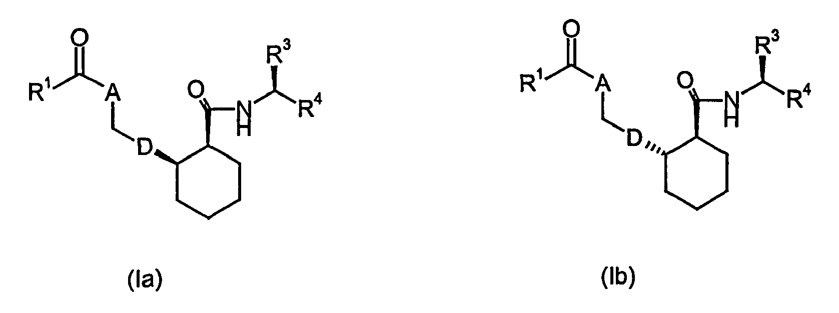

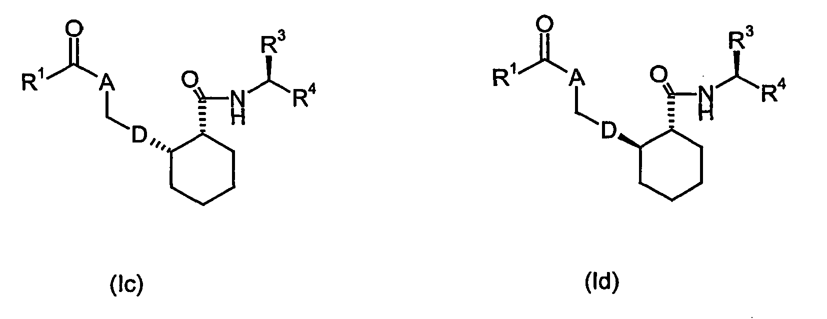

7. Verbindungen nach Anspruch 1, **gekennzeichnet durch** die folgende stereochemische Konfiguration gemäß Formel (Id):

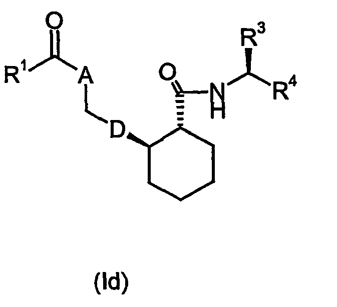

8. Verbindungen nach Anspruch 1 mit den folgenden Strukturen:
(1*R*,2*R*)-*N*-[(1*S*)-2-Amino-1-(4-fluorphenyl)-2-oxoethyl]-2-(4-{[{[ethyl(2-hydroxyethyl)amino]carbonyl}(4-fluorphenyl)amino]methyl}phenyl)cyclohexancarbonsäureamid 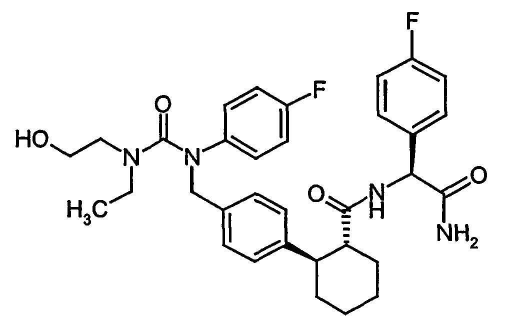
(1*R*,2*R*)-*N*-[(1*S*)-2-Amino-2-oxo-1-phenylethyl]-2-(4-{[[(dimethylamino)-carbonyl](phenyl)amino]methyl} phenyl)cyclohexancarbonsäureamid 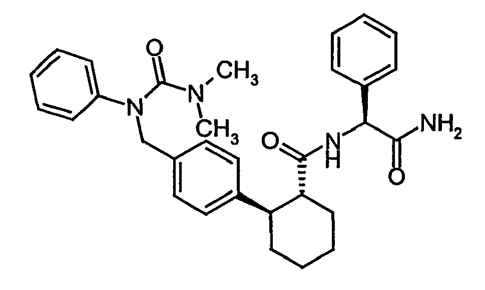
(1*R*,2*R*)-*N*-[(1*S*)-2-Amino-2-oxo-1-phenylethyl]-2-[4-({cyclopropyl[(dimethylamino)carbonyl]amino}methyl)phenyl]cyclohexancarbonsäureamid 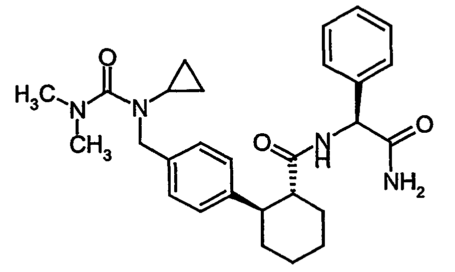
(1*R*,2*R*)-*N*-[(1*S*)-2-Amino-2-oxo-1-phenylethyl]-2-(4-{[[(diethylamino)carbonyl](2-pyridinyl)amino]methyl} phenyl)cyclohexancarbonsäureamid 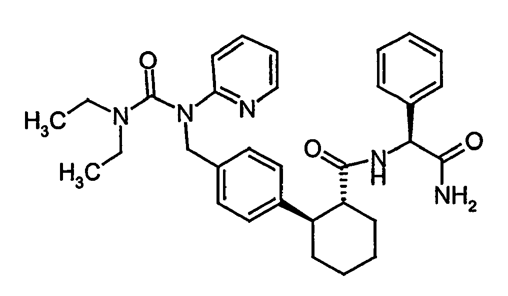
*N*-{4-[(1*R*,2*R*)-2-({[(1*S*)-2-Amino-2-oxo-1-phenylethyl]amino}carbonyl)-cyclohexyl]benzyl}-*N*-phenyl-4-morpholincarbonsäureamid 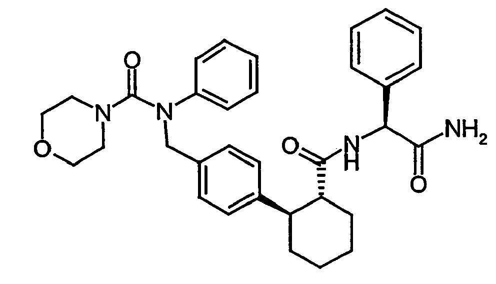
(*S*)-N-{{(1*R*,2*R*)-2-(4-{[{[2-Hydroxyethylamino]carbonyl}(phenyl)amino]-methyl}phenyl)cyclohex-1-yl}carbonyl}-phenylglycinamid 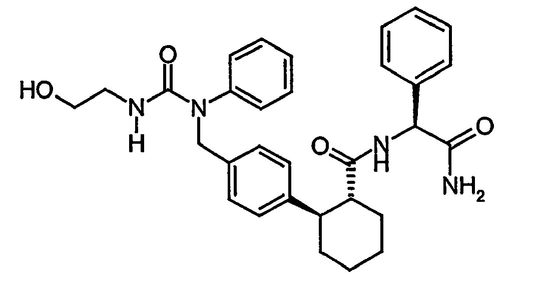
(1*R*,2*R*)-2-(4-{[Acetyl(2-pyridinyl)amino]methyl}phenyl)-N-[(1*S*)-2-amino-2-oxo-1-phenylethyl]cyclohexancarboxamid 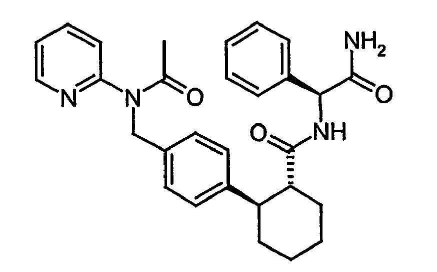
(1*R*,2*R*)-*N*-[(1*S*)-2-Amino-1-phenyl-2-oxoethyl]-2-(4-{[{[ethyl(2-hydroxyethyl)amino]carbonyl}(phenyl)amino]methyl}phenyl)cyclohexancarbonsäureamid 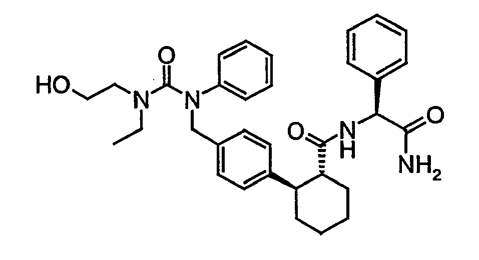
4-[(1*R*,2*R*)-2-({[(1*S*)-2-Amino-1-(4-fluorphenyl)-2-oxoethyl]amino}-carbonyl)cyclohexyl]benzyl 4-(2-hydroxyethyl)-1-piperazincarbamat 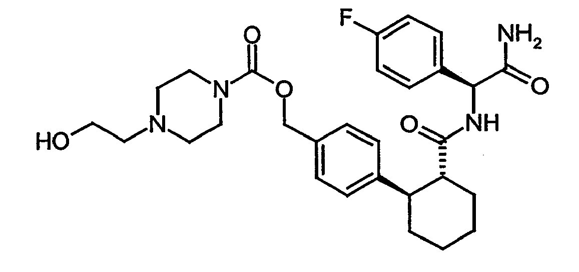
4-[(1*R*,2*R*)-2-({[(1*S*)-2-Amino-1-phenyl-2-oxoethyl]amino}carbonyl)-cyclohexyl]benzyl-4-(2-hydroxyethyl)-1-piperazincarbamat 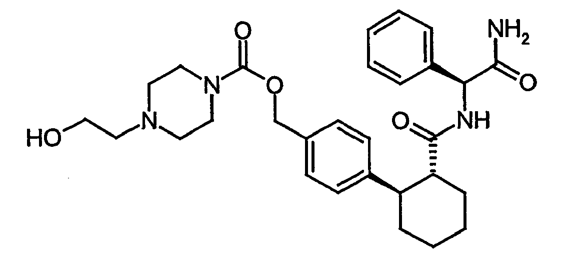
und ihre Salze, Hydrate, Hydrate der Salze und Solvate.

9. Verfahren zur Herstellung von Verbindungen der Formel (I), **dadurch gekennzeichnet, dass** man
[A] Verbindungen der Formel (II) 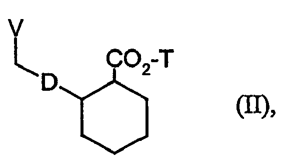 in welcher
D die in Anspruch 1 angegebene Bedeutung hat,
T für (C₁-C₄)-Alkyl steht,
und
V für eine geeignete Abgangsgruppe steht,
zunächst durch Umsetzung mit Verbindungen der Formel (III)
B-H (III),
in welcher
B für 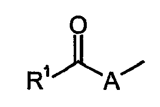 oder
gegebenenfalls für den Fall, dass R¹ für OR⁹ steht,
für 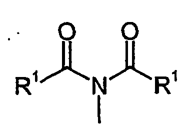 steht,
und
R¹ und A die in Anspruch 1 angegebenen Bedeutungen haben,
in die Verbindungen der Formel (IV) 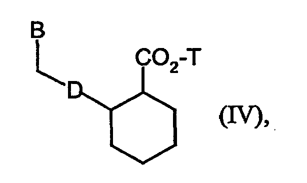 in welcher B und T die oben angegebene und D die in Anspruch 1 angegebene Bedeutung haben,
überführt,
in einem nächsten Schritt mit Säuren oder Basen in die entsprechenden Carbonsäuren der Formel (V) 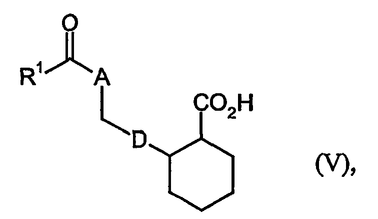 in welcher
R¹, A und D die in Anspruch 1 angegebene Bedeutung haben,
überführt,
und abschließend nach bekannten Methoden mit Verbindungen der Formel (VI) oder deren Salzen 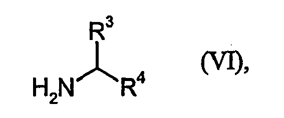 in welcher
R³ und R⁴ die in Anspruch 1 angegebene Bedeutung haben,
in inerten Lösemitteln umsetzt
oder
[B] für den Fall, dass A ein Sauerstoffatom oder NR⁵ bedeutet,
Verbindungen der Formel (VII) 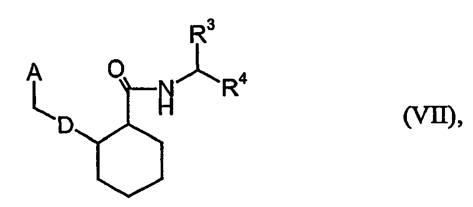
in welcher
D, R³ und R⁴ die in Anspruch 1 angegebenen Bedeutungen haben,
und
A ein Sauerstoffatom oder eine Gruppe der Formel N-R⁵ bedeutet,
wobei R⁵ die in Anspruch 1 angegebene Bedeutung hat,
entweder mit Verbindungen der Formel (VIII) 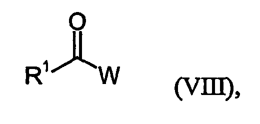 in welcher
R¹ die in Anspruch 1 angegebene Bedeutung hat und W für eine geeignete Abgangsgruppe steht
oder
mit einem Phosgenäquivalent und anschliessend mit Verbindungen der Formel (IX)
R⁷R⁸NH (IX),
in welcher
R⁷ und R⁸ die in Anspruch 1 angegebenen Bedeutungen haben,
oder
mit einem Isocyanat der Formel (X)
R⁷NCO (X),
in welcher
R⁷ die in Anspruch 1 angegebenen Bedeutungen hat,
umsetzt.

10. Verfahren zur Herstellung von Verbindungen der Formel (I), **dadurch gekennzeichnet, dass** man
[A] Verbindungen der Formel (V) 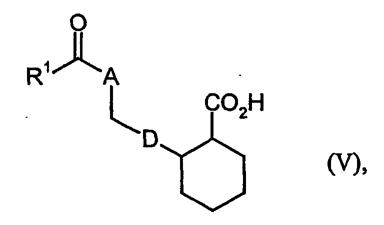 in welcher
R¹, A und D die in Anspruch 1 angegebene Bedeutung haben,
nach bekannten Methoden mit Verbindungen der Formel (VI) oder deren Salzen 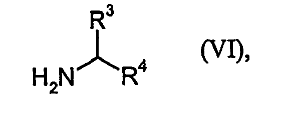 in welcher
R³ und R⁴ die in Anspruch 1 angegebene Bedeutung haben,
in inerten Lösemitteln umsetzt
oder
[B] für den Fall, dass A ein Sauerstoffatom oder NR⁵ bedeutet,
Verbindungen der Formel (VII) 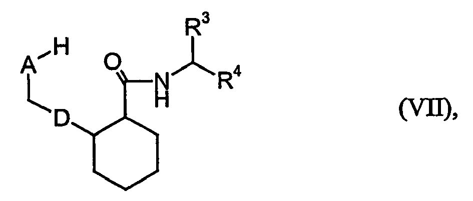
in welcher
D, R³ und R⁴ die in Anspruch 1 angegebenen Bedeutungen haben,
und
A ein Sauerstoffatom oder eine Gruppe der Formel N-R⁵ bedeutet,
wobei R⁵ die in Anspruch 1 angegebene Bedeutung hat,
entweder mit Verbindungen der Formel (VIII) 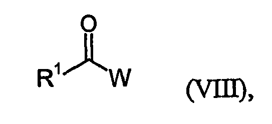 in welcher
R¹ die in Anspruch 1 angegebene Bedeutung hat und W für eine geeignete Abgangsgruppe steht
oder
mit einem Phosgenäquivalent und anschliessend mit Verbindungen der Formel (IX)
R⁷R⁸NH (IX),
in welcher
R⁷ und R⁸ die in Anspruch 1 angegebenen Bedeutungen haben,
oder
mit einem Isocyanat der Formel (X)
R⁷NCO (X),
in welcher
R⁷ die in Anspruch 1 angegebenen Bedeutungen hat,
umsetzt.

11. Verbindungen der Formel (V) 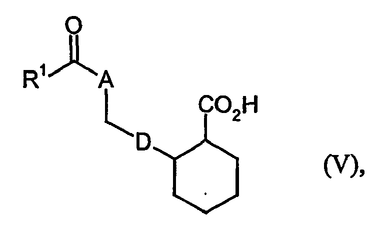 in welcher
R¹, A und D die in Anspruch 1 angegebene Bedeutung haben,
und ihre Salze, Hydrate, Hydrate der Salze und Solvate.

12. Verbindungen der Formel (VII) 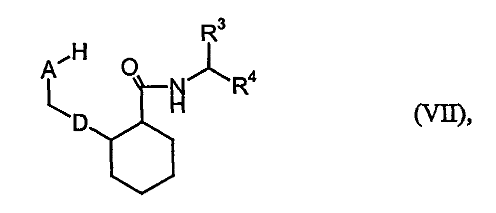 in welcher
R³, R⁴, A und D die in Anspruch 1 angegebene Bedeutung haben,
und ihre Salze, Hydrate, Hydrate der Salze und Solvate.

13. Verbindungen der Formel (I), wie in einem der vorhergehenden Ansprüche definiert, zur Bekämpfung von Erkrankungen.

14. Arzneimittel, enthaltend mindestens eine Verbindung der Formel (I), wie in einem der vorhergehenden Ansprüche definiert, und mindestens einen weiteren Wirkstoff.

15. Arzneimittel, enthaltend mindestens eine Verbindung der Formel (I), wie in einem der vorhergehenden Ansprüche definiert, und mindestens einen weiteren Hilfsstoff.

16. Verwendung von Verbindungen der Formel (I), wie in einem der vorhergehenden Ansprüche definiert, zur Herstellung von Arzneimitteln zur Prävention und/oder Behandlung von ischämiebedingten periphären und kardiovaskulären Erkrankungen.

17. Verwendung von Verbindungen der Formel (I), wie in einem der vorhergehenden Ansprüche definiert, zur Herstellung von Arzneimitteln zur akuten und chronischen Behandlung von ischämischen Erkrankungen des Herz-Kreislauf-Systems, wie z.B. der koronaren Herzkrankheit, der stabilen und instabilen Angina pectoris, von peripheren und arteriellen Verschlusskrankheiten, von thrombotischen Gefäßverschlüssen, des Myocardinfarkts und von Reperfusionsschäden

## Claims

1. Compounds of the formula (I) 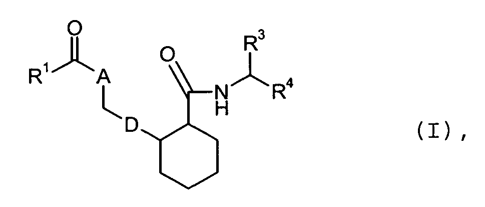 in which
D represents a radical 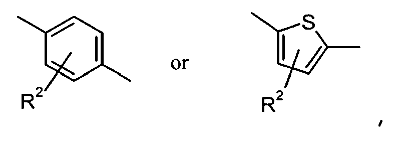 in which
R² represents hydrogen, halogen, hydroxyl, carboxyl, cyano, nitro, trifluoromethyl, trifluoromethoxy, (C₁-C₆)-alkyl, (C₁-C₆)-alkoxy or (C₁-C₆)-alkoxycarbonyl,
A represents an oxygen atom or a group of the formula N-R⁵ or CH-R⁶,
in which
R⁵ represents hydrogen, (C₁-C₆)-alkyl, (C₃-C₇)-cycloalkyl, where alkyl and cycloalkyl for their part may be substituted up to three times independently of one another by hydroxyl or mono- or di-(C₁-C₆)-alkylamino, represents (C₆-C₁₀)-aryl, 5- to 10-membered heteroaryl having up to three heteroatoms from the group consisting of N, O and S or 5- to 6-membered heterocyclyl having up to three heteroatoms from the group consisting of N, O and S, where aryl, heteroaryl and heterocyclyl for their part may be substituted up to three times independently of one another by halogen, hydroxyl, cyano, nitro, trifluoromethyl, trifluoromethoxy, (C₁-C₆)-alkyl, (C₁-C₆)-alkoxy, (C₁-C₆)-alkoxycarbonyl or mono-or di-(C₁-C₆)-alkylamino,
R⁶ represents hydrogen, (C₁-C₆)-alkoxycarbonyl or carboxyl,
R¹ represents hydrogen, (C₁-C₆)-alkyl, which for its part may be substituted by hydroxyl or (C₁-C₄) -alkoxy, represents (C₃-C₇)-cycloalkyl, (C₆-C₁₀)-aryl, 5- to 10-membered heteroaryl having up to two heteroatoms from the group consisting of N, O and S, where aryl and heteroaryl for their part may be substituted independently of one another by halogen, or represents a radical of the formula -NR⁷R⁸ or -OR⁹,
in which
R⁷ and R⁸ independently of one another represent hydrogen, (C₆-C₁₀) -aryl, adamantyl, (C₁-C₈) -alkyl, whose chain may be interrupted by one or two oxygen atoms and which may be substituted up to three times independently of one another by hydroxyl, phenyl, trifluoromethyl, (C₃-C₈) -cycloalkyl, (C₁-C₆) -alkoxy, mono-or di- (C₁-C₆) -alkylamino, 5- or 6-membered heterocyclyl having up to three heteroatoms from the group consisting of N, O and S or by 5- to 10-membered heteroaryl having up to three heteroatoms from the group consisting of N, O and S, represent (C₃-C₈)-cycloalkyl, which may be substituted up to three times independently of one another by (C₁-C₄)-alkyl, hydroxyl or oxo, or represent 5- or 6-membered heterocyclyl having up to two heteroatoms from the group consisting of N, O and S, where N is substituted by hydrogen or (C₁-C₄)-alkyl,
or
R⁷ and R⁸ together with the nitrogen atom to which they are attached form a 4- to 7-membered saturated heterocycle which may contain up to two further heteroatoms from the group consisting of N, O and S and which is optionally substituted by hydroxyl, oxo or (C₁-C₆)-alkyl, which for its part may be substituted by hydroxyl,
and
R⁹ represents (C₆-C₁₀)-aryl, adamantyl, (C₁-C₈)-alkyl, whose chain may be interrupted by one or two oxygen atoms and which may be substituted up to three times independently of one another by hydroxyl, phenyl, trifluoroomethyl, (C₃-C₈) -cycloalkyl, (C₁-C₆) -alkoxy, mono-or di- (C₁-C₆) -alkylamino, 5- or 6-membered heterocyclyl having up to three heteroatoms from the group consisting of N, O and S or by 5- to 10-membered heteroaryl having up to three heteroatoms from the group consisting of N, O and S, represents (C₃-C₈)-cycloalkyl, which may be substituted up to three times independently of one another by (C₁-C₄)-alkyl, hydroxyl or oxo, or represents 5- or 6-membered heterocyclyl having up to two hetero-atoms from the group consisting of N, O and S, where N is substituted by hydrogen or (C₁-C₄) -alkyl,
R³ represents (C₁-C₈)-alkyl, whose chain may be interrupted by a sulphur or oxygen atom or an S(O) or SO₂ group, represents phenyl, benzyl or 5- or 6-membered heteroaryl having up to two heteroatoms from the group consisting of N, O and S, where phenyl, benzyl and heteroaryl may be substituted up to three times independently of one another by halogen, trifluoroomethyl, cyano, nitro, hydroxyl, (C₁-C₆)-alkyl or (C₁-C₆)-alkoxy,
and
R⁴ represents a radical of the formula -C(O)-NR¹⁰R¹¹,
in which
R¹⁰ and R¹¹ independently of one another represent hydrogen or (C₁-C₆) -alkyl,
and their salts, hydrates, hydrates of the salts and solvates.

2. Compounds according to Claim 1,
in which
D represents a radical 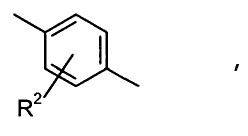 in which
R² represents hydrogen, halogen, hydroxyl, carboxyl, cyano, nitro, trifluoromethyl, trifluoroomethoxy, (C₁-C₆)-alkyl, (C₁-C₆)-alkoxy or (C₁-C₆)-alkoxycarbonyl,
A represents an oxygen atom or a group of the formula N-R⁵ or CH-R⁶,
in which
R⁵ represents hydrogen, (C₁-C₆) -alkyl, (C₃-C₇)-cycloalkyl, where alkyl and cycloalkyl for their part may be substituted up to three times independently of one another by hydroxyl or mono- or di-(C₁-C₆)-alkylamino, represents (C₆-C₁₀)-aryl, 5- to 10-membered heteroaryl having up to three heteroatoms from the group consisting of N, O and S or 5- to 6-membered heterocyclyl having up to three heteroatoms from the group consisting of N, O and S, where aryl, heteroaryl and heterocyclyl for their part may be substituted up to three times independently of one another by halogen, hydroxyl, cyano, nitro, trifluoroomethyl, trifluoroomethoxy, (C₁-C₆) -alkyl, (C₁-C₆) -alkoxy, (C₁-C₆)-alkoxycarbonyl or mono- or di- (C₁-C₆)-alkylamino,
R⁶ represents hydrogen, (C₁-C₆)-alkoxycarbonyl or carboxyl,
R¹ represents hydrogen, (C₁-C₆) -alkyl, which for its part may be substituted by hydroxyl or (C₁-C₄) -alkoxy, represents (C₃-C₇)-cycloalkyl, (C₆-C₁₀) -aryl, 5- to 10-membered heteroaryl having up to two heteroatoms from the group consisting of N, O and S, where aryl and heteroaryl for their part may be substituted independently of one another by halogen, or represents a radical of the formula -NR⁷R⁸ or -OR⁹,
in which
R⁷ and R⁸ independently of one another represent hydrogen, (C₆-C₁₀) -aryl, adamantyl, (C₁-C₈) -alkyl, whose chain may be interrupted by one or two oxygen atoms and which may be substituted up to three times independently of one another by hydroxyl, phenyl, trifluoromethyl, (C₃-C₈) -cycloalkyl, (C₁-C₆) -alkoxy, mono-or di- (C₁-C₆) -alkylamino, 5- or 6-membered heterocyclyl having up to three heteroatoms from the group consisting of N, O and S or by 5- to 10-membered heteroaryl having up to three heteroatoms from the group consisting of N, O and S, represent (C₃-C₈)-cycloalkyl, which may be substituted up to three times independently of one another by (C₁-C₄) -alkyl, hydroxyl or oxo, or represent 5- or 6-membered heterocyclyl having up to two heteroatoms from the group consisting of N, O and S, where N is substituted by hydrogen or (C₁-C₄)-alkyl,
or
R⁷ and R⁸ together with the nitrogen atom to which they are attached form a 4- to 7-membered saturated heterocycle which may contain up to two heteroatoms from the group consisting of N, O and S and which is optionally substituted by hydroxyl, oxo or (C₁-C₆) -alkyl, which for its part may be substituted by hydroxyl,
and
R⁹ represents (C₆-C₁₀)-aryl, adamantyl, (C₁-C₈) -alkyl, whose chain may be interrupted by one or two oxygen atoms and which may be substituted up to three times independently of one another by hydroxyl, phenyl, trifluoromethyl, (C₃-C₈)-cycloalkyl, (C₁-C₆)-alkoxy, mono-or di-(C₁-C₆)-alkylamino, 5- or 6-membered heterocyclyl having up to three heteroatoms from the group consisting of N, O and S or by 5- to 10-membered heteroaryl having up to three heteroatoms from the group consisting of N, O and S, represents (C₃-C₈)-cycloalkyl, which may be substituted up to three times independently of one another by (C₁-C₄) -alkyl, hydroxyl or oxo, or represents 5- or 6-membered heterocyclyl having up to two heteroatoms from the group consisting of N, O and/or S, where N is substituted by hydrogen or (C₁-C₄)-alkyl,
R³ represents (C₁-C₈)-alkyl, whose chain may be interrupted by a sulphur atom or an S(O) or SO₂ group, represents phenyl, benzyl or 5- or 6-membered heteroaryl having up to two heteroatoms from the group consisting of N, O and S, where phenyl, benzyl and heteroaryl may be substituted up to three times independently of one another by halogen, trifluoromethyl, cyano, nitro, hydroxyl, (C₁-C₆)-alkyl or (C₁-C₆)-alkoxy,
and
R⁴ represents a radical of the formula -C(O)-NR¹⁰R¹¹,
in which
R¹⁰ and R¹¹ independently of one another represent hydrogen or (C₁-C₆)-alkyl,
and their salts, hydrates, hydrates of the salts and solvates.

3. Compounds according to Claim 1,
in which
D represents a radical 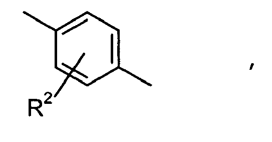 in which
R² represents hydrogen, chlorine or fluorine,
A represents an oxygen atom or a group of the formula N-R⁵,
in which
R⁵ represents hydrogen, (C₁-C₆)-alkyl, which for its part may be substituted up to two times by hydroxyl, represents (C₃-C₇) -cycloalkyl, phenyl or 5- or 6-membered heteroaryl having up to three heteroatoms from the group consisting of N, O and S, where phenyl and heteroaryl for their part may be substituted up to two times independently of one another by halogen, cyano, trifluoromethyl, trifluoromethoxy, (C₁-C₄) -alkyl, (C₁-C₄) - alkoxy or di- (C₁-C₄) -alkylamino,
R¹ represents hydrogen, (C₁-C₆) -alkyl, which for its part may be substituted by hydroxyl or (C₁-C₄) -alkoxy, represents (C₃-C₇)-cycloalkyl, phenyl, 5- to 6-membered heteroaryl having up to two heteroatoms from the group consisting of N, O and S, where phenyl and heteroaryl for their part independently may be substituted independently of one another by halogen, or represents a radical of the formula -NR⁷R⁸ or -OR⁹,
in which
R⁷ and R⁸ independently of one another represent hydrogen, phenyl, adamantyl, (C₁-C₆) -alkyl, whose chain may be interrupted by one or two oxygen atoms and which may be substituted up to two times independently of one another by hydroxyl, phenyl, trifluoroomethyl, (C₃-C₆) -cycloalkyl, (C₁-C₄)-alkoxy, mono- or di-(C₁-C₄)-alkylamino, 5- to 6-membered heterocyclyl having up to two heteroatoms from the group consisting of N and O or by 5- to 6-membered heteroaryl having up to three heteroatoms from the group consisting of N, O and S, represents (C₃-C₈)-cycloalkyl, which may be substituted up to two times by hydroxyl, or represent 5- to 6-membered heterocyclyl having up to two heteroatoms from the group consisting of N, O and S, where N is substituted by hydrogen or (C₁-C₄)-alkyl,
or
R⁷ and R⁸ together with the nitrogen atom to which they are attached form a 4- to 7-membered saturated heterocycle which may contain up to two further heteroatoms from the group consisting of N, O and S and which is optionally substituted by by hydroxyl, oxo or (C₁-C₆) -alkyl, which for its part may be substituted by hydroxyl,
and
R⁹ represents phenyl, adamantyl, (C₁-C₆)-alkyl, whose chain may be interrupted by one or two oxygen atoms and which may be substituted up to two times independently of one another by hydroxyl, phenyl, trifluoroomethyl, (C₃-C₆) -cycloalkyl, (C₁-C₃) -alkoxy, mono-or di-(C₁-C₄)-alkylamino, 5- to 6-membered heterocyclyl having up to two heteroatoms from the group consisting of N and O or by 5- to 6-membered heteroaryl having up to three heteroatoms from the group consisting of N, O and S, represents (C₃-C₈)-cycloalkyl, which may be substituted up to two times by hydroxyl, or represents 5- to 6-membered heterocyclyl having up to two heteroatoms from the group consisting of N, O and S, where N is substituted by hydrogen or (C₁-C₄)-alkyl,
R³ represents (C₁-C₈)-alkyl, whose chain may be interrupted by a sulphur atom or an S(O) or SO₂ group, represents phenyl, benzyl or 5- or 6-membered heteroaryl having up to two heteroatoms from the group consisting of N, O and S, where phenyl, benzyl and heteroaryl may be substituted up to two times independently of one another by halogen, trifluoroomethyl, cyano, (C₁-C₃)-alkyl, (C₁-C₃) -alkoxy or hydroxyl,
and
R⁴ represents a radical of the formula -C(O)-NR¹⁰R¹¹,
in which
R¹⁰ and R¹¹ independently of one another represent hydrogen or (C₁-C₆) -alkyl,
and their salts, hydrates, hydrates of the salts and solvates.

4. Compounds according to Claim 1,
in which
D represents a radical of the formula 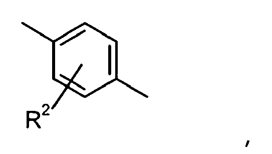 in which
R² represents hydrogen,
A represents an oxygen atom or a group of the formula N-R⁵,
in which
R⁵ represents hydrogen, (C₁-C₆) -alkyl, which for its part may be substituted up to two times by hydroxyl, represents (C₃-C₇) -cycloalkyl, phenyl or 5- to 6-membered heteroaryl having up to three heteroatoms from the group consisting of N, O and S, where phenyl and heteroaryl for their part may be substituted up to two times independently of one another by fluorine, chlorine, cyano, trifluoroomethyl, trifluoroomethoxy, (C₁-C₃) -alkyl, (C₁-C₃) -alkoxy or di- (C₁-C₃)-alkylamino,
R¹ represents (C₁-C₄)-alkyl or a radical of the formula -NR⁷R⁸,
in which
R⁷ and R⁸ independently of one another represent hydrogen, phenyl, adamantyl, (C₁-C₄) -alkyl, whose chain may be interrupted by one or two oxygen atoms and which may be substituted up to two times independently of one another by hydroxyl, phenyl, trifluoroomethyl, (C₃-C₆)-cycloalkyl, (C₁-C₃)-alkoxy, mono- or di-(C₁-C₃)-alkylamino, 5- to 6-membered heterocyclyl having up to two heteroatoms from the group consisting of N and O or by 5- to 6-membered heteroaryl having up to three heteroatoms from the group consisting of N, O and S, represent (C₃-C₈)-cycloalkyl, which may be substituted up two times by hydroxyl, or represents 5- to 6-membered heterocyclyl having up to two heteroatoms from the group consisting of N, O and S, where N is substituted by hydrogen or (C₁-C₄)-alkyl,
or
R⁷ and R⁸ together with the nitrogen atom to which they are attached form a 4- to 7-membered saturated heterocycle which may contain up to two further heteroatoms from the group consisting of N, O and S and which is optionally substituted by by hydroxyl, oxo or (C₁-C₆)-alkyl, which for its part may be substituted by hydroxyl,
R³ represents (C₁-C₈)-alkyl, whose chain may be interrupted by a sulphur atom or an S(O) or SO₂ group, represents phenyl, benzyl or 5- to 6-membered heteroaryl having up to two heteroatoms from the group consisting of N, O and S, where phenyl, benzyl and heteroaryl may be substituted up to two times independently of one another by halogen, trifluoroomethyl, cyano, (C₁-C₃)-alkyl, (C₁-C₃)-alkoxy or hydroxyl,
and
R⁴ represents a radical of the formula -C(O)-NR¹⁰R¹¹,
in which
R¹⁰ and R¹¹ independently of one another represent hydrogen, methyl or ethyl,
and their salts, hydrates, hydrates of the salts and solvates.

5. Compounds according to Claim 1,
in which
D represents a radical 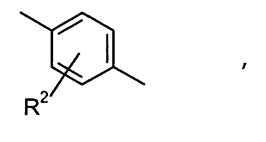 in which
R² represents hydrogen,
A represents an oxygen atom or a group of the formula N-R⁵,
in which
R⁵ represents (C₃-C₇)-cycloalkyl, phenyl, which for its part may be substituted by fluorine, or represents pyridyl,
R¹ represents methyl or a radical of the formula-NR⁷R⁸,
in which
R⁷ and R⁸ independently of one another represent (C₁-C₄) -alkyl, which may be mono- or disubstituted by hydroxyl,
or
R⁷ and R⁸ together with the nitrogen atom to which they are attached form a 5- to 6-membered saturated heterocycle which may contain a further heteroatom O or N, where N is substituted by hydrogen or (C₁-C₃) -alkyl, which for its part may be substituted by hydroxyl,
R³ represents phenyl, which is optionally substituted in the para-position by fluorine, or represents pyridyl,
and
R⁴ represents a radical of the formula -C(O)-NR¹⁰R¹¹,
in which
R¹⁰ and R¹¹ represent hydrogen,
and their salts, hydrates, hydrates of the salts and solvates.

6. Compounds according to Claim 1, **characterized by** one of the following stereochemical configurations according to formulae (Ia) to (Id):

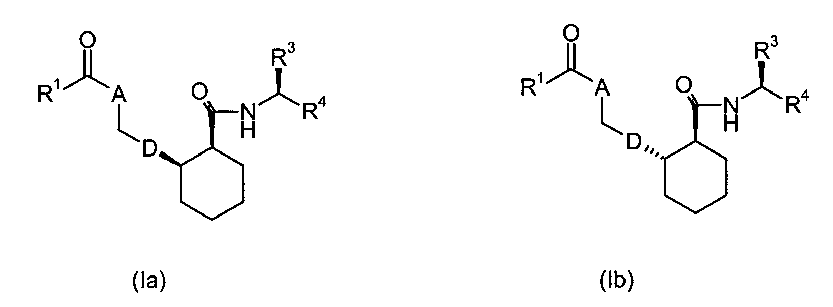

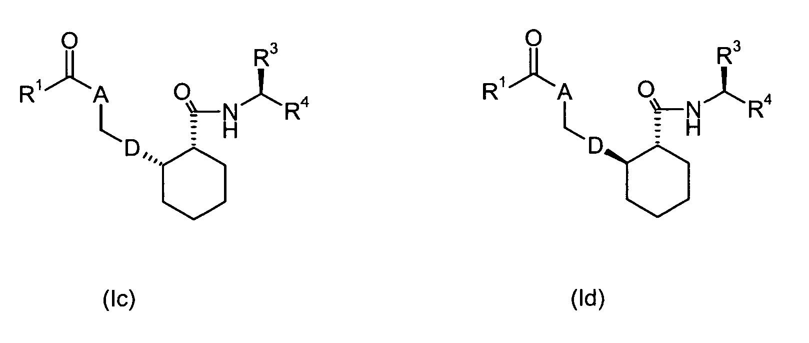

7. Compounds according to Claim 1, **characterized by** the following stereochemical configuration according to formula (Id):

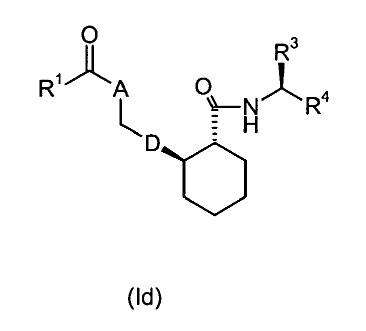

8. Compounds according to Claim 1 having the following structures:
(1*R*,2*R*)-*N*-[(1*S*)-2-amino-1-(4-fluorophenyl)-2-oxoethyl]-2-(4-{[{[ethyl(2-hydroxyethyl)-amino]carbonyl}(4-fluorophenyl)amino]methyl}phenyl)cyclohexanecarboxamide 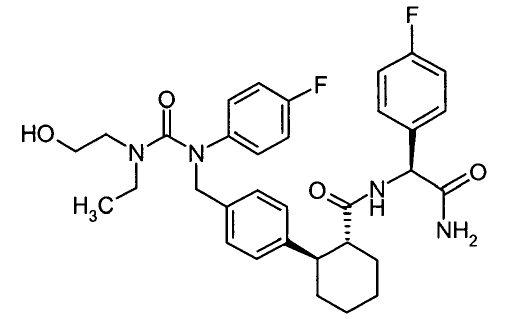
(1*R*,2*R*)-*N*-[(1*S*)-2-amino-2-oxo-1-phenylethyl]-2-(4-{[[(dimethylamino)carbonyl]-(phenyl)amino]methyl}phenyl)cyclohexanecarboxamide 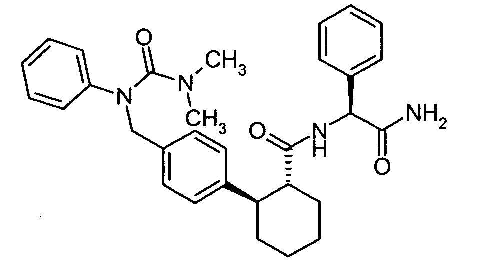
(1*R*, 2*R*)-*N*-[(1*S*)-2-amino-2-oxo-1-phenylethyl]-2-[4-({cyclopropyl[(dimethylamino)-carbonyl]amino}methyl)phenyl]cyclohexanecarboxamid e 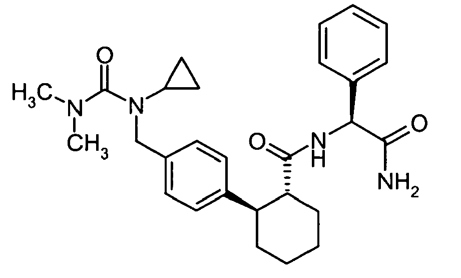
(1*R*,2*R*)-*N*-[(1*S*)-2-amino-2-oxo-1-phenylethyl]-2-(4-{[[(diethylamino)carbonyl](2-pyridinyl)amino]methyl}phenyl)cyclohexanecarboxami de 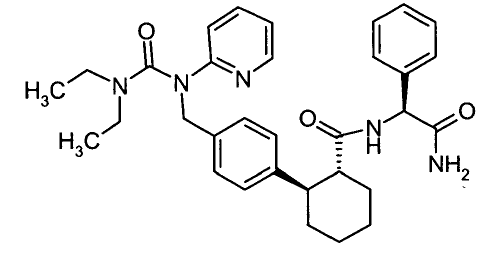
*N*-{4-[(1*R*,2*R*)-2-({[(1*S*)-2-amino-2-oxo-1-phenylethyl]amino}carbonyl)cyclohexyl]benzyl}-*N-*phenyl-4-morpholinecarboxamide 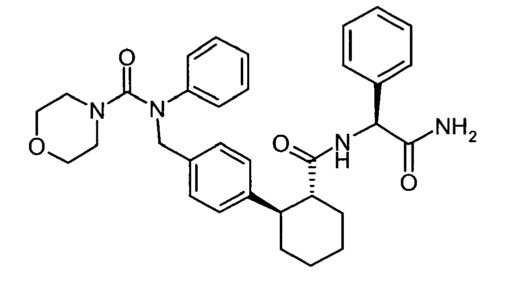
(S) -N-{{(1*R*, 2*R*) -2- (4-{[{[2-hydroxylethylamino]carbonyl}(phenyl)amino]methyl}-phenyl)cyclohex-1-yl}carbonyl}-phenylglycinamide 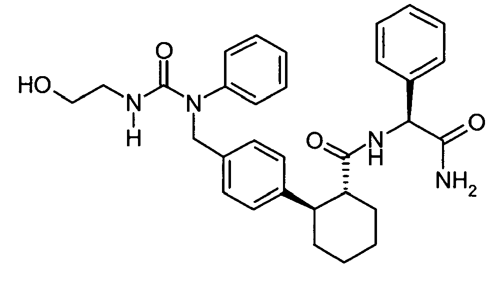
(1R,2R)-2-(4-{[acetyl (2-pyridinyl)amino]methyl}phenyl)-N-[(1S)-2-amino-2-oxo-1-phenylethyl]cyclohexancarboxamide 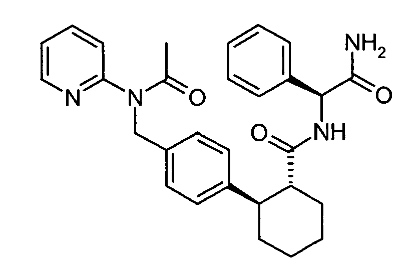
(1*R*, 2*R*)-*N*-[(1*S*)-2-amino-1-phenyl-2-oxoethyl]-2-(4-{[{[ethyl(2-hydroxyethyl)amino]-carbonyl}(phenyl)amino]methyl}phenyl)cyclohexanecarboxamide 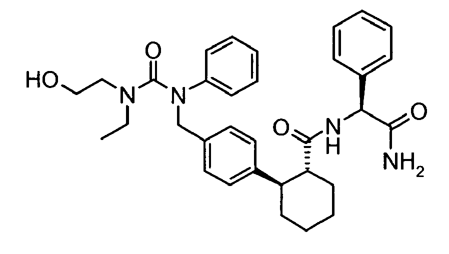
4-[(1*R*,2*R*)-2-({[(1*S*)-2-amino-1-(4-fluorophenyl)-2-oxoethyl]amino}carbonyl)cyclohexyl]benzyl 4-(2-hydroxyethyl)-1-piperazinecarbamate 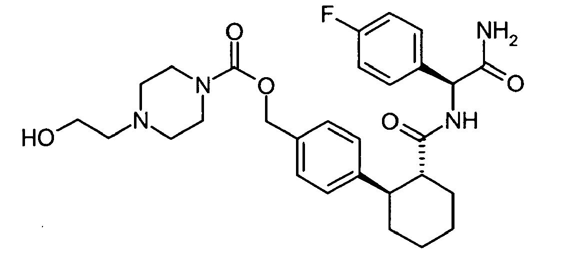
4-[(1*R*,2*R*)-2-({[(1*S*)-2-amino-1-phenyl-2-oxoethyl]amino}carbonyl)cyclohexyl]benzyl-4-(2-hydroxyethyl)-1-piperazinecarbamate 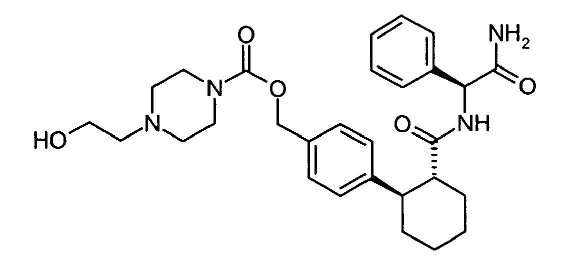
and their salts, hydrates, hydrates of the salts and solvates.

9. Process for preparing compounds of the formula (I), **characterized in that**
[A] compounds of the formula (II) 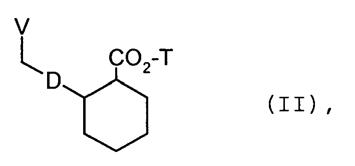 in which
D is as defined in Claim 1,
T represents (C₁-C₄) -alkyl,
and
V represents a suitable leaving group
are initially converted by reaction with compounds of the formula (III)
B-H (III),
in which
B represents 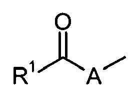 or
optionally, if R¹ reprsents OR⁹,
represents 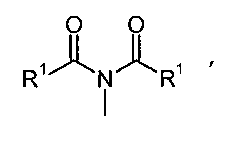 and
R¹ and A are as defined in Claim 1,
and to the compounds of the formula (IV) 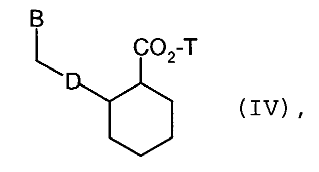 in which B and T are as defined above and D is as defined in Claim 1,
these compounds are in a next step converted with acids or bases into the corresponding carboxylic acids of the formula (V) 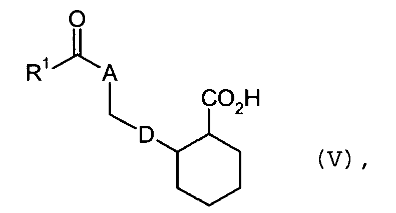 in which
R¹, A and D are as defined in Claim 1,
and these compounds are finally reacted in inert solvents according to known methods with compounds of the formula (VI) or salts thereof 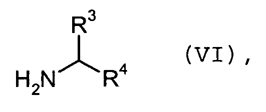 in which
R³ and R⁴ are as defined in Claim 1,
or
[B] if A represents an oxygen atom or NR⁵,
compounds of the formula (VII) 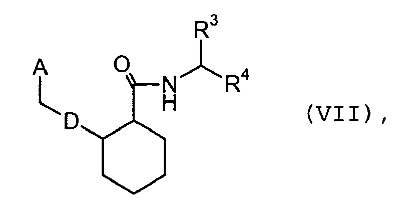
in which
D, R³ and R⁴ are as defined in Claim 1,
and
A represents an oxygen atom or a group of the formula N-R⁵,
where R⁵ is as defined in Claim 1,
are reacted either with compounds of the formula (VIII) 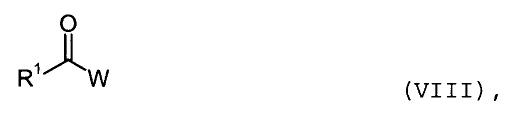 in which
R¹ is as defined in Claim 1 and W represents a suitable leaving group
or
with a phosgene equivalent and then with compounds of the formula (IX)
R⁷R⁸NH (IX),
in which
R⁷and R⁸ are as defined in Claim 1
or
with an isocyanate of the formula (X)
R⁷NCO (X),
in which
R⁷ is as defined in Claim 1.

10. Process for preparing compounds of the formula (I), **characterized in that**
[A] compounds of the formula (V) 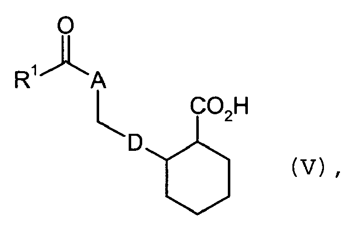 in which
R¹, A and D are as defined in Claim 1,
are reacted in inert solvents according to known methods with compounds of the formula (VI) or salts thereof 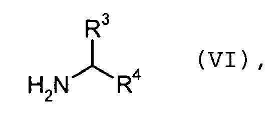 in which
R³ and R⁴ are as defined in Claim 1,
or
[B] if A represents an oxygen atom or NR⁵,
compounds of the formula (VII) 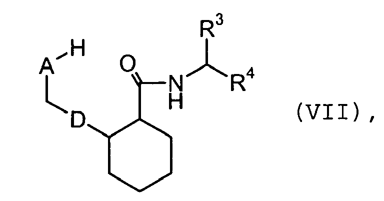
in which
D, R³ and R⁴ are as defined in Claim 1,
and
A represents an oxygen atom or a group of the formula N-R⁵,
where R⁵ is as defined in Claim 1,
are reacted either with compounds of the formula (VIII) 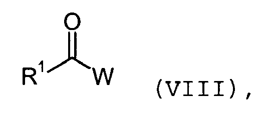 in which
R¹ is as defined in Claim 1 and W represents a suitable leaving group
or
with a phosgene equivalent and then with compounds of the formula (IX)
R⁷R⁸NH (IX),
in which
R⁷ and R⁸ are as defined in Claim 1,
or
with an isocyanate of the formula (X)
R⁷NCO (X),
in which
R⁷ is as defined in Claim 1.

11. Compounds of the formula (V) 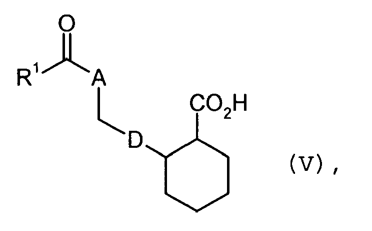 in which
R¹, A and D are as defined in Claim 1,
and their salts, hydrates, hydrates of the salts and solvates.

12. Compounds of the formula (VII) 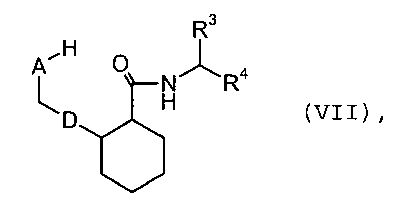 in which
R³, R⁴, A and D are as defined in Claim 1,
and their salts, hydrates, hydrates of the salts and solvates.

13. Compounds of the formula 1 as defined in any of the preceding claims for controlling diseases.

14. Medicaments, comprising at least one compound of the formula (I) as defined in any of the preceding claims and at least one further active compound.

15. Medicaments, comprising at least one compound of the formula (I) as defined in any of the preceding claims and at least one further auxiliary.

16. Use of compounds of the formula (I) as defined in any of the preceding claims for preparing medicaments for the prevention and/or treatment of peripheral and cardiovascular disorders caused by ischaemia.

17. Use of compounds of the formula (I) as defined in any of the preceding claims for preparing medicaments for the acute and chronic treatment of ischaemic disorders of the cardiovascular system such as, for example, coronary heart disease, stable and unstable angina pectoris, of peripheral and arterial occlusive diseases, of thrombotic vascular occlusions, of myocardial infarction and of reperfusion damage.

## Revendications

1. Composés de formule (I) 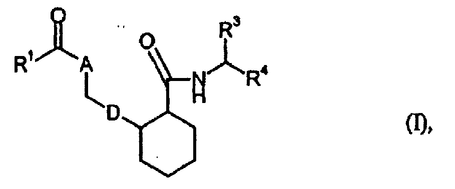 dans laquelle
D représente un reste 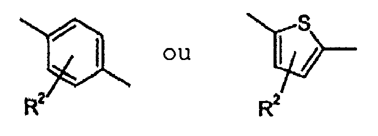 où
R² représente l'hydrogène, un halogène, un groupe hydroxy, carboxyle, cyano, nitro, trifluorométhyle, trifluorométhoxy, alkyle en C₁ à C₆, alkoxy en C₁ à C₆ ou (alkoxy en C₁ à C₆)carbonyle,
A désigne un atome d'oxygène ou un groupe de formule N-R⁵ ou CH-R⁶,
où
R⁵ représente l'hydrogène, un reste alkyle en C₁ à C₆, cycloalkyle en C₃ à C₇, les restes alkyle et cycloalkyle pouvant eux-mêmes porter jusqu'à trois substituants, indépendamment les uns des autres, hydroxy ou mono- ou di(alkyle en C₁ à C₆)amino, un reste aryle en C₆ à C₁₀, un reste hétéroaryle penta- à décagonal ayant jusqu'à trois hétéroatomes de la série N, O et/ou S ou un reste hétérocyclyle penta- ou hexagonal ayant jusqu'à trois hétéroatomes de la série N, O et/ou S, les restes aryle, hétéroaryle et hétérocyclyle pouvant eux-mêmes porter jusqu'à trois substituants, indépendants les uns des autres, halogéno, hydroxy, cyano, nitro, trifluorométhyle, trifluorométhoxy, alkyle en C₁ à C₆, alkoxy en C₁ à C₆, (alkoxy en C₁ à C₆) carbonyle ou mono- ou di (alkyle en C₁ à C₆)amino,
R⁶ représente l'hydrogène, un reste (alkoxy en C₁ à C₆)carbonyle ou un groupe carboxyle,
R¹ représente l'hydrogène, un reste alkyle en C₁ à C₆ qui peut lui-même être substitué par un radical hydroxy ou alkoxy en C₁ à C₄, un reste cycloalkyle en C₃ à C₇, aryle en C₆ à C₁₀, un reste hétéroaryle penta- à décagonal ayant jusqu'à trois hétéroatomes de la série N, O et/ou S, les restes aryle et hétéroaryle pouvant eux-mêmes être substitués indépendamment les uns des autres par un radical halogéno, ou bien un reste de formule -NR⁷R⁸ ou -OR⁹,
où
R⁷ et R⁸ représentent, indépendamment l'un de l'autre, l'hydrogène, un reste aryle en C₆ à C₁₀, adamantyle, alkyle en C₁ à C₈, dont la chaîne peut être interrompue par un ou deux atomes d'oxygène et qui peut porter jusqu'à trois substituants, indépendants les uns des autres, hydroxy, phényle, trifluorométhyle, cycloalkyle en C₃ à C₈, alkoxy en C₁ à C₆, mono- ou di (alkyle en C₁ à C₆)amino, hétérocyclyle penta- ou hexagonal ayant jusqu'à trois hétéroatomes de la série N, O et/ou S ou un radical hétéroaryle penta- à décagonal ayant jusqu'à trois hétéroatomes de la série N, O et/ou S, un reste cycloalkyle en C₃ à C₈ qui peut porter jusqu'à trois substituants, indépendamment les uns des autres, alkyle en C₁ à C₄, hydroxy ou oxo, ou bien un reste hétérocyclyle penta- ou hexagonal ayant jusqu'à deux hétéroatomes de la série N, O et/ou S, N étant substitué par de l'hydrogène ou un reste alkyle en C₁ à C₄,
ou bien
R⁷ et R⁸ forment, conjointement avec l'atome d'azote auquel ils sont liés, un hétérocycle saturé tetra-à heptagonal qui peut contenir jusqu'à deux autres hétéroatomes de la série N, O et/ou S et qui est éventuellement substitué par un radical hydroxy, oxo ou alkyle en C₁ à C₆ qui peut lui-même porter un substituant hydroxy,
et
R⁹ représente un reste aryle en C₆ à C₁₀, adamantyle, alkyle en C₁ à C₈, dont la chaîne peut être interrompue par un ou deux atomes d'oxygène et qui peut porter jusqu'à trois substituants, indépendamment les uns des autres, hydroxy, phényle, trifluorométhyle, cycloalkyle en C₃ à C₈, alkoxy en C₁ à C₆, mono- ou di (alkyle en C₁ à C₆)amino, hétérocyclyle penta- ou hexagonal ayant jusqu'à trois hétéroatomes de la série N, O et/ou S ou hétéroaryle penta- à décagonal ayant jusqu'à trois hétéroatomes de la série N, O et/ou S, un reste cycloalkyle en C₃ à C₈ qui peut porter jusqu'à trois substituants, indépendamment les uns des autres, alkyle en C₁ à C₄, hydroxy ou oxo, ou bien un reste hétérocyclyle penta- ou hexagonal ayant jusqu'à deux hétéroatomes de la série N, O et/ou S, N étant substitué par de l'hydrogène ou un radical alkyle en C₁ à C₄,
R³ représente un reste alkyle en C₁ à C₈, dont la chaîne peut être interrompue par un atome de soufre ou d'oxygène, ou par un groupe S(O) ou SO₂, un reste phényle, benzyle, hétéroaryle penta- ou hexagonal ayant jusqu'à deux hétéroatomes de la série N, O et/ou S, les restes phényle, benzyle et hétéroaryle pouvant porter jusqu'à trois substituants, indépendamment les uns des autres, halogéno, trifluorométhyle, cyano, nitro, hydroxy, alkyle en C₁ à C₆ ou alkoxy en C₁ à C₆,
et
R⁴ représente un reste de formule -C (O) -NR¹⁰R¹¹,
où
R¹⁰ et R¹¹ représentent, indépendamment l'un de l'autre, l'hydrogène ou un reste alkyle en C₁ à C₆,
et leurs sels, leurs hydrates, les hydrates de leurs sels et leurs produits de solvatation

2. Composés suivant la revendication 1,
dans lesquels
D représente un reste de formule 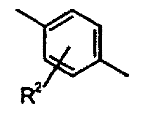 dans laquelle
R² représente l'hydrogène, un halogène, un groupe hydroxy, carboxyle, cyano, nitro, un reste trifluorométhyle, trifluorométhoxy, alkyle en C₁ à C₆, alkoxy en C₁ à C₆ ou (alkoxy en C₁ à C₆) carbonyle,
A représente un atome d'oxygène ou un groupe de formule N-R⁵ ou CH-R⁶,
où
R⁵ représente l'hydrogène, un reste alkyle en C₁ à C₆, cycloalkyle en C₃ à C₇, les restes alkyle et cycloalkyle pouvant eux-mêmes porter jusqu'à trois substituants, indépendants les uns des autres, hydroxy ou mono- ou di(alkyle en C₁ à C₆)amino, un reste aryle en C₆ à C₁₀, hétéroaryle penta- à décagonal ayant jusqu'à trois hétéroatomes de la série N, O et/ou S ou hétérocyclyle penta- ou hexagonal ayant jusqu'à trois hétéroatomes de la série N, O et/ou S, les restes aryle, hétéroaryle et hétérocyclyle pouvant eux-mêmes porter jusqu'à trois substituants, indépendants les uns des autres, halogéno, hydroxy, cyano, nitro, trifluorométhyle, trifluorométhoxy, alkyle en C₁ à C₆, alkoxy en C₁ à C₆, (alkoxy en C₁ à C₆) carbonyle ou mono- ou di(alkyle en C₁ à C₆)amino,
R⁶ représente l'hydrogène, un reste (alkoxy en C₁ à C₆)carbonyle ou un groupe carboxyle,
R¹ représente l'hydrogène, un reste alkyle en C₁ à C₆ qui peut lui-même être substitué par un radical hydroxy ou alkoxy en C₁ à C₄, un reste cycloalkyle en C₃ à C₇, aryle en C₆ à C₁₀, hétéroaryle penta- à décagonal ayant jusqu'à deux hétéroatomes de la série N, O et/ou S, les restes aryle et hétéroaryle pouvant eux-mêmes être substitués indépendamment l'un de l'autre par un halogène, ou bien un reste de formule -NR⁷R⁸ ou -OR⁹,
où
R⁷ et R⁸ représentent, indépendamment l'un de l'autre, l'hydrogène, un reste aryle en C₆ à C₁₀, adamantyle, alkyle en C₁ à C₈, dont la chaîne peut être interrompue par un ou deux atomes d'oxygène et qui peut porter jusqu'à trois substituants, indépendamment les uns des autres, hydroxy, phényle, trifluorométhyle, cycloalkyle en C₃ à C₈, alkoxy en C₁ à C₆, mono- ou di (alkyle en C₁ à C₆)amino, hétérocyclyle penta- ou hexagonal ayant jusqu'à trois hétéroatomes de la série N, O et/ou S ou un hétéroaryle penta- à décagonal ayant jusqu'à trois hétéroatomes de la série N, O et/ou S, un reste cycloalkyle en C₃ à C₈ qui peut porter jusqu'à trois substituants, indépendants les uns des autres, alkyle en C₁ à C₄, hydroxy ou oxo, ou bien un reste hétérocyclyle penta- ou hexagonal ayant jusqu'à deux hétéroatomes de la série N, O et/ou S, N étant substitué par de l'hydrogène ou par un reste alkyle en C₁ à C₄,
ou bien
R⁷ et R⁸ forment, conjointement avec l'atome d'azote auquel ils sont liés, un hétérocycle saturé tétra-à heptagonal qui peut contenir jusqu'à deux autres hétéroatomes de la série N, O et/ou S et qui est éventuellement substitué par un radical hydroxy, oxo ou alkyle en C₁ à C₆ qui peut lui-même porter un radical hydroxy,
et
R⁹ représente un reste aryle en C₆ à C₁₀, adamantyle, alkyle en C₁ à C₈, dont la chaîne peut être interrompue par un ou deux atomes d'oxygène et qui peut porter un à trois substituants, indépendants les uns des autres, hydroxy, phényle, trifluorométhyle, cycloalkyle en C₃ à C₈, alkoxy en C₁ à C₆, mono- ou di (alkyle en C₁ à C₆)amino, hétérocyclyle penta- ou hexagonal ayant jusqu'à trois hétéroatomes de la série N, O et/ou S, ou hétéroaryle penta- à décagonal ayant jusqu'à trois hétéroatomes de la série N, O et/ou S, cycloalkyle en C₃ à C₈ qui peut porter jusqu'à trois substituants, indépendamment les uns des autres, alkyle en C₁ à C₄, hydroxy ou oxo, ou bien un reste hétérocyclyle penta- ou hexagonal ayant jusqu'à deux hétéroatomes de la série N, O et/ou S, N étant substitué par de l'hydrogène ou un reste alkyle en C₁ à C₄,
R³ représente un reste alkyle en C₁ à C₈, dont la chaîne peut être interrompue par un atome de soufre ou par un groupe S(O) ou SO₂, un reste phényle, benzyle ou un reste hétéroaryle penta- ou hexagonal ayant jusqu'à deux hétéroatomes de la série N, O et/ou S, les restes phényle, benzyle et hétéroaryle pouvant porter jusqu'à trois substituants, indépendants les uns des autres, halogéno, trifluorométhyle, cyano, nitro, hydroxy, alkyle en C₁ à C₆ ou alkoxy en C₁ à C₆,
et
R⁴ représente un reste de formule -C(O)-NR¹⁰R¹¹,
dans laquelle
R¹⁰ et R¹¹ représentent, indépendamment l'un de l'autre, l'hydrogène ou un reste alkyle en C₁ à C₆,
et leurs sels, leurs hydrates, les hydrates de leurs sels et leurs produits de solvatation.

3. Composés suivant la revendication 1,
dans lesquels
D représente un reste de formule 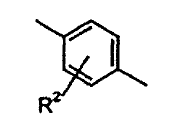 où
R² représente l'hydrogène, le chlore ou le fluor,
A désigne un atome d'oxygène ou un groupe de formule N-R⁵,
dans laquelle
R⁵ représente l'hydrogène, un reste alkyle en C₁ à C₆ qui peut lui-même porter jusqu'à deux substituants hydroxy, un reste cycloalkyle en C₃ à C₇, phényle ou hétéroaryle penta- ou hexagonal ayant jusqu'à trois hétéroatomes de la série N, O et/ou S, les restes phényle et hétéroaryle pouvant eux-mêmes être substitués jusqu'à deux fois, indépendamment l'un de l'autre, par un radical halogéno, cyano, trifluorométhyle, trifluorométhoxy, alkyle en C₁ à C₄, alkoxy en C₁ à C₄, ou di (alkyle en C₁ à C₆)amino,
R¹ représente l'hydrogène, un reste alkyle en C₁ à C₆, qui peut lui-même être substitué par un radical hydroxy ou alkoxy en C₁ à C₄; un reste cycloalkyle en C₃ à C₇, phényle, hétéroaryle penta- ou hexagonal ayant jusqu'à deux hétéroatomes de la série N, O et/ou S, les restes phényle et hétéroaryle pouvant eux-mêmes être substitués indépendamment l'un de l'autre par un halogène, ou bien un reste de formule -NR⁷R⁸ ou -OR⁹,
où
R⁷ et R⁸ représentent, indépendamment l'un de l'autre, l'hydrogène, un reste phényle, adamantyle, alkyle en C₁ à C₆, dont la chaîne peut être interrompue par un ou deux atomes d'oxygène et qui peut porter jusqu'à deux substituants, indépendants l'un de l'autre, hydroxy, phényle, trifluorométhyle, cycloalkyle en C₃ à C₆, alkoxy en C₁ à C₄, mono-ou di(alkyle en C₁ à C₄)amino, hétérocyclyle penta- ou hexagonal ayant jusqu'à deux hétéroatomes de la série N et/ou O ou hétéroaryle penta- ou hexagonal ayant jusqu'à trois hétéroatomes de la série N, O et/ou S, un reste cycloalkyle en C₃ à C₈ qui être substitué jusqu'à deux fois par un radical hydroxy, ou bien un reste hétérocyclyle penta- ou hexagonal ayant jusqu'à deux hétéroatomes de la série N, O et/ou S, N étant substitué par de l'hydrogène ou un radical alkyle en C₁ à C₄,
ou bien
R⁷ et R⁸ forment, conjointement avec l'atome d'azote auquel ils sont liés, un hétérocycle saturé tetra-à heptagonal qui peut contenir jusqu'à deux autres hétéroatomes de la série N, O et/ou S et qui est éventuellement substitué par un radical hydroxy, oxo ou alkyle en C₁ à C₆ qui peut lui-même porter un radical hydroxy,
et
R⁹ représente un reste phényle, adamantyle, alkyle en C₁ à C₆, dont la chaîne peut être interrompue par un ou deux atomes d'oxygène et qui peut porter jusqu'à deux substituants, indépendants l'un de l'autre, hydroxy, phényle, trifluorométhyle, cycloalkyle en C₃ à C₆, alkoxy en C₁ à C₃, mono-ou di(alkyle en C₁ à C₄)amino, hétérocyclyle penta- ou hexagonal ayant jusqu'à deux hétéroatomes de la série N, et/ou O ou hétéroaryle penta- ou hexagonal ayant jusqu'à trois hétéroatomes de la série N, O et/ou S, un reste cycloalkyle en C₃ à C₈ qui peut porter jusqu'à deux substituants hydroxy, ou un reste hétérocyclyle penta- ou hexagonal ayant jusqu'à deux hétéroatomes de la série N, O et/ou S, N étant substitué par de l'hydrogène ou par un radical alkyle en C₁ à C₄,
R³ représente un reste alkyle en C₁ à C₈, dont la chaîne peut être interrompue par un atome de soufre ou par un groupe S(O) ou SO₂, un reste phényle, benzyle ou un reste hétéroaryle penta- ou hexagonal ayant jusqu'à deux hétéroatomes de la série N, O et/ou S, les restes phényle, benzyle et hétéroaryle pouvant porter jusqu'à deux substituants, indépendants l'un de l'autre, halogéno, trifluorométhyle, cyano, alkyle en C₁ à C₃, alkoxy en C₁ à C₃ ou hydroxy,
et
R⁴ représente un reste de formule -C(O)-NR¹⁰R¹¹,
dans laquelle
R¹⁰ et R¹¹ représentent, indépendamment l'un de l'autre, l'hydrogène ou un reste alkyle en C₁ à C₆,
et leurs sels, leurs hydrates, les hydrates de leurs sels et leurs produits de solvatation.

4. Composés suivant la revendication 1, dans lesquels
D représente un reste 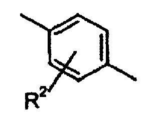 où
R² représente l'hydrogène,
A représente un atome d'oxygène ou un groupe de formule N-R⁵,
dans laquelle
R⁵ représente l'hydrogène, un reste alkyle en C₁ à C₆ qui peut lui-même être substitué jusqu'à deux fois par un radical hydroxy, un reste cycloalkyle en C₃ à C₇, phényle ou hétéroaryle penta- ou hexagonal ayant jusqu'à trois hétéroatomes de la série N, O et/ou S, les restes phényle et hétéroaryle pouvant eux-mêmes porter jusqu'à deux substituants, indépendants l'un de l'autre, fluoro, chloro, cyano, trifluorométhyle, trifluorométhoxy, alkyle en C₁ à C₃, alkoxy en C₁ à C₃, ou di(alkyle en C₁ à C₃)amino,
R¹ représente un reste alkyle en C₁ à C₄ ou un reste de formule -NR⁷R⁸,
dans laquelle
R⁷ et R⁸ représentent, indépendamment l'un de l'autre, l'hydrogène, un reste phényle, adamantyle, alkyle en C₁ à C₄, dont la chaîne peut être interrompue par un ou deux atomes d'oxygène et qui peut porter jusqu'à deux substituants, indépendants l'un de l'autre, hydroxy, phényle, trifluorométhyle, cycloalkyle en C₃ à C₆, alkoxy en C₁ à C₃, mono-ou di(alkyle en C₁ à C₃)amino, hétérocyclyle penta- ou hexagonal ayant jusqu'à deux hétéroatomes de la série N et/ou O, ou hétéroaryle penta- ou hexagonal ayant jusqu'à trois hétéroatomes de la série N, O et/ou S, un reste cycloalkyle en C₃ à C₈ qui peut porter jusqu'à deux substituants hydroxy, ou un reste hétérocyclyle penta- ou hexagonal ayant jusqu'à deux hétéroatomes de la série N, O et/ou S, N étant substitué par de l'hydrogène ou un radical alkyle en C₁ à C₄,
ou bien
R⁷ et R⁸ forment, conjointement avec l'atome d'azote auquel ils sont liés, un hétérocycle saturé tétra-à heptagonal qui peut contenir jusqu'à deux autres hétéroatomes de la série N, O et/ou S et qui est éventuellement substitué par un radical hydroxy, oxo ou alkyle en C₁ à C₆ qui peut lui-même porter un substituant hydroxy,
R³ représente un reste alkyle en C₁ à C₈, dont la chaîne peut être interrompue par un atome de soufre ou par un groupe S(O) ou SO₂, un reste phényle, benzyle ou un hétéroaryle penta- ou hexagonal ayant jusqu'à deux hétéroatomes de la série N, O et/ou S, les restes phényle, benzyle et hétéroaryle pouvant porter jusqu'à deux substituants, indépendants l'un de l'autre, halogéno, trifluorométhyle, cyano, alkyle en C₁ à C₃, alkoxy en C₁ à C₃ ou hydroxy,
et
R⁴ représente un reste de formule -C (O) -NR¹⁰R¹¹, dans laquelle
R¹⁰ et R¹¹ représentent, indépendamment l'un de l'autre, l'hydrogène, un reste méthyle ou éthyle,
et leurs sels, leurs hydrates, les hydrates de leurs sels et leurs produits de solvatation.

5. Composés suivant la revendication 1,
dans lesquels
D représente un reste 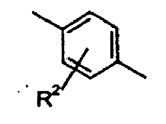 où
R² représente l'hydrogène,
A représente un atome d'oxygène ou un groupe de formule N-R⁵,
dans laquelle
R⁵ représente un reste cycloalkyle en C₃ à C₇, phényle, qui peut être substitué par du fluor, ou pyridyle,
R¹ représente un reste méthyle ou un reste de formule -NR⁷R⁸,
dans laquelle
R⁷ et R⁸ représentent, indépendamment l'un de l'autre, un reste alkyle en C₁ à C₄, qui peut être substitué une ou deux fois par un radical hydroxy,
ou bien
R⁷ et R⁸ forment, conjointement avec l'atome d'azote auquel ils sont liés, un hétérocycle saturé penta-ou hexagonal qui peut contenir un autre hétéroatome O ou N, N étant substitué par de l'hydrogène ou par un radical alkyle en C₁ à C₃ qui peut lui-même être substitué par un radical hydroxy,
R³ est un reste phényle qui peut être substitué éventuellement en position para par du fluor, ou un reste pyridyle,
et
R⁴ représente un reste de formule -C(O)-NR¹⁰R¹¹,
dans laquelle
R¹⁰ et R¹¹ représentent l'hydrogène,
et leurs sels, leurs hydrates, les hydrates de leurs sels et leurs produits de solvatation.

6. Composés suivant la revendication 1, **caractérisés par** l'une des configurations stéréochimiques suivantes, selon les formules (Ia) à (Id) :

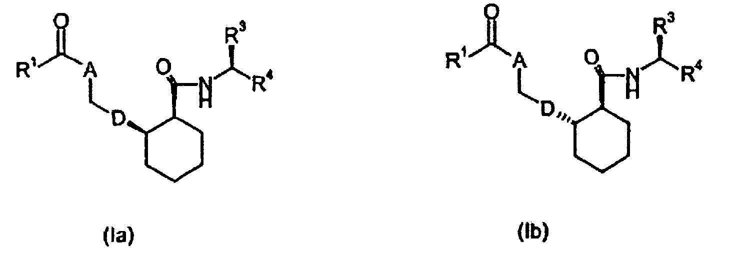

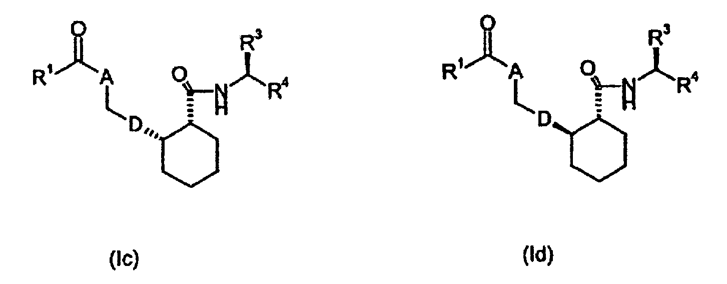

7. Composés suivant la revendication 1, **caractérisés par** la configuration stéréochimique suivante, selon la formule (Id) :

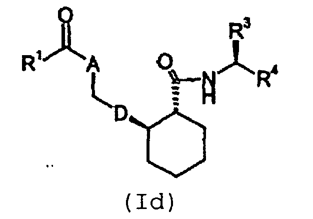

8. Composés suivant la revendication 1, de structures suivantes :
amide d'acide (1*R*,2*R*)-*N*-[(1*S*)-2-amino-(4-fluorophényl)-2-oxoéthyl]-2-(4-{[{[éthyl(2-hydroxyéthyl)amino]carbonyl}(4-fluorophényl)amino]méthyl}phényl)cyclohexanecarboxylique 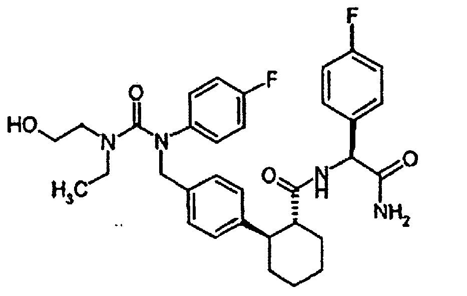
amide d'acide (1*R*,2*R*)-*N*-[(1*S*)-2-amino-2-oxo-1-phényléthyl]-2-(4-{[[(diméthylamino)-carbonyl](phényl)amino]méthyl}phényl)-cyclohexanecarboxylique 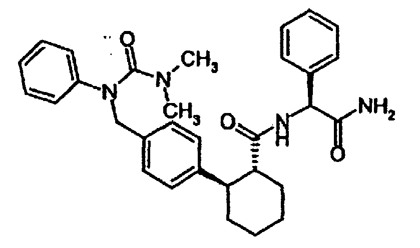
amide d'acide (1*R*,2*R*)-*N*-[(1*S*)-2-amino-2-oxo-1-phényléthyl]-2-[4-({cyclopropyl[[(diméthylamino)carbonyl]amino}méthyl)-phényl]-cyclohexanecarboxylique 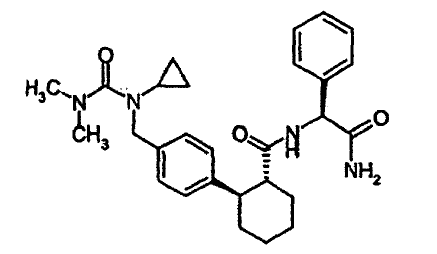
amide d'acide (1*R*,2*R*)-*N*-[(1*S*)-2-amino-2-oxo-1-phényléthyl]-2- (4-{[[(diéthylamino)-carbonyl](2-pyridinyl)amino]méthyl}-phényl)cyclohexanecarboxylique 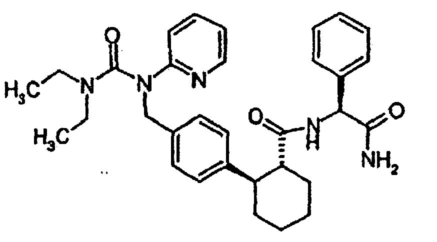
amide d'acide *N*-{4-[(1*R*,2*R*)-2-({[(1*S*)-2-amino-2-oxo-1-phényléthyl]amino}carbonyl)-cyclohexyl]benzyl}-N-phényl-4-morpholinocarboxylique 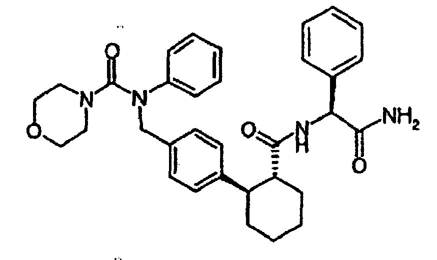
(*S*) (N-{{(1*R*,2*R*)-2-(4-{[{[2-hydroxyéthylamino]carbonyl}-(phényl)amino]-méthyl}phényl)cyclohex-1-yl}carbonyl}-phényl-glycinamide 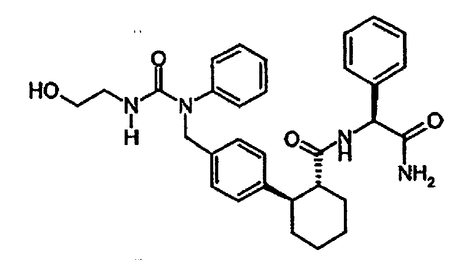
(1*R*,2*R*)-2-(4-{[acétyl(2-pyridinyl)amino]méthyl}phényl)-N-[(1*S*)-2-amino-2-oxo-1-phényléthyl]cyclohexanecarboxylique 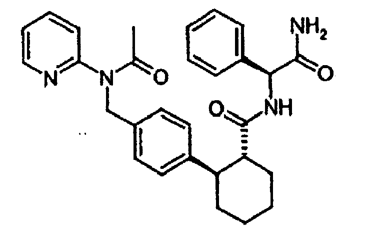
amide d'acide (1*R*,2*R*)-N-[(1*S*)-2-amino-1-phényl-2-oxoéthyl]-2-(4-{[{[éthyl(2-hydroxyéthyl)amino]carbonyl}(phényl)amino]-méthyl}phényl)cyclohexanecarboxylique 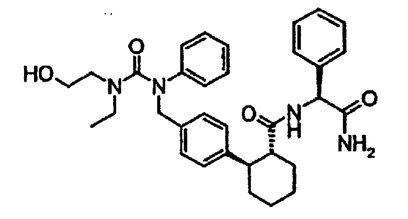
4-[(1*R*,2*R*)-2-({[(1*S*)-2-amino-1-(4-fluorophényl)-2-oxoéthyl]-amino}-carbonyl)cyclohexyl]benzyl-4-(2-hydroxyéthyl)-1-pipérazine-carbamate 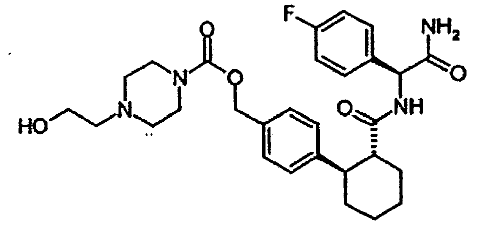
4-[(1*R*,2*R*)-2-({[(1*S*)-2-amino-1-phényl)-2-oxoéthyl]amino}-carbonyl)cyclohexyl]benzyl-4-(2-hydroxyéthyl)-1-pipérazine-carbamate 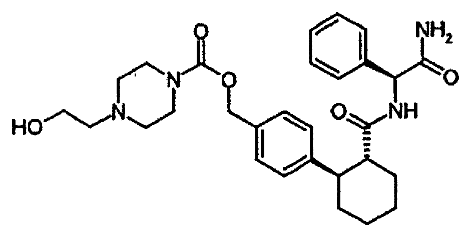 et leurs sels, leurs hydrates, les hydrates de leurs sels et leurs produits de solvatation.

9. Procédé de production de composés de formule (I), **caractérisé en ce que** :
[A] on transforme tout d'abord des composés de formule (II) 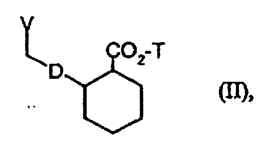 dans laquelle
D a la définition indiquée dans la revendication 1,
T représente un reste alkyle en C₁ à C₄,
et
V représente un groupe partant convenable,
par réaction avec des composés de formule (III)
B-H (III),
dans laquelle
B représente 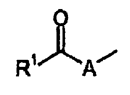
ou bien
le cas échéant, lorsque R¹ représente OR⁹,
un reste 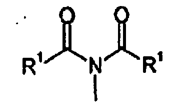 et
R¹ et A ont les définitions indiquées dans la revendication 1, en composés de formule (IV), 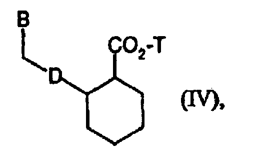 dans laquelle B et T ont la définition indiquée ci-dessus et D a la définition indiquée dans la revendication 1,
que l'on transforme dans une étape subséquente, par réaction avec des acides ou des bases, en acides carboxyliques correspondants de formule (V) 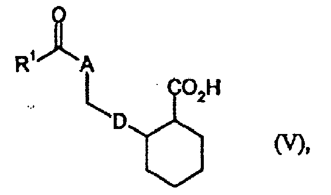 dans laquelle
R¹, A et D ont la définition indiquée ci-dessus,
que l'on fait finalement réagir selon des modes opératoires connus avec des composés de formule (VI) ou leurs sels 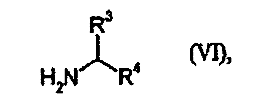 dans laquelle
R³ et R⁴ ont la définition indiquée dans la revendication 1,
dans des solvants inertes,
ou bien
[B] au cas où A désigne un atome d'oxygène ou un groupe NR⁵, on fait réagir des composés de formule (VII) 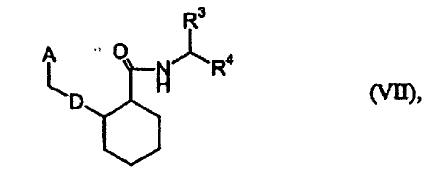
dans laquelle
D, R³ et R⁴ ont les définitions indiquées dans la revendication 1,
et
A représente un atome d'oxygène ou un groupe de formule N-R⁵,
dans laquelle R⁵ a la définition indiquée dans la revendication 1,
soit avec des composés de formule (VIII) 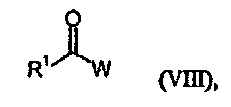 dans laquelle
R¹ a la définition indiquée dans la revendication 1 et W représente un groupe partant convenable,
soit avec un équivalent de phosgène puis avec des composés de formule (IX)
R⁷R⁸NH (IX)
dans laquelle
R⁷ et R⁸ ont les définitions indiquées dans la revendication 1,
ou bien
avec un isocyanate de formule (X)
R⁷NCO (X)
dans laquelle
R⁷ a les définitions indiquées dans la revendication 1.

10. Procédé de production de composés de formule (I), **caractérisé en ce que** :
[A] on transforme des composés de formule (V) 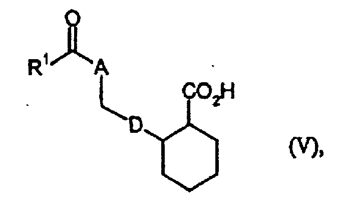 dans laquelle
R¹, A et D ont la définition indiquée dans la revendication 1,
selon des modes opératoires connus, avec des composés de formule (VI) ou leurs sels 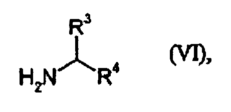 dans laquelle
R³ et R⁴ ont la définition indiquée dans la revendication 1,
dans des solvants inertes,
ou bien
[B] au cas où A représente un atome d'oxygène ou un groupe NR⁵, on fait réagir des composés de formule (VII) 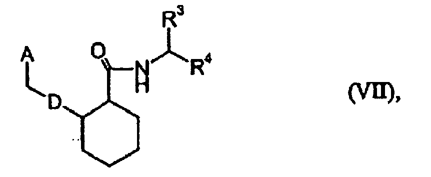
dans laquelle
D, R³ et R⁴ ont les définitions indiquées dans la revendication 1,
et
A représente un atome d'oxygène ou un groupe de formule N-R⁵,
dans laquelle R⁵ a la définition indiquée dans la revendication 1,
soit avec des composés de formule (VIII) 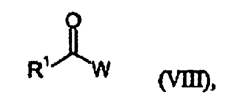 dans laquelle
R¹ a la définition indiquée dans la revendication 1 et W représente un groupe partant convenable,
soit avec un équivalent de phosgène puis avec des composés de formule (IX)
R⁷R⁸NH (IX)
dans laquelle
R⁷ et R⁸ ont les définitions indiquées dans la revendication 1,
ou bien
avec un isocyanate de formule (X)
R⁷NCO (X)
dans laquelle
R⁷ a les définitions indiquées dans la revendication 1.

11. Composés de formule (V) 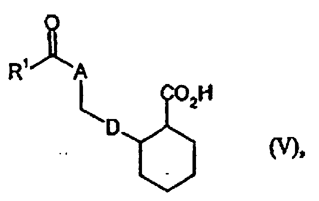 dans laquelle
R¹, A et D ont la définition indiquée dans la revendication 1,
et leurs sels, leurs hydrates, les hydrates de leurs sels et leurs produits de solvatation.

12. Composés de formule (VII) 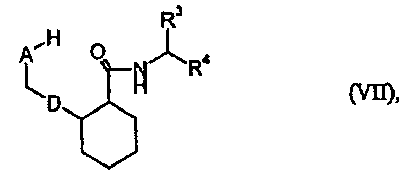 dans laquelle
R³, R⁴, A et D ont la définition indiquée dans la revendication 1, et leurs sels, leurs hydrates, les hydrates de leurs sels et leurs produits de solvatation.

13. Composés de formule (I) telle que définie dans l'une des revendications précédentes, destinés à combattre des maladies.

14. Médicament contenant au moins un composé de formule (I) telle que définie dans l'une des revendications précédentes, et au moins une autre substance active.

15. Médicament contenant au moins un composé de formule (I) telle que définie dans l'une des revendications précédentes, et au moins une autre substance auxiliaire.

16. Utilisation de composés de formule (I) telle que définie dans l'une des revendications précédentes, pour la préparation de médicaments destinés à la prévention et/ou au traitement de maladies périphériques et cardiovasculaires dues à une ischémie.

17. Utilisation de composés de formule (I) telle que définie dans l'une des revendications précédentes, pour la préparation de médicaments destinés au traitement aigu et chronique de maladies ischémiques du système cardiovasculaire, par exemple de la maladie cardiaque coronarienne, de l'angor stable et instable, de pathologies d'occlusion périphérique et artérielle, d'occlusions thrombotiques de vaisseaux, de l'infarctus du myocarde et de lésions de reperfusion.
